(19) Europäisches Patentamt
European Patent Office
Office européen des brevets

(11) Veröffentlichungsnummer: **0 341 489 B1**

(12) **EUROPÄISCHE PATENTSCHRIFT**

(45) Veröffentlichungstag der Patentschrift: **23.08.95**

(21) Anmeldenummer: **89107529.3**

(22) Anmeldetag: **26.04.89**

(51) Int. Cl.6: **C07D 249/12**, C07D 401/12, C07D 403/12, C07D 405/12, C07D 409/12, C07D 413/12, C07D 417/12, C07D 471/04, C07D 487/04, A01N 43/653

# (54) Sulfonylaminocarbonyltriazolinone.

(30) Priorität: **09.05.88 DE 3815765**

(43) Veröffentlichungstag der Anmeldung:
**15.11.89 Patentblatt 89/46**

(45) Bekanntmachung des Hinweises auf die Patenterteilung:
**23.08.95 Patentblatt 95/34**

(84) Benannte Vertragsstaaten:
**BE CH DE FR GB IT LI NL**

(56) Entgegenhaltungen:
**DE-A- 2 707 801**
**DE-A- 3 206 235**
**DE-A- 3 709 574**
**US-A- 4 213 773**

**CHEMICAL ABSTRACTS, Band 105, Nr. 19, 10. November 1986, Columbus, Ohio, USA YAMADA, KURA et al. " Bicyclic triazolones." Seite 749, Spalte 2, Zusammenfassung Nr. 172 473j & Jpn. Kokai Tokkyo Koho JP 61 69,776 (86 69,776)**

(73) Patentinhaber: **BAYER AG**
**D-51368 Leverkusen (DE)**

(72) Erfinder: **Daum, Werner, Dr.**
**Baerenstrasse 18**
**D-4150 Krefeld 1 (DE)**
Erfinder: **Müller, Klaus-Helmut, Dr.**
**Bockhackstrasse 55**
**D-4000 Düsseldorf 13 (DE)**
Erfinder: **Schwamborn, Michael, Dr.**
**von-Lohe-Strasse 9**
**D-5000 Köln 80 (DE)**
Erfinder: **Babczinski, Peter, Dr.**
**In der Lohrenbeck 11**
**D-5600 Wuppertal 1 (DE)**
Erfinder: **Santel, Hans-Joachim, Dr.**
**Gruenstrasse 9 a**
**D-5090 Leverkusen 1 (DE)**
Erfinder: **Schmidt, Robert R., Dr.**
**Im Waldwinkel 110**
**D-5060 Bergisch Gladbach 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 341 489 B1**

**CHEMICAL ABSTRACTS, Band 97, Nr. 5, 2. August 1982, Columbus, Ohio, USA LANGLOIS, MICHEL et al. "Synthesis of new bicyclic amidines. 1. Derivatives of imidazole, 1,3,4-triazole and tetrazole." Seite 570, Spalte 2, Zusammenfassung Nr. 38 890f & J. Heterocycl. Chem. 1982, 19 (1), 193-200**

**CHEMICAL ABSTRACTS, Band 90, Nr. 19, 7. Mai 1979, Columbus,Ohio, USA IWAI,SADAYOSHI et al. "Triazoline derivatives." Seite 617, Spalte 1, Zusammenfassung Nr. 152 195p & Jpn. Kokai Tokkyo Koho 78,135,981**

**CHEMICAL ABSTRACTS, Band 94, Nr. 21, 25. Mai 1981, Columbus, Ohio, USA MILCENT, RENE et al. "Research on a series of 1,2,4-triazoles. II. Reactivity of 4-amino-3-aryl-1,2,4-triazol-5-ones." Seite 720, Spalte 2, Zusammenfassung Nr. 175 000t & J. Heterocycl. Chem. 1980, 17(8), 1691-6**

**CHEMICAL ABSTRACTS, Band 73, Nr. 5, 3. August 1970, Columbus, Ohio, USA REIMLINGER, HANS et al. "Synthesis and properties of N-acylated condensed 3-oxo-2,3-dihydro-s-triazoles and the isomeric 2-oxo-2,3-dihydro-1,3,4-oxadiazoles." Seite 357, Spalte 2, Zusammenfassung Nr. 25 365t & Chem. Ber. 1970, 103(6), 1934-41**

Erfinder: **Strang, Harry, Dr.**
**Unterdorfstrasse 6 a**
**D-4000 Düsseldorf 31 (DE)**

**Beschreibung**

Die Erfindung betrifft Sulfonylaminocarbonyltriazolinone, ein Verfahren zu ihrer Herstellung und ihre Verwendung als Pflanzenbehandlungsmittel.

Es ist bekannt, daß bestimmte substituierte Aminocarbonylimidazolidinone, wie z. B. 1-Isobutylamino-carbonyl-2-imidazolidinon (Isocarbamid) herbizide Eigenschaften aufweisen (vgl. R. Wegler, Chemie der Pflanzenschutz- und Schädlingsbekämpfungsmittel, Band 5, S. 219, Springer-Verlag, Berlin-Heidelberg-New York, 1977). Die Wirkung dieser Verbindung ist jedoch nicht in allen Belangen zufriedenstellend.

Es wurden nun die Sulfonylaminocarbonyl-triazolinone der allgemeinen Formel (I) sowie Salze von Verbindungen der Formel (I) gefunden,

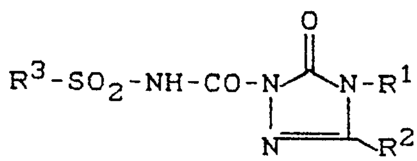

(I)

in welcher

$R^1$ für Wasserstoff, Hydroxy, Amino, für gegebenenfalls durch Fluor, Chlor, Brom, Cyano, $C_1$-$C_4$-Alkoxy, $C_1$-$C_4$-Alkylcarbonyl oder $C_1$-$C_4$-Alkoxy-carbonyl substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio, $C_1$-$C_4$-Alkylsulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für gegebenenfalls durch Fluor, Cyano, $C_1$-$C_4$-Alkoxy oder $C_1$-$C_4$-Alkoxy-carbonyl substituiertes $C_1$-$C_4$-Alkylamino oder für Di-($C_1$-$C_4$-alkyl)-amino steht,

$R^2$ für Wasserstoff, Hydroxy, Mercapto, Amino, für gegebenenfalls durch Fluor, Chlor, Brom, Cyano, $C_1$-$C_4$-Alkoxy oder $C_1$-$C_4$-Alkoxy-carbonyl substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio,$C_1$-$C_4$-Alkyl-sulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für gegebenenfalls durch Fluor, Chlor, Cyano, $C_1$-$C_4$-Alkoxy oder $C_1$-$C_4$-Alkoxy-carbonyl substituiertes Alkylthio, für $C_1$-$C_4$-Alkylamino oder Di-($C_1$-$C_4$-alkyl)-amino steht, oder

$R^1$ und $R^2$ zusammen für gegebenenfalls verzweigtes Alkandiyl mit 3 bis 11 Kohlenstoffatomen stehen und

$R^3$ für die Gruppierung

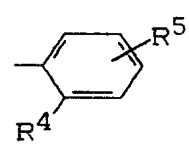

steht, worin

$R^4$ und $R^5$ gleich oder verschieden sind und für Wasserstoff, Fluor, Chlor, Brom, Iod, Nitro, $C_1$-$C_6$-Alkyl [welches gegebenenfalls durch Fluor, Chlor, Brom, Cyano, Carboxy, $C_1$-$C_4$-

Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-Alkoxycarbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxycarbonyl substituiert ist], $C_3$-$C_6$-Alkinyloxy, $C_3$-$C_6$-Alkinylthio oder für den Rest -S-$(O)_p$-$R^6$ stehen, wobei

p für die Zahlen 1 oder 2 steht und

$R^6$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest -NHOR$^7$ steht, wobei

$R^7$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^4$ und/oder $R^5$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest -CO-$R^8$ stehen, wobei

$R^8$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^4$ und/oder $R^5$ weiterhin für $C_1$-$C_4$-Alkylsulfonyloxy, Di-($C_1$-$C_4$-alkyl)-aminosulfonyl-amino oder für den Rest -CH=N-$R^9$ stehen, wobei

$R^9$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^3$ für den Rest

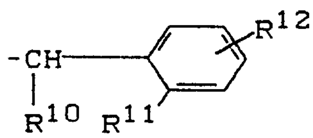

steht, worin

$R^{10}$ für Wasserstoff oder $C_1$-$C_4$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/ oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^3$ für den Rest

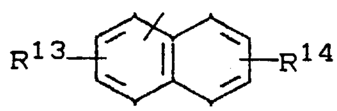

steht, worin

$R^{17}$ und $R^{18}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$ Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter

$R^3$ für den Rest

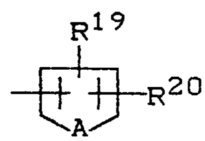

steht, worin

$R^{19}$ und $R^{20}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und

A für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter

$R^3$ für den Rest

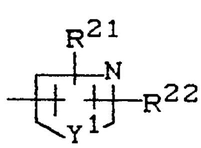

steht, worin

$R^{21}$ und $R^{22}$ gleich oder verschieden sind und für Wasserstoff, $C_1$-$C_4$-Alkyl, Halogen, $C_1$-$C_4$-Alkox-

ycarbonyl, $C_1$-$C_4$-Alkoxy oder $C_1$-$C_4$-Halogenalkoxy stehen,

$Y^1$ für Schwefel oder die Gruppierung N-$R^{23}$ steht, wobei

$R^{23}$ für Wasserstoff oder $C_1$-$C_4$-Alkyl steht; worin weiter

$R^3$ für den Rest

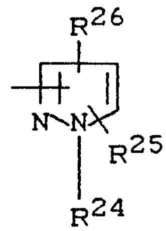

steht, worin

$R^{24}$ für Wasserstoff, $C_1$-$C_4$-Alkyl, Benzyl oder Phenyl steht,

$R^{25}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder $C_1$-$C_4$-Alkoxy-carbonyl steht und

$R^{26}$ für Wasserstoff, Halogen oder $C_1$-$C_4$-Alkyl steht.

Man erhalt die neuen Sulfonylaminocarbonyltriazolinone der allgemeinen Formel (I), wenn man Triazolinone der allgemeinen Formel (II)

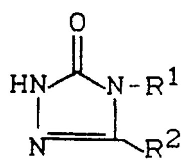

(II)

in welcher

$R^1$ und $R^2$ die oben angegebenen Bedeutungen haben,

mit Sulfonylisocyanaten der allgemeinen Formel (III)

$R^3 - SO_2 - N = C = O$ (III)

in welcher

$R^3$ die oben angegebene Bedeutung hat,

gegebenenfalls in Gegenwart eines Verdünnungsmittels umsetzt und gegebenenfalls im Anschluß daran Salze nach üblichen Methoden erzeugt.

Eine weitere mögliche Herstellungsmethode für die Verbindungen der Formel (I) ist nachstehend skizziert, wobei $R^1$, $R^2$ und $R^3$ die oben angegebenen Bedeutungen haben (Z : Chlor, $C_1$-$C_4$-Alkoxy, Benzyloxy, Phenoxy):

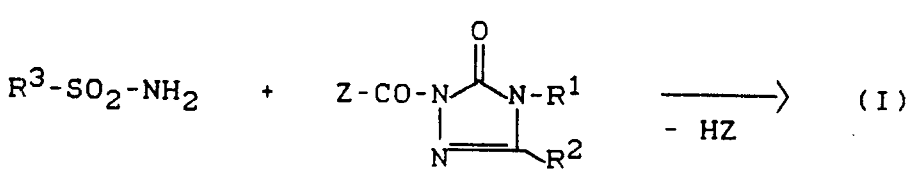


Die neuen Sulfonylaminocarbonyltriazolinone der allgemeinen Formel (I) zeichnen sich durch starke herbizide und zusätzlich durch fungizide Wirksamkeit aus.

Überraschenderweise zeigen die neuen Verbindungen der Formel (I) erheblich bessere herbizide Wirkung als das strukturell ähnliche bekannte Herbizid 1-Isobutylaminocarbonyl-2-imidazolidinon (Isocarbamid).

Gegenstand der Erfindung sind weiter vorzugsweise Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, $C_5$- oder $C_6$-

Cycloalkyl-ammonium- und Di-($C_1$-$C_2$-alkyl)-benzyl-ammonium-Salze von Verbindungen der Formel (I), in welcher $R^1$, $R^2$ und $R^3$ die oben angegebenen Bedeutungen haben.

Gegenstand der Erfindung sind insbesondere Verbindungen der Formel (I), in welcher

$R^1$ für Wasserstoff, für gegebenenfalls durch Fluor, Cyano, Methoxy oder Ethoxy substituiertes $C_1$-$C_4$-Alkyl, für Cyclopropyl, Benzyl oder Dimethylamino steht,

$R^2$ für Wasserstoff oder für gegebenenfalls durch Fluor und/oder Chlor, Methoxy oder Ethoxy substituiertes $C_1$-$C_4$-Alkyl steht, oder

$R^1$ und $R^2$ zusammen für Alkandiyl mit 3 bis 11 Kohlenstoffatomen stehen und

$R^3$ für die Gruppierung

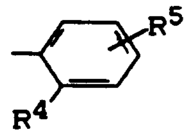

steht, worin

$R^4$ für Fluor, Chlor, Brom, Methyl, Trifluormethyl, Methoxy, Difluormethoxy, Trifluormethoxy, 2-Chlor-ethoxy, 2-Methoxy-ethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und

$R^5$ für Wasserstoff, Fluor, Chlor oder Brom steht; worin weiter

$R^3$ für den Rest

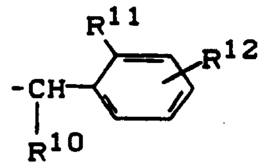

steht, worin

$R^{10}$ für Wasserstoff steht,

$R^{11}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{12}$ für Wasserstoff steht; worin weiter

$R^3$ für den Rest

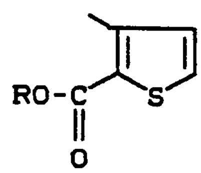

steht,
worin R für $C_1$-$C_4$-Alkyl steht, oder
für den Rest

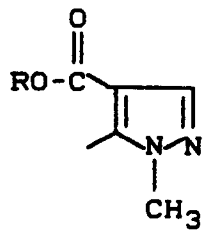

steht,

worin R für $C_1$-$C_4$-Alkyl steht.

Beispiele für die erfindungsgemäßen Verbindungen sind in der nachstehenden Tabelle 1 aufgeführt - vgl. auch die Herstellungsbeispiele.

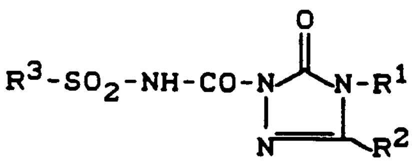

(I)

**Tabelle 1: Beispiele für die Verbindungen der Formel (I)**

| $R^1$ | $R^2$ | $R^3$ |
|---|---|---|
| H | H | 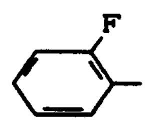 |
| H | H | 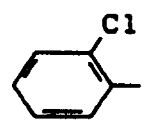 |

Tabelle 1 - Fortsetzung

| $R^1$ | $R^2$ | $R^3$ |
|---|---|---|
| H | H | 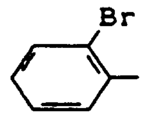 |
| H | H | 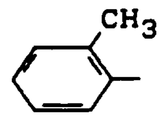 |
| H | H | 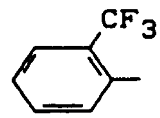 |
| H | H | 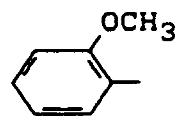 |
| H | H | 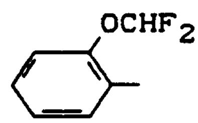 |
| H | H | 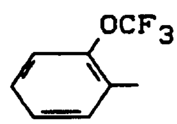 |
| $-(CH_2)_6-$ | | 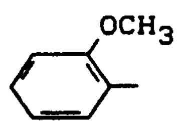 |
| $-(CH_2)_7-$ | | 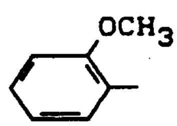 |

Tabelle 1 - Fortsetzung

| $R^1$ | $R^2$ | $R^3$ |
|---|---|---|
| $-(CH_2)_{11}-$ | | 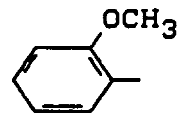 |
| H | H | 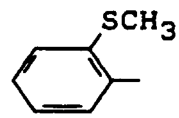 |
| H | H | 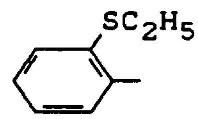 |
| H | H | 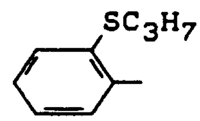 |
| H | H | 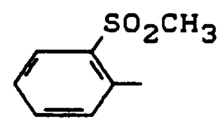 |
| H | H | 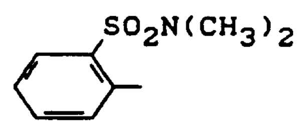 |
| H | H | 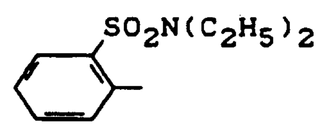 |
| H | H | 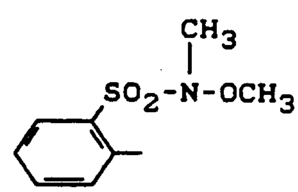 |

Tabelle 1 - Fortsetzung

| $R^1$ | $R^2$ | $R^3$ |
|---|---|---|
| H | H | 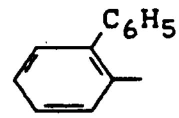 |
| H | H | 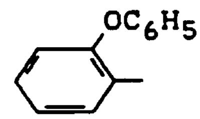 |
| H | H | 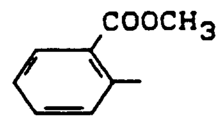 |
| H | H | 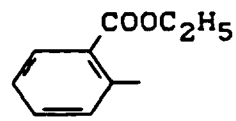 |
| H | H | 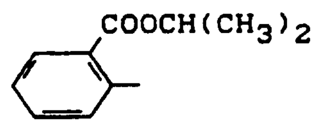 |
| $-(CH_2)_6-$ | | 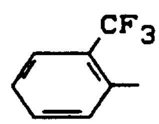 |
| $CH_3$ | H | 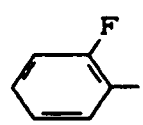 |
| $C_2H_5$ | H | 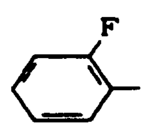 |

Tabelle 1 - Fortsetzung

| $R^1$ | $R^2$ | $R^3$ |
|---|---|---|
| $C_3H_7$ | H | 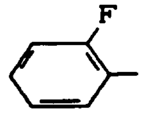 |
| $CH(CH_3)_2$ | H | 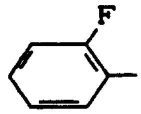 |
| $C_4H_9$ | H | 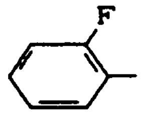 |
| $CH_2CH(CH_3)_2$ | H | 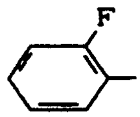 |
| $C(CH_3)_3$ | H | 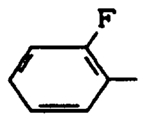 |
| H | $CH_3$ | 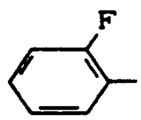 |
| H | $C_2H_5$ | 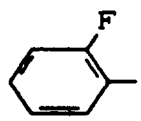 |
| H | $C_3H_7$ | 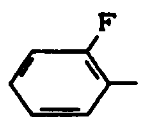 |

Tabelle 1 - Fortsetzung

| $R^1$ | $R^2$ | $R^3$ |
|---|---|---|
| H | $CH(CH_3)_2$ | 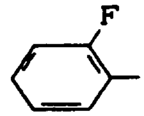 |
| H | $C_4H_9$ | 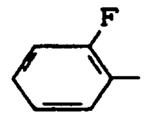 |
| H | $CH_2CH(CH_3)_2$ | 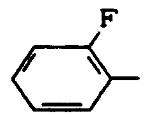 |
| H | $C(CH_3)_3$ | 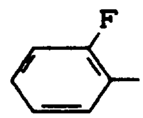 |
| $CHF_2$ | H | 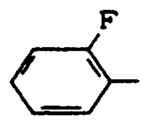 |
| $CH_2CH_2CN$ | H | 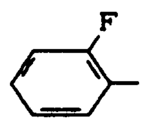 |
| $CH_2CH_2OCH_3$ | H | 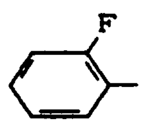 |
| H | $CF_3$ | 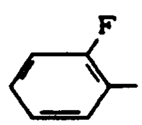 |

Tabelle 1 - Fortsetzung

| $R^1$ | $R^2$ | $R^3$ |
|---|---|---|
| $CH_2OCH_3$ | H | 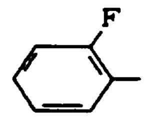 |
| H | $CH_2OCH_3$ | 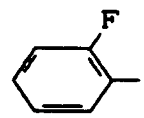 |
| H | $CH_2CH_2OCH_3$ | 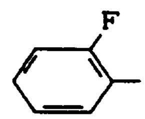 |
| $CH_3$ | $CH_3$ | 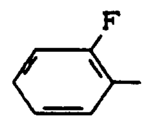 |
| $CH_3$ | $C_2H_5$ | 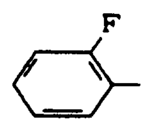 |
| $CH_3$ | $C_3H_7$ | 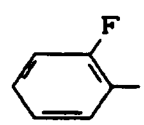 |
| $CH_3$ | $CH(CH_3)_2$ | 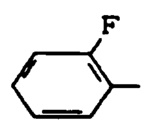 |
| $CH_3$ | $C_4H_9$ | 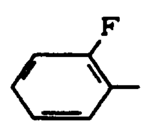 |

Tabelle 1 - Fortsetzung

| $R^1$ | $R^2$ | $R^3$ |
|---|---|---|
| $CH_3$ | $CH_2CH(CH_3)_2$ | 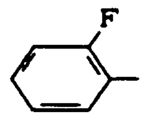 |
| $CH_3$ | $C(CH_3)_3$ | 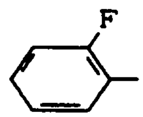 |
| $C_2H_5$ | $CH_3$ | 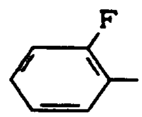 |
| $C_3H_7$ | $CH_3$ | 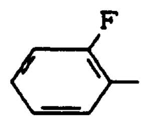 |
| $CH(CH_3)_2$ | $CH_3$ | 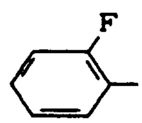 |
| $C_4H_9$ | $CH_3$ | 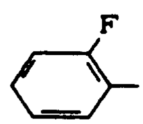 |
| $C_2H_5$ | $C_2H_5$ | 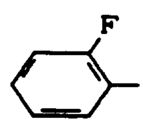 |
| $CHF_2$ | $CH_3$ | 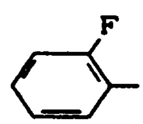 |

Tabelle 1 - Fortsetzung

| $R^1$ | $R^2$ | $R^3$ |
|---|---|---|
| $CHF_2$ | $C_2H_5$ | 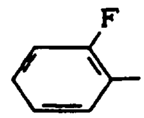 |
| $CH_3$ | $CF_3$ | 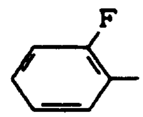 |
| $C_2H_5$ | $CF_3$ | 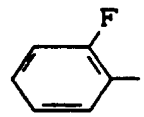 |
| $-(CH_2)_3-$ | | 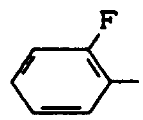 |
| $-(CH_2)_4-$ | | 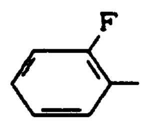 |
| $-(CH_2)_5-$ | | 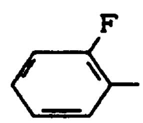 |
| $CH_3$ | H | 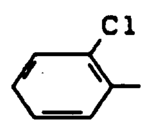 |
| $C_2H_5$ | H | 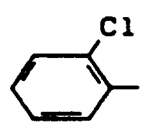 |

Tabelle 1 - Fortsetzung

| $R^1$ | $R^2$ | $R^3$ |
|---|---|---|
| $C_3H_7$ | H | 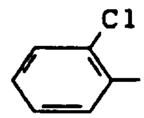 |
| $CH(CH_3)_2$ | H | 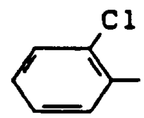 |
| $C_4H_9$ | H | 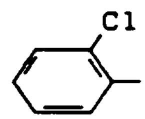 |
| $CH_2CH(CH_3)_2$ | H | 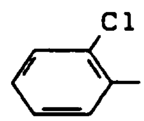 |
| $C(CH_3)_3$ | H | 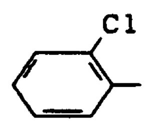 |
| H | $CH_3$ | 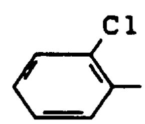 |
| H | $C_2H_5$ | 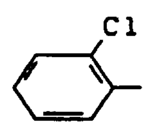 |
| H | $C_3H_7$ | 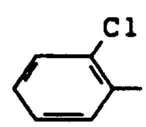 |

Tabelle 1 - Fortsetzung

| $R^1$ | $R^2$ | $R^3$ |
|---|---|---|
| H | $CH(CH_3)_2$ | 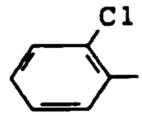 |
| H | $C_4H_9$ | 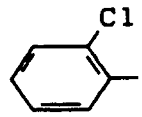 |
| H | $CH_2CH(CH_3)_2$ | 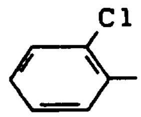 |
| H | $C(CH_3)_3$ | 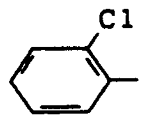 |
| $CHF_2$ | H | 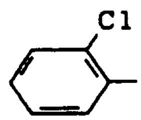 |
| $CH_2CH_2CN$ | H | 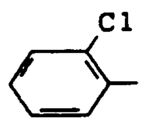 |
| $CH_2CH_2OCH_3$ | H | 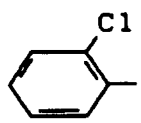 |
| H | $CF_3$ | 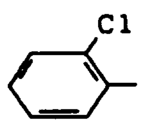 |

Tabelle 1 - Fortsetzung

| $R^1$ | $R^2$ | $R^3$ |
|---|---|---|
| $CH_2OCH_3$ | H | 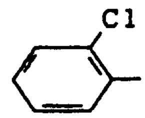 |
| H | $CH_2OCH_3$ | 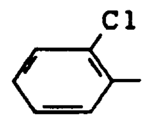 |
| H | $CH_2CH_2OCH_3$ | 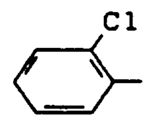 |
| $CH_3$ | $CH_3$ | 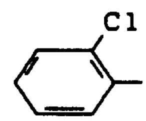 |
| $CH_3$ | $C_2H_5$ | 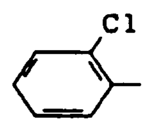 |
| $CH_3$ | $C_3H_7$ | 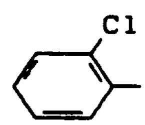 |
| $CH_3$ | $CH(CH_3)_2$ | 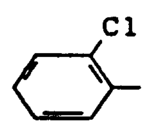 |
| $CH_3$ | $C_4H_9$ | 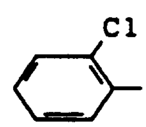 |

Tabelle 1 - Fortsetzung

| $R^1$ | $R^2$ | $R^3$ |
|---|---|---|
| $CH_3$ | $CH_2CH(CH_3)_2$ | 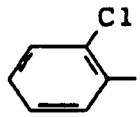 |
| $CH_3$ | $C(CH_3)_3$ | 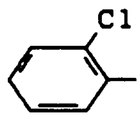 |
| $C_2H_5$ | $CH_3$ | 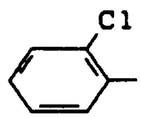 |
| $C_3H_7$ | $CH_3$ | 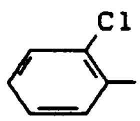 |
| $CH(CH_3)_2$ | $CH_3$ | 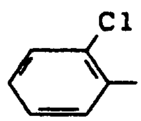 |
| $C_4H_9$ | $CH_3$ | 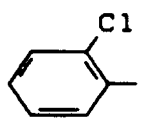 |
| $C_2H_5$ | $C_2H_5$ | 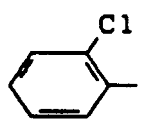 |
| $CHF_2$ | $CH_3$ | 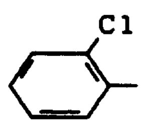 |

Tabelle 1 - Fortsetzung

| $R^1$ | $R^2$ | $R^3$ |
|---|---|---|
| $CHF_2$ | $C_2H_5$ | 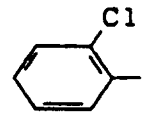 |
| $CH_3$ | $CF_3$ | 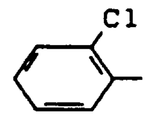 |
| $C_2H_5$ | $CF_3$ | 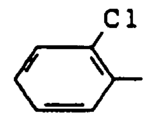 |
| $-(CH_2)_3-$ | | 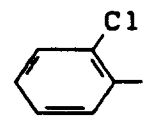 |
| $-(CH_2)_4-$ | | 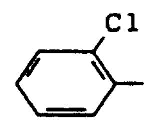 |
| $-(CH_2)_5-$ | | 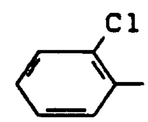 |
| $CH_3$ | $CH_3$ | 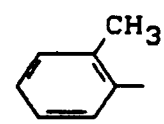 |
| H | $CH_3$ | 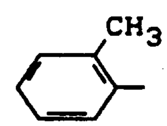 |

Tabelle 1 - Fortsetzung

| $R^1$ | $R^2$ | $R^3$ |
|---|---|---|
| $CH_3$ | H | 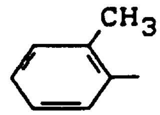 |
| $CH_3$ | H | 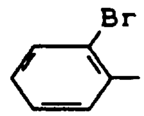 |
| $C_2H_5$ | H | 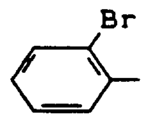 |
| $C_3H_7$ | H | 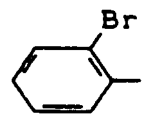 |
| $CH(CH_3)_2$ | H | 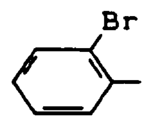 |
| $C_4H_9$ | H | 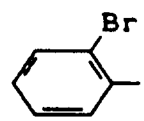 |
| $CH_2CH(CH_3)_2$ | H | 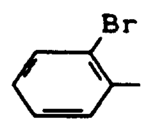 |
| $C(CH_3)_3$ | H | 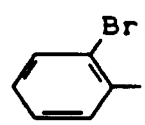 |

Tabelle 1 - Fortsetzung

| $R^1$ | $R^2$ | $R^3$ |
|---|---|---|
| H | $CH_3$ | 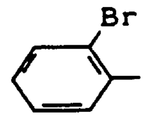 |
| H | $C_2H_5$ | 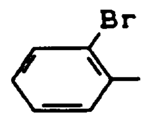 |
| H | $C_3H_7$ | 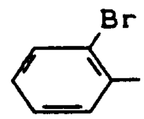 |
| H | $CH(CH_3)_2$ | 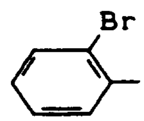 |
| H | $C_4H_9$ | 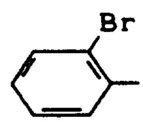 |
| H | $CH_2CH(CH_3)_2$ | 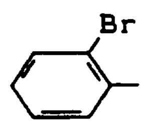 |
| H | $C(CH_3)_3$ | 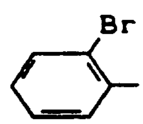 |
| $CHF_2$ | H | 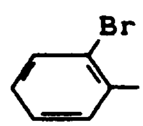 |

<u>Tabelle 1</u> - Fortsetzung

| $R^1$ | $R^2$ | $R^3$ |
|---|---|---|
| $CH_2CH_2CN$ | H | 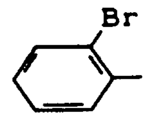 |
| $CH_2CH_2OCH_3$ | H | 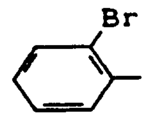 |
| H | $CF_3$ | 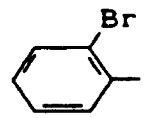 |
| $CH_2OCH_3$ | H | 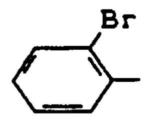 |
| H | $CH_2OCH_3$ | 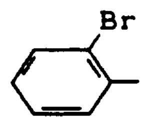 |
| H | $CH_2CH_2OCH_3$ | 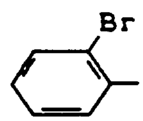 |
| $CH_3$ | $CH_3$ | 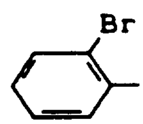 |
| $CH_3$ | $C_2H_5$ | 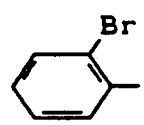 |

Tabelle 1 - Fortsetzung

| $R^1$ | $R^2$ | $R^3$ |
|---|---|---|
| $CH_3$ | $C_3H_7$ | 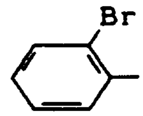 |
| $CH_3$ | $CH(CH_3)_2$ | 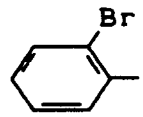 |
| $CH_3$ | $C_4H_9$ | 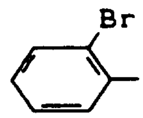 |
| $CH_3$ | $CH_2CH(CH_3)_2$ | 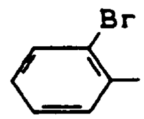 |
| $CH_3$ | $C(CH_3)_3$ | 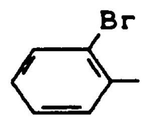 |
| $C_2H_5$ | $CH_3$ | 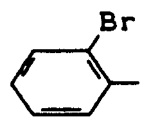 |
| $C_3H_7$ | $CH_3$ | 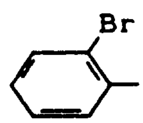 |
| $CH(CH_3)_2$ | $CH_3$ | 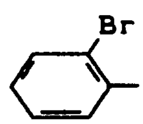 |

Tabelle 1 - Fortsetzung

| $R^1$ | $R^2$ | $R^3$ |
|---|---|---|
| $C_4H_9$ | $CH_3$ | 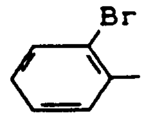 |
| $C_2H_5$ | $C_2H_5$ | 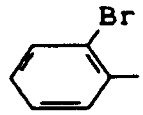 |
| $CHF_2$ | $CH_3$ | 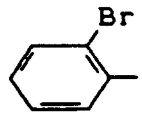 |
| $CHF_2$ | $C_2H_5$ | 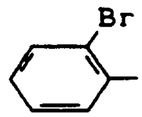 |
| $CH_3$ | $CF_3$ | 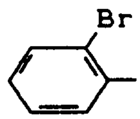 |
| $C_2H_5$ | $CF_3$ | 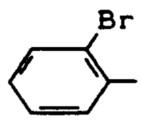 |
| $-(CH_2)_3-$ | | 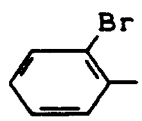 |
| $-(CH_2)_4-$ | | 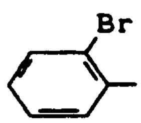 |

Tabelle 1 - Fortsetzung

| $R^1$ | $R^2$ | $R^3$ |
|---|---|---|
| $-(CH_2)_5-$ | | 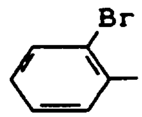 |
| $CH_3$ | $CH_3$ | 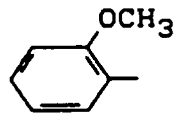 |
| H | $CH_3$ | 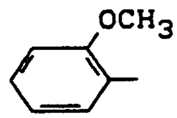 |
| $CH_3$ | H | 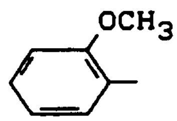 |
| $CH_3$ | H | 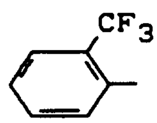 |
| $C_2H_5$ | H | 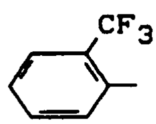 |
| $C_3H_7$ | H | 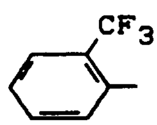 |
| $CH(CH_3)_2$ | H | 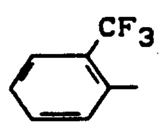 |

Tabelle 1 - Fortsetzung

| $R^1$ | $R^2$ | $R^3$ |
|---|---|---|
| $C_4H_9$ | H | 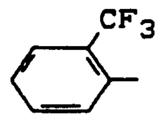 |
| $CH_2CH(CH_3)_2$ | H | 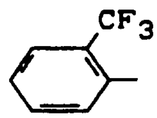 |
| $C(CH_3)_3$ | H | 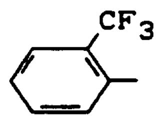 |
| H | $CH_3$ | 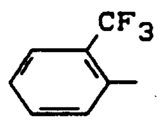 |
| H | $C_2H_5$ | 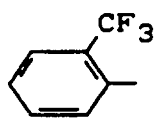 |
| H | $C_3H_7$ | 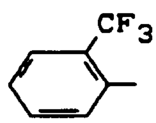 |
| H | $CH(CH_3)_2$ | 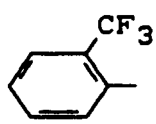 |
| H | $C_4H_9$ | 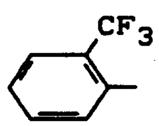 |

Tabelle 1 - Fortsetzung

| $R^1$ | $R^2$ | $R^3$ |
|---|---|---|
| H | $CH_2CH(CH_3)_2$ | 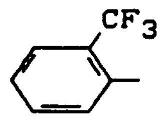 |
| H | $C(CH_3)_3$ | 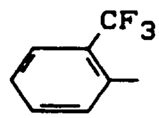 |
| $CHF_2$ | H | 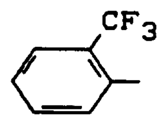 |
| $CH_2CH_2CN$ | H | 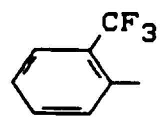 |
| $CH_2CH_2OCH_3$ | H | 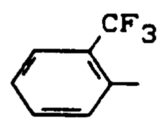 |
| H | $CF_3$ | 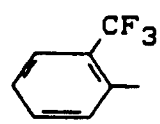 |
| $CH_2OCH_3$ | H | 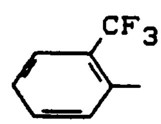 |
| H | $CH_2OCH_3$ | 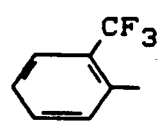 |

<u>Tabelle 1</u> - Fortsetzung

| $R^1$ | $R^2$ | $R^3$ |
|---|---|---|
| H | $CH_2CH_2OCH_3$ | 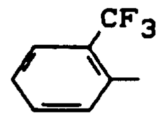 |
| $CH_3$ | $CH_3$ | 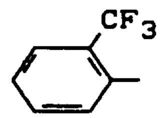 |
| $CH_3$ | $C_2H_5$ | 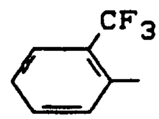 |
| $CH_3$ | $C_3H_7$ | 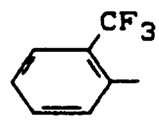 |
| $CH_3$ | $CH(CH_3)_2$ | 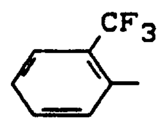 |
| $CH_3$ | $C_4H_9$ | 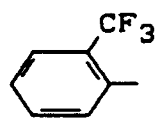 |
| $CH_3$ | $CH_2CH(CH_3)_2$ | 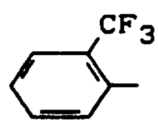 |
| $CH_3$ | $C(CH_3)_3$ | 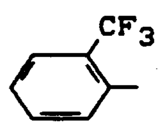 |

Tabelle 1 - Fortsetzung

| $R^1$ | $R^2$ | $R^3$ |
|---|---|---|
| $C_2H_5$ | $CH_3$ | 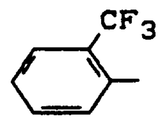 |
| $C_3H_7$ | $CH_3$ | 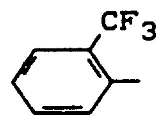 |
| $CH(CH_3)_2$ | $CH_3$ | 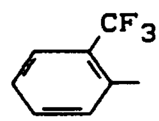 |
| $C_4H_9$ | $CH_3$ | 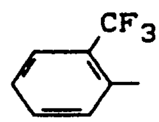 |
| $C_2H_5$ | $C_2H_5$ | 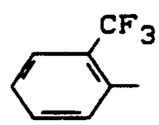 |
| $CHF_2$ | $CH_3$ | 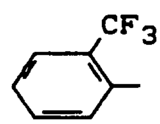 |
| $CHF_2$ | $C_2H_5$ | 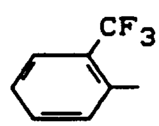 |
| $CH_3$ | $CF_3$ | 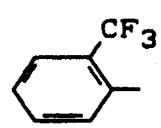 |

Tabelle 1 - Fortsetzung

| $R^1$ | $R^2$ | $R^3$ |
|---|---|---|
| $C_2H_5$ | $CF_3$ | 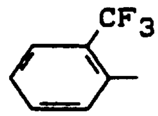 |
| $-(CH_2)_3-$ | | 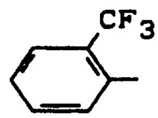 |
| $-(CH_2)_4-$ | | 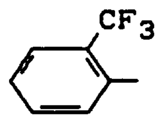 |
| $-(CH_2)_5-$ | | 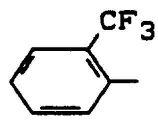 |
| $CH_3$ | $CH_3$ | 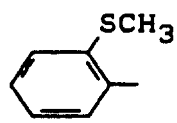 |
| H | $CH_3$ | 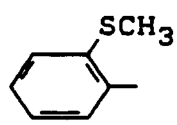 |
| $CH_3$ | H | 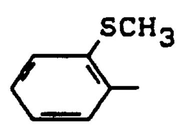 |
| $CH_3$ | H | 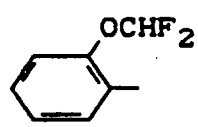 |

Tabelle 1 - Fortsetzung

| $R^1$ | $R^2$ | $R^3$ |
|---|---|---|
| $C_2H_5$ | H | 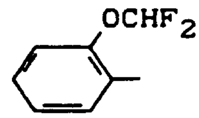 |
| $C_3H_7$ | H | 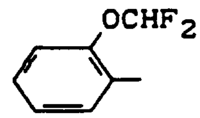 |
| $CH(CH_3)_2$ | H | 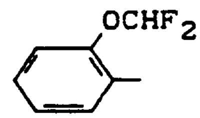 |
| $C_4H_9$ | H | 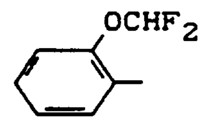 |
| $CH_2CH(CH_3)_2$ | H | 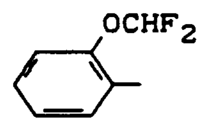 |
| $C(CH_3)_3$ | H | 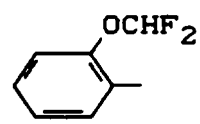 |
| H | $CH_3$ | 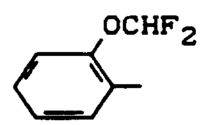 |
| H | $C_2H_5$ | 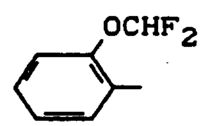 |

Tabelle 1 - Fortsetzung

| $R^1$ | $R^2$ | $R^3$ |
|---|---|---|
| H | $C_3H_7$ | 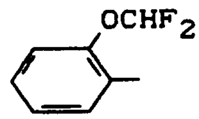 |
| H | $CH(CH_3)_2$ | 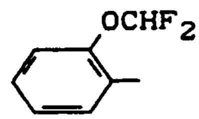 |
| H | $C_4H_9$ | 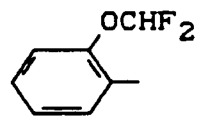 |
| H | $CH_2CH(CH_3)_2$ | 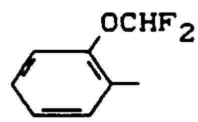 |
| H | $C(CH_3)_3$ | 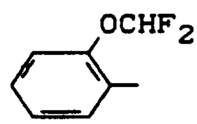 |
| $CHF_2$ | H | 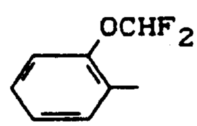 |
| $CH_2CH_2CN$ | H | 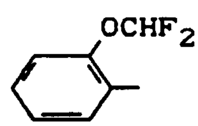 |
| $CH_2CH_2OCH_3$ | H | 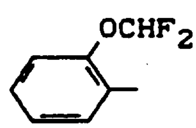 |

Tabelle 1 - Fortsetzung

| $R^1$ | $R^2$ | $R^3$ |
|---|---|---|
| H | $CF_3$ | 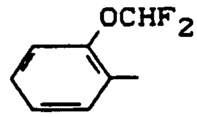 |
| $CH_2OCH_3$ | H | 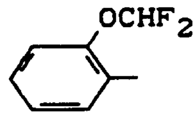 |
| H | $CH_2OCH_3$ | 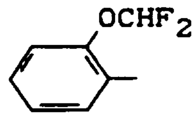 |
| H | $CH_2CH_2OCH_3$ | 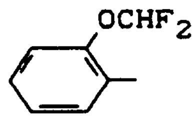 |
| $CH_3$ | $CH_3$ | 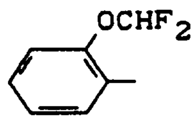 |
| $CH_3$ | $C_2H_5$ | 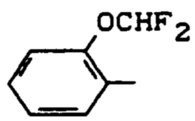 |
| $CH_3$ | $C_3H_7$ | 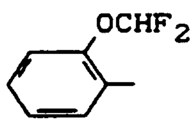 |
| $CH_3$ | $CH(CH_3)_2$ | 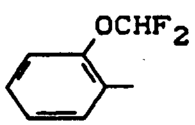 |

Tabelle 1 - Fortsetzung

| $R^1$ | $R^2$ | $R^3$ |
|---|---|---|
| $CH_3$ | $C_4H_9$ | 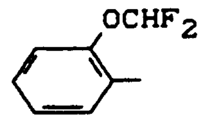 |
| $CH_3$ | $CH_2CH(CH_3)_2$ | 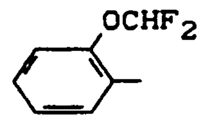 |
| $CH_3$ | $C(CH_3)_3$ | 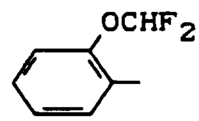 |
| $C_2H_5$ | $CH_3$ | 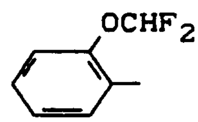 |
| $C_3H_7$ | $CH_3$ | 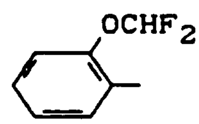 |
| $CH(CH_3)_2$ | $CH_3$ | 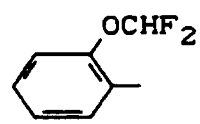 |
| $C_4H_9$ | $CH_3$ | 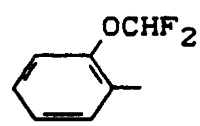 |
| $C_2H_5$ | $C_2H_5$ | 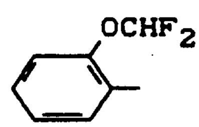 |

Tabelle 1 - Fortsetzung

| $R^1$ | $R^2$ | $R^3$ |
|---|---|---|
| $CHF_2$ | $CH_3$ | 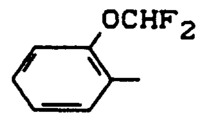 |
| $CHF_2$ | $C_2H_5$ | 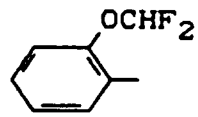 |
| $CH_3$ | $CF_3$ | 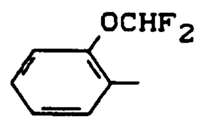 |
| $C_2H_5$ | $CF_3$ | 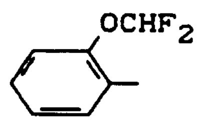 |
| $-(CH_2)_3-$ | | 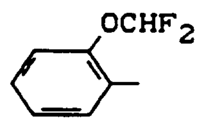 |
| $-(CH_2)_4-$ | | 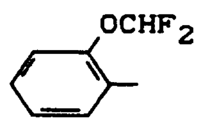 |
| $-(CH_2)_5-$ | | 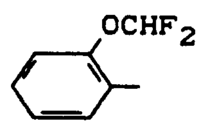 |
| $CH_3$ | $CH_3$ | 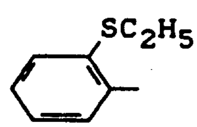 |

Tabelle 1 - Fortsetzung

| $R^1$ | $R^2$ | $R^3$ |
|---|---|---|
| H | $CH_3$ | 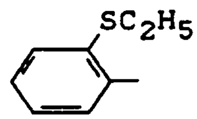 |
| $CH_3$ | H | 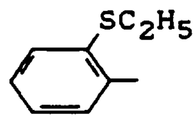 |
| $CH_3$ | H | 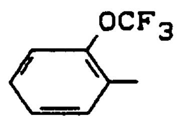 |
| $C_2H_5$ | H | 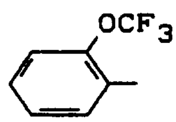 |
| $C_3H_7$ | H | 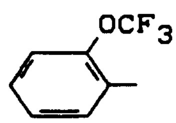 |
| $CH(CH_3)_2$ | H | 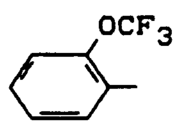 |
| $C_4H_9$ | H | 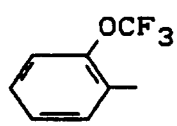 |
| $CH_2CH(CH_3)_2$ | H | 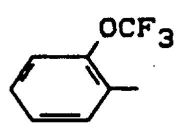 |

<u>Tabelle 1</u> - Fortsetzung

| $R^1$ | $R^2$ | $R^3$ |
|---|---|---|
| $C(CH_3)_3$ | H | 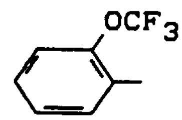 |
| H | $CH_3$ | 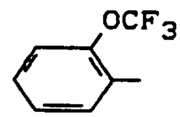 |
| H | $C_2H_5$ | 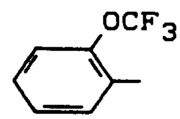 |
| H | $C_3H_7$ | 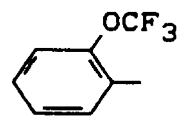 |
| H | $CH(CH_3)_2$ | 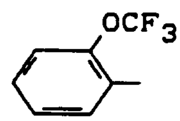 |
| H | $C_4H_9$ | 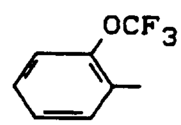 |
| H | $CH_2CH(CH_3)_2$ | 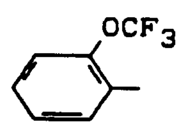 |
| H | $C(CH_3)_3$ | 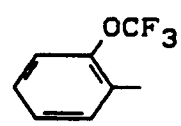 |

Tabelle 1 - Fortsetzung

| $R^1$ | $R^2$ | $R^3$ |
|---|---|---|
| $CHF_2$ | H | 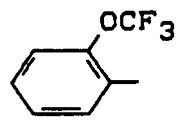 |
| $CH_2CH_2CN$ | H | 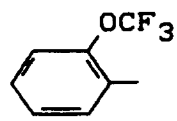 |
| $CH_2CH_2OCH_3$ | H | 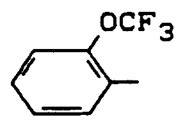 |
| H | $CF_3$ | 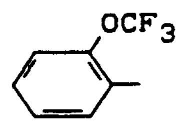 |
| $CH_2OCH_3$ | H | 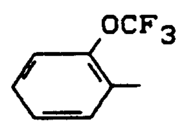 |
| H | $CH_2OCH_3$ | 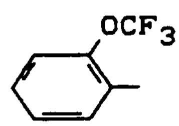 |
| H | $CH_2CH_2OCH_3$ | 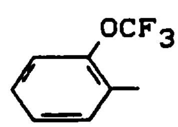 |
| $CH_3$ | $CH_3$ | 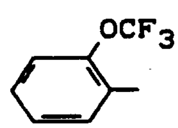 |

Tabelle 1 - Fortsetzung

| $R^1$ | $R^2$ | $R^3$ |
|---|---|---|
| $CH_3$ | $C_2H_5$ | 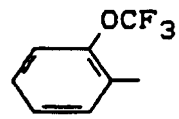 |
| $CH_3$ | $C_3H_7$ | 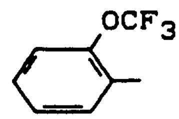 |
| $CH_3$ | $CH(CH_3)_2$ | 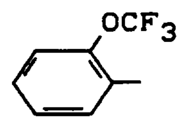 |
| $CH_3$ | $C_4H_9$ | 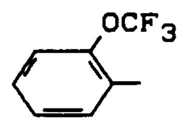 |
| $CH_3$ | $CH_2CH(CH_3)_2$ | 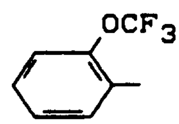 |
| $CH_3$ | $C(CH_3)_3$ | 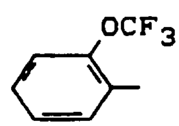 |
| $C_2H_5$ | $CH_3$ | 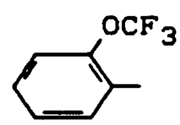 |
| $C_3H_7$ | $CH_3$ | 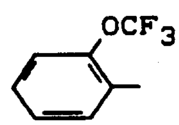 |

Tabelle 1 - Fortsetzung

| $R^1$ | $R^2$ | $R^3$ |
|---|---|---|
| $CH(CH_3)_2$ | $CH_3$ | 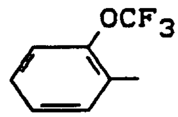 |
| $C_4H_9$ | $CH_3$ | 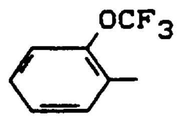 |
| $C_2H_5$ | $C_2H_5$ | 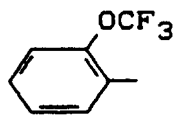 |
| $CHF_2$ | $CH_3$ | 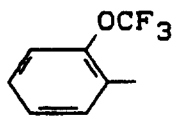 |
| $CHF_2$ | $C_2H_5$ | 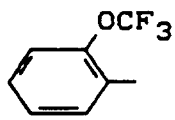 |
| $CH_3$ | $CF_3$ | 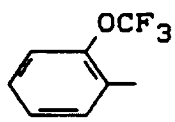 |
| $C_2H_5$ | $CF_3$ | 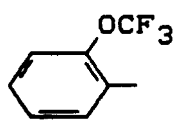 |
| $-(CH_2)_3-$ | | 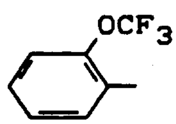 |

Tabelle 1 - Fortsetzung

| $R^1$ | $R^2$ | $R^3$ |
|---|---|---|
| $-(CH_2)_4-$ | | 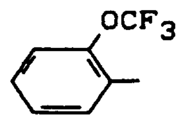 |
| $-(CH_2)_5-$ | | 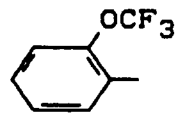 |
| $CH_3$ | $CH_3$ | 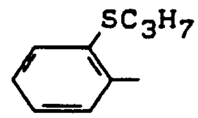 |
| H | $CH_3$ | 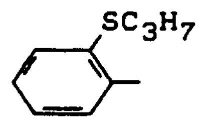 |
| $CH_3$ | H | 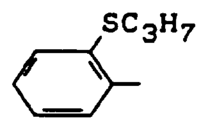 |
| $CH_3$ | H | 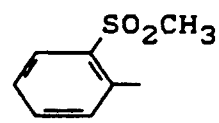 |
| $C_2H_5$ | H | 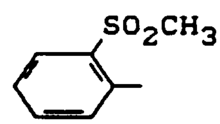 |
| $C_3H_7$ | H | 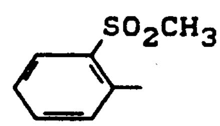 |

Tabelle 1 - Fortsetzung

| $R^1$ | $R^2$ | $R^3$ |
|---|---|---|
| $CH(CH_3)_2$ | H | 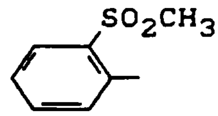 |
| $C_4H_9$ | H | 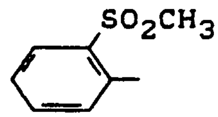 |
| $CH_2CH(CH_3)_2$ | H | 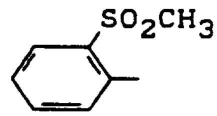 |
| $C(CH_3)_3$ | H | 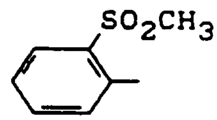 |
| H | $CH_3$ | 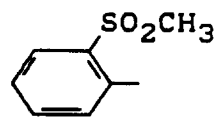 |
| H | $C_2H_5$ | 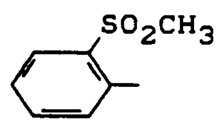 |
| H | $C_3H_7$ | 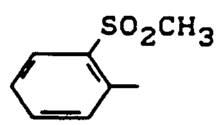 |
| H | $CH(CH_3)_2$ | 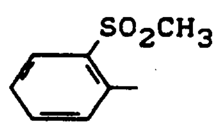 |

Tabelle 1 - Fortsetzung

| $R^1$ | $R^2$ | $R^3$ |
|---|---|---|
| H | $C_4H_9$ | 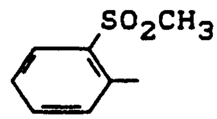 |
| H | $CH_2CH(CH_3)_2$ | 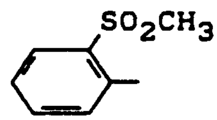 |
| H | $C(CH_3)_3$ | 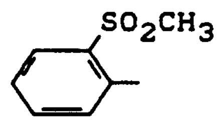 |
| $CHF_2$ | H | 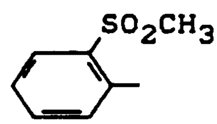 |
| $CH_2CH_2CN$ | H | 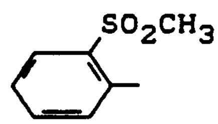 |
| $CH_2CH_2OCH_3$ | H | 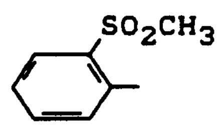 |
| H | $CF_3$ | 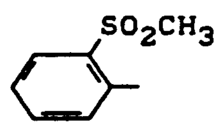 |
| $CH_2OCH_3$ | H | 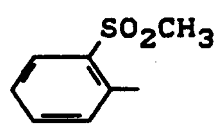 |

Tabelle 1 - Fortsetzung

| $R^1$ | $R^2$ | $R^3$ |
|---|---|---|
| H | $CH_2OCH_3$ | 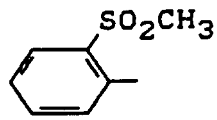 |
| H | $CH_2CH_2OCH_3$ | 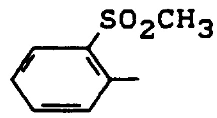 |
| $CH_3$ | $CH_3$ | 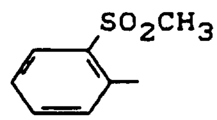 |
| $CH_3$ | $C_2H_5$ | 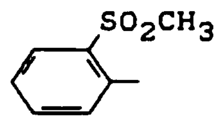 |
| $CH_3$ | $C_3H_7$ | 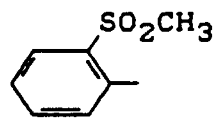 |
| $CH_3$ | $CH(CH_3)_2$ | 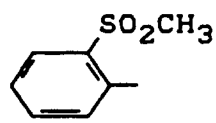 |
| $CH_3$ | $C_4H_9$ | 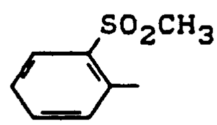 |
| $CH_3$ | $CH_2CH(CH_3)_2$ | 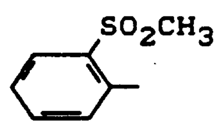 |

Tabelle 1 - Fortsetzung

| $R^1$ | $R^2$ | $R^3$ |
|---|---|---|
| $CH_3$ | $C(CH_3)_3$ | 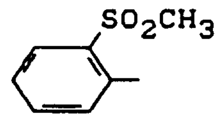 |
| $C_2H_5$ | $CH_3$ | 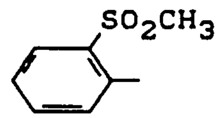 |
| $C_3H_7$ | $CH_3$ | 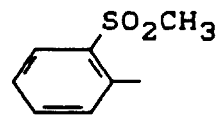 |
| $CH(CH_3)_2$ | $CH_3$ | 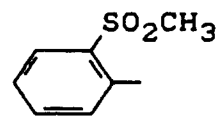 |
| $C_4H_9$ | $CH_3$ | 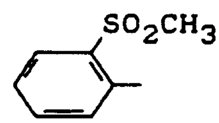 |
| $C_2H_5$ | $C_2H_5$ | 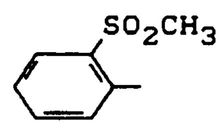 |
| $CHF_2$ | $CH_3$ | 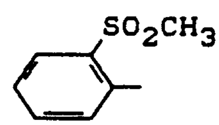 |
| $CHF_2$ | $C_2H_5$ | 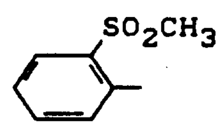 |

Tabelle 1 - Fortsetzung

| $R^1$ | $R^2$ | $R^3$ |
|---|---|---|
| $CH_3$ | $CF_3$ | 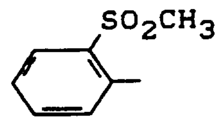 |
| $C_2H_5$ | $CF_3$ | 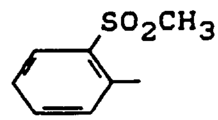 |
| $-(CH_2)_3-$ | | 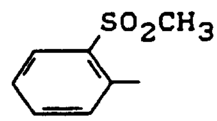 |
| $-(CH_2)_4-$ | | 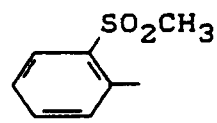 |
| $-(CH_2)_5-$ | | 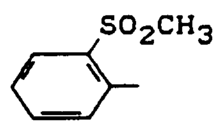 |
| $CH_3$ | $CH_3$ | 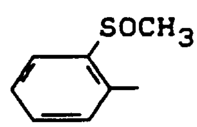 |
| H | $CH_3$ | 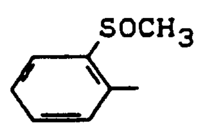 |
| $CH_3$ | H | 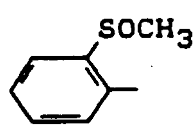 |

Tabelle 1 - Fortsetzung

| $R^1$ | $R^2$ | $R^3$ |
|---|---|---|
| $CH_3$ | H | 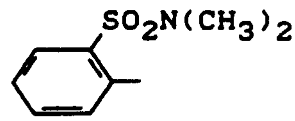 |
| $C_2H_5$ | H | 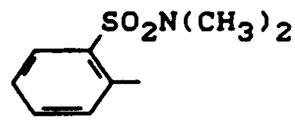 |
| $C_3H_7$ | H | 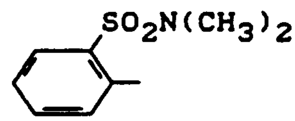 |
| $CH(CH_3)_2$ | H | 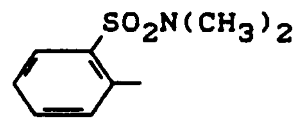 |
| $C_4H_9$ | H | 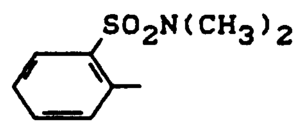 |
| $CH_2CH(CH_3)_2$ | H | 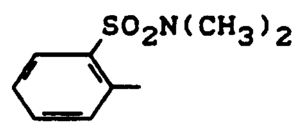 |
| $C(CH_3)_3$ | H | 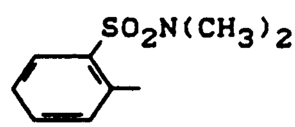 |
| H | $CH_3$ | 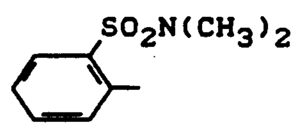 |

<u>Tabelle 1</u> - Fortsetzung

| $R^1$ | $R^2$ | $R^3$ |
|---|---|---|
| H | $C_2H_5$ | 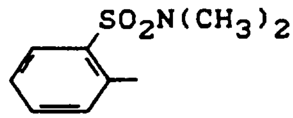 |
| H | $C_3H_7$ | 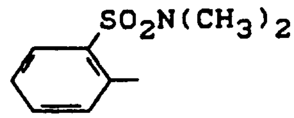 |
| H | $CH(CH_3)_2$ | 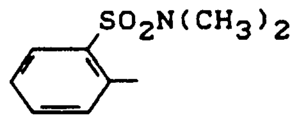 |
| H | $C_4H_9$ | 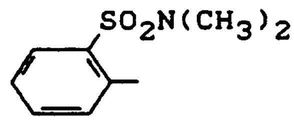 |
| H | $CH_2CH(CH_3)_2$ | 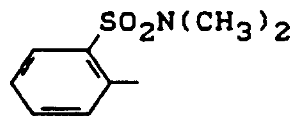 |
| H | $C(CH_3)_3$ | 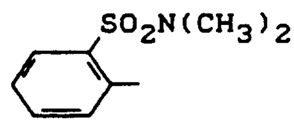 |
| $CHF_2$ | H | 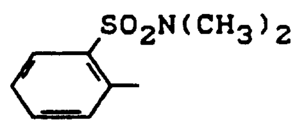 |
| $CH_2CH_2CN$ | H | 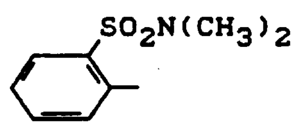 |

<u>Tabelle 1</u> - Fortsetzung

| $R^1$ | $R^2$ | $R^3$ |
|---|---|---|
| $CH_2CH_2OCH_3$ | H | 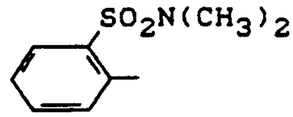 |
| H | $CF_3$ | 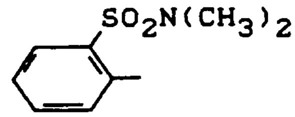 |
| $CH_2OCH_3$ | H | 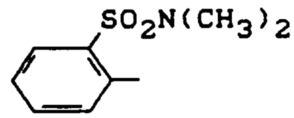 |
| H | $CH_2OCH_3$ | 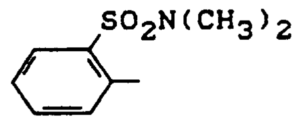 |
| H | $CH_2CH_2OCH_3$ | 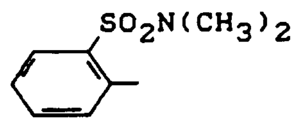 |
| $CH_3$ | $CH_3$ | 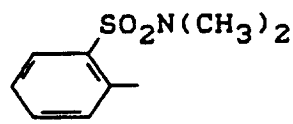 |
| $CH_3$ | $C_2H_5$ | 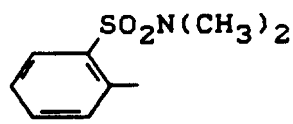 |
| $CH_3$ | $C_3H_7$ | 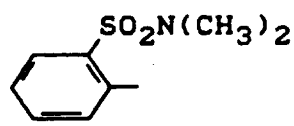 |

Tabelle 1 - Fortsetzung

| $R^1$ | $R^2$ | $R^3$ |
|---|---|---|
| $CH_3$ | $CH(CH_3)_2$ | 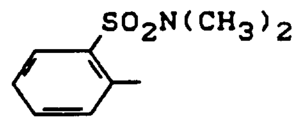 |
| $CH_3$ | $C_4H_9$ | 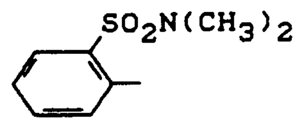 |
| $CH_3$ | $CH_2CH(CH_3)_2$ | 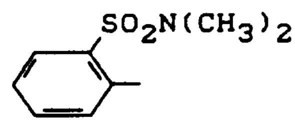 |
| $CH_3$ | $C(CH_3)_3$ | 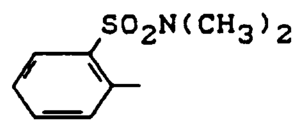 |
| $C_2H_5$ | $CH_3$ | 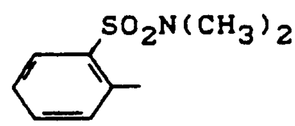 |
| $C_3H_7$ | $CH_3$ | 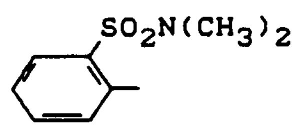 |
| $CH(CH_3)_2$ | $CH_3$ | 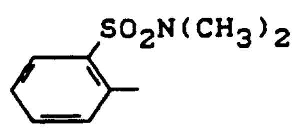 |
| $C_4H_9$ | $CH_3$ | 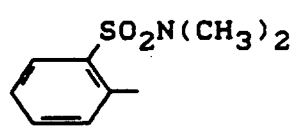 |

<u>Tabelle 1</u> - Fortsetzung

| $R^1$ | $R^2$ | $R^3$ |
|---|---|---|
| $C_2H_5$ | $C_2H_5$ | 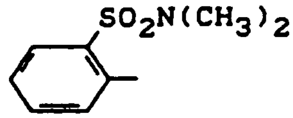 |
| $CHF_2$ | $CH_3$ | 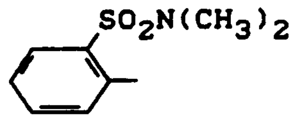 |
| $CHF_2$ | $C_2H_5$ | 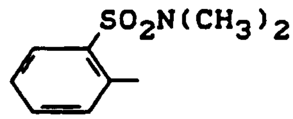 |
| $CH_3$ | $CF_3$ | 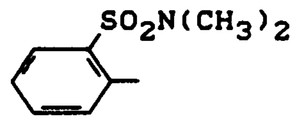 |
| $C_2H_5$ | $CF_3$ | 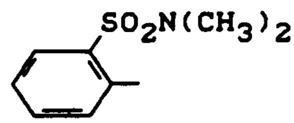 |
| $-(CH_2)_3-$ | | 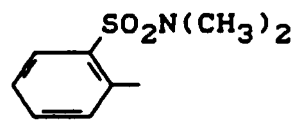 |
| $-(CH_2)_4-$ | | 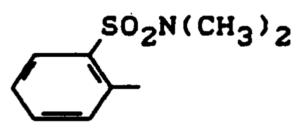 |
| $-(CH_2)_5-$ | | 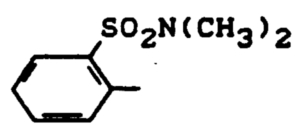 |

Tabelle 1 - Fortsetzung

| $R^1$ | $R^2$ | $R^3$ |
|---|---|---|
| $CH_3$ | $CH_3$ | 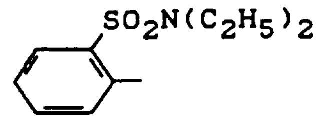 |
| H | $CH_3$ | 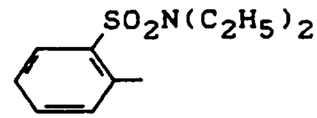 |
| $CH_3$ | H | 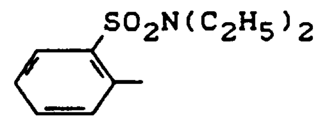 |
| $CH_3$ | H | 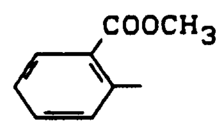 |
| $C_2H_5$ | H | 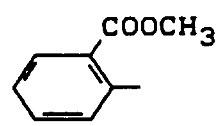 |
| $C_3H_7$ | H | 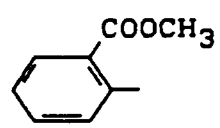 |
| $CH(CH_3)_2$ | H | 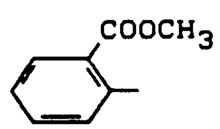 |
| $C_4H_9$ | H | 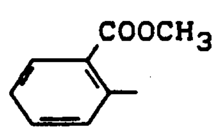 |

Tabelle 1 - Fortsetzung

| $R^1$ | $R^2$ | $R^3$ |
|---|---|---|
| $CH_2CH(CH_3)_2$ | H | 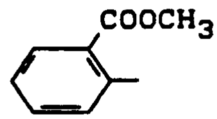 |
| $C(CH_3)_3$ | H | 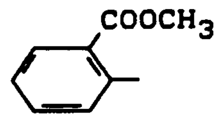 |
| H | $CH_3$ | 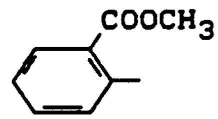 |
| H | $C_2H_5$ | 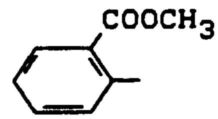 |
| H | $C_3H_7$ | 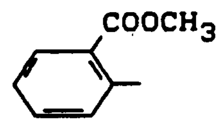 |
| H | $CH(CH_3)_2$ | 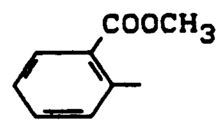 |
| H | $C_4H_9$ | 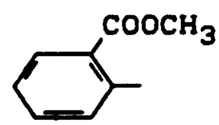 |
| H | $CH_2CH(CH_3)_2$ | 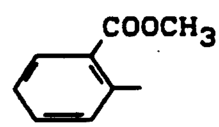 |

Tabelle 1 - Fortsetzung

| $R^1$ | $R^2$ | $R^3$ |
|---|---|---|
| H | $C(CH_3)_3$ | 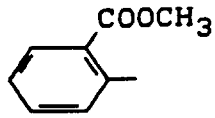 |
| $CHF_2$ | H | 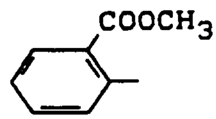 |
| $CH_2CH_2CN$ | H | 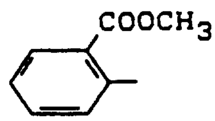 |
| $CH_2CH_2OCH_3$ | H | 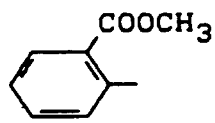 |
| H | $CF_3$ | 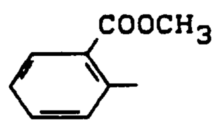 |
| $CH_2OCH_3$ | H | 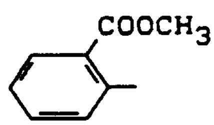 |
| H | $CH_2OCH_3$ | 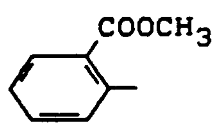 |
| H | $CH_2CH_2OCH_3$ | 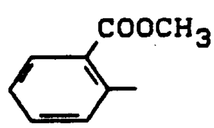 |

Tabelle 1 - Fortsetzung

| $R^1$ | $R^2$ | $R^3$ |
|---|---|---|
| $CH_3$ | $CH_3$ | 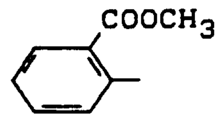 |
| $CH_3$ | $C_2H_5$ | 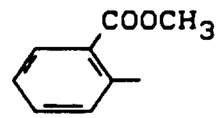 |
| $CH_3$ | $C_3H_7$ | 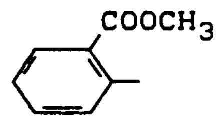 |
| $CH_3$ | $CH(CH_3)_2$ | 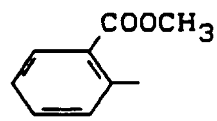 |
| $CH_3$ | $C_4H_9$ | 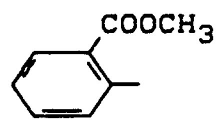 |
| $CH_3$ | $CH_2CH(CH_3)_2$ | 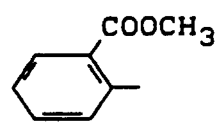 |
| $CH_3$ | $C(CH_3)_3$ | 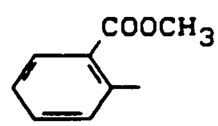 |
| $C_2H_5$ | $CH_3$ | 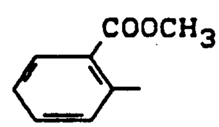 |

Tabelle 1 - Fortsetzung

| $R^1$ | $R^2$ | $R^3$ |
|---|---|---|
| $C_3H_7$ | $CH_3$ | 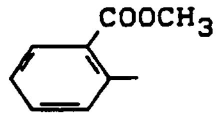 |
| $CH(CH_3)_2$ | $CH_3$ | 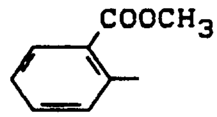 |
| $C_4H_9$ | $CH_3$ | 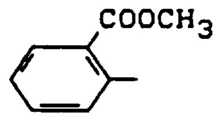 |
| $C_2H_5$ | $C_2H_5$ | 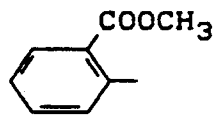 |
| $CHF_2$ | $CH_3$ | 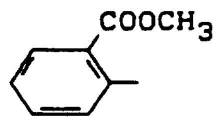 |
| $CHF_2$ | $C_2H_5$ | 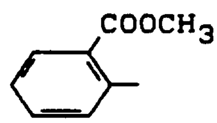 |
| $CH_3$ | $CF_3$ | 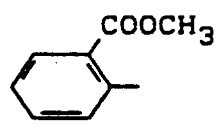 |
| $C_2H_5$ | $CF_3$ | 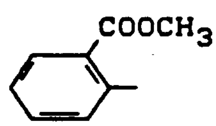 |

Tabelle 1 - Fortsetzung

| $R^1$ | $R^2$ | $R^3$ |
|---|---|---|
| $-(CH_2)_3-$ | | 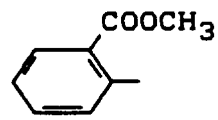 |
| $-(CH_2)_4-$ | | 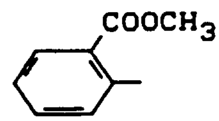 |
| $-(CH_2)_5-$ | | 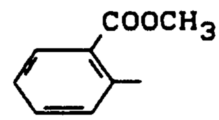 |
| $CH_3$ | $CH_3$ | 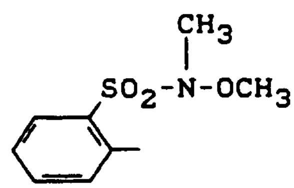 |
| H | $CH_3$ | 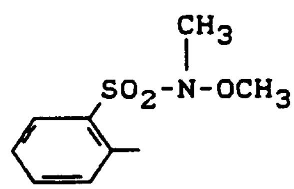 |
| $CH_3$ | H | 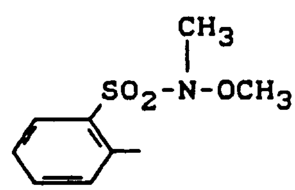 |
| $CH_3$ | H | 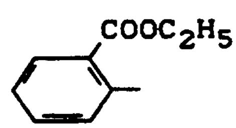 |

<u>Tabelle 1</u> - Fortsetzung

| $R^1$ | $R^2$ | $R^3$ |
|---|---|---|
| $C_2H_5$ | H | 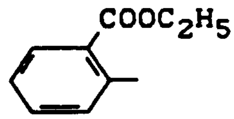 |
| $C_3H_7$ | H | 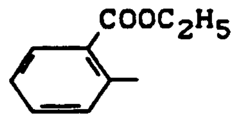 |
| $CH(CH_3)_2$ | H | 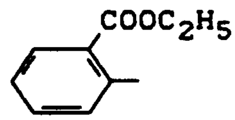 |
| $C_4H_9$ | H | 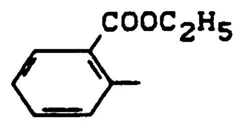 |
| $CH_2CH(CH_3)_2$ | H | 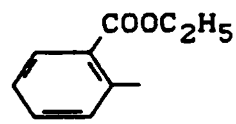 |
| $C(CH_3)_3$ | H | 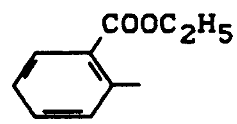 |
| H | $CH_3$ | 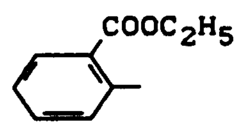 |
| H | $C_2H_5$ | 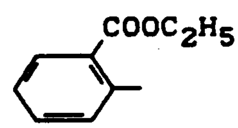 |

Tabelle 1 - Fortsetzung

| $R^1$ | $R^2$ | $R^3$ |
|---|---|---|
| H | $C_3H_7$ | 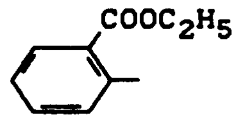 |
| H | $CH(CH_3)_2$ | 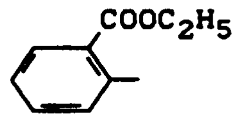 |
| H | $C_4H_9$ | 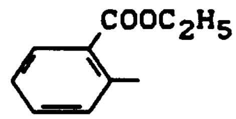 |
| H | $CH_2CH(CH_3)_2$ | 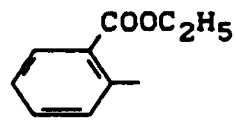 |
| H | $C(CH_3)_3$ | 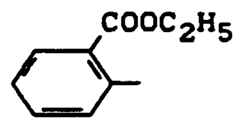 |
| $CHF_2$ | H | 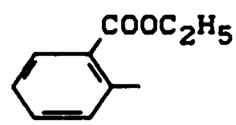 |
| $CH_2CH_2CN$ | H | 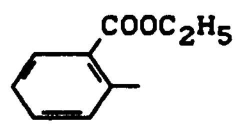 |
| $CH_2CH_2OCH_3$ | H | 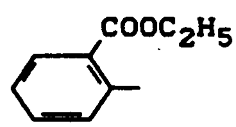 |

<u>Tabelle 1</u> - Fortsetzung

| $R^1$ | $R^2$ | $R^3$ |
|---|---|---|
| H | $CF_3$ | 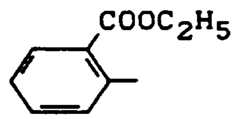 |
| $CH_2OCH_3$ | H | 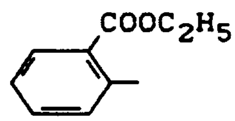 |
| H | $CH_2OCH_3$ | 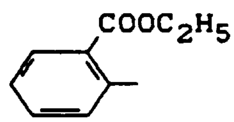 |
| H | $CH_2CH_2OCH_3$ | 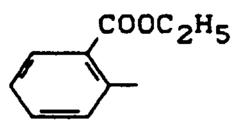 |
| $CH_3$ | $CH_3$ | 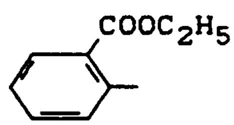 |
| $CH_3$ | $C_2H_5$ | 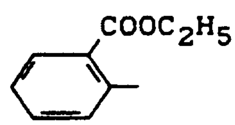 |
| $CH_3$ | $C_3H_7$ | 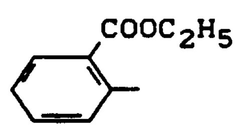 |
| $CH_3$ | $CH(CH_3)_2$ | 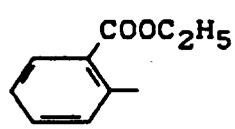 |

**Tabelle 1** - Fortsetzung

| $R^1$ | $R^2$ | $R^3$ |
|---|---|---|
| $CH_3$ | $C_4H_9$ | 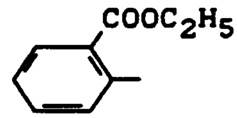 |
| $CH_3$ | $CH_2CH(CH_3)_2$ | 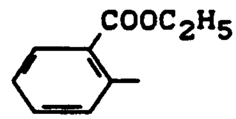 |
| $CH_3$ | $C(CH_3)_3$ | 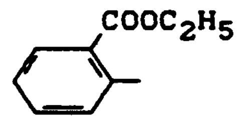 |
| $C_2H_5$ | $CH_3$ | 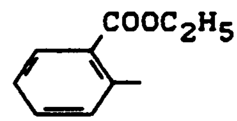 |
| $C_3H_7$ | $CH_3$ | 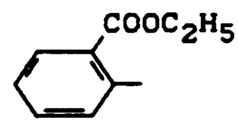 |
| $CH(CH_3)_2$ | $CH_3$ | 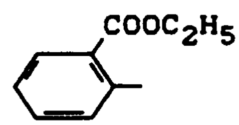 |
| $C_4H_9$ | $CH_3$ | 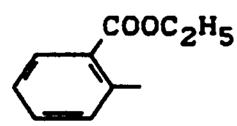 |
| $C_2H_5$ | $C_2H_5$ | 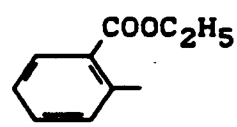 |

Tabelle 1 - Fortsetzung

| $R^1$ | $R^2$ | $R^3$ |
|---|---|---|
| $CHF_2$ | $CH_3$ | 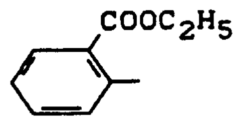 |
| $CHF_2$ | $C_2H_5$ | 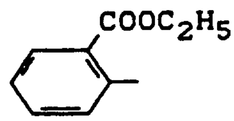 |
| $CH_3$ | $CF_3$ | 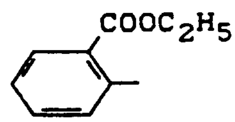 |
| $C_2H_5$ | $CF_3$ | 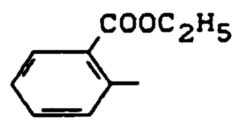 |
| $-(CH_2)_3-$ | | 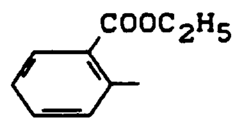 |
| $-(CH_2)_4-$ | | 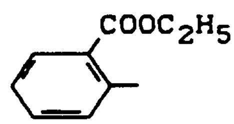 |
| $-(CH_2)_5-$ | | 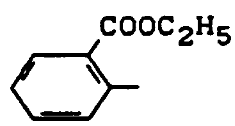 |
| $CH_3$ | $CH_3$ | 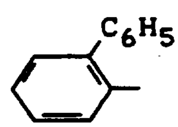 |

Tabelle 1 - Fortsetzung

| $R^1$ | $R^2$ | $R^3$ |
|---|---|---|
| H | $CH_3$ | 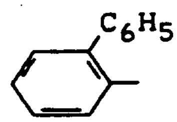 |
| $CH_3$ | H | 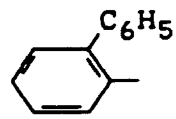 |
| $CH_3$ | H | 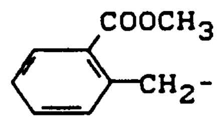 |
| $C_2H_5$ | H | 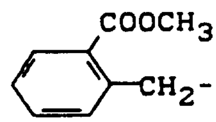 |
| $C_3H_7$ | H | 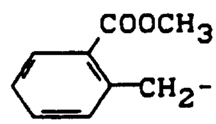 |
| $CH(CH_3)_2$ | H | 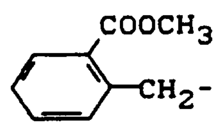 |
| $C_4H_9$ | H | 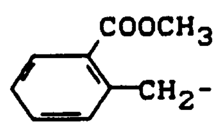 |
| $CH_2CH(CH_3)_2$ | H | 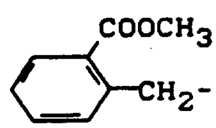 |

Tabelle 1 - Fortsetzung

| $R^1$ | $R^2$ | $R^3$ |
|---|---|---|
| $C(CH_3)_3$ | H | 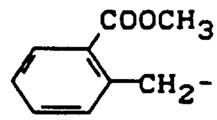 |
| H | $CH_3$ | 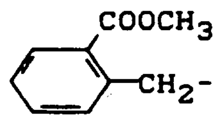 |
| H | $C_2H_5$ | 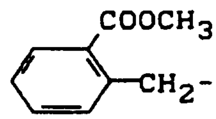 |
| H | $C_3H_7$ | 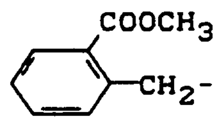 |
| H | $CH(CH_3)_2$ | 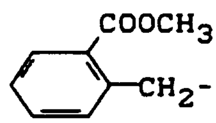 |
| H | $C_4H_9$ | 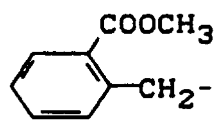 |
| H | $CH_2CH(CH_3)_2$ | 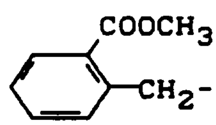 |
| H | $C(CH_3)_3$ | 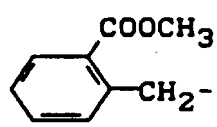 |

<u>Tabelle 1</u> - Fortsetzung

| $R^1$ | $R^2$ | $R^3$ |
|---|---|---|
| $CHF_2$ | H | 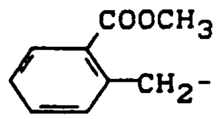 |
| $CH_2CH_2CN$ | H | 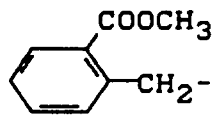 |
| $CH_2CH_2OCH_3$ | H | 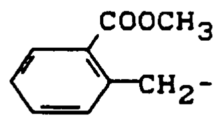 |
| H | $CF_3$ | 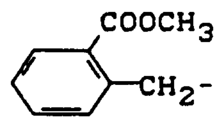 |
| $CH_2OCH_3$ | H | 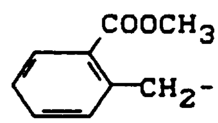 |
| H | $CH_2OCH_3$ | 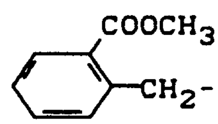 |
| H | $CH_2CH_2OCH_3$ | 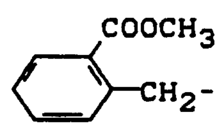 |
| $CH_3$ | $CH_3$ | 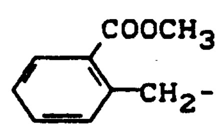 |

Tabelle 1 - Fortsetzung

| $R^1$ | $R^2$ | $R^3$ |
|---|---|---|
| $CH_3$ | $C_2H_5$ | 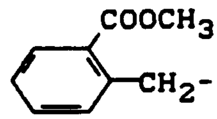 |
| $CH_3$ | $C_3H_7$ | 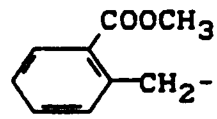 |
| $CH_3$ | $CH(CH_3)_2$ | 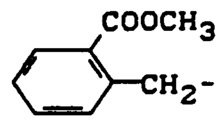 |
| $CH_3$ | $C_4H_9$ | 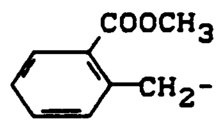 |
| $CH_3$ | $CH_2CH(CH_3)_2$ | 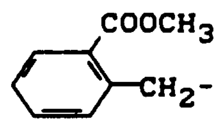 |
| $CH_3$ | $C(CH_3)_3$ | 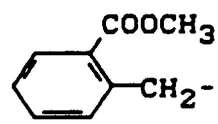 |
| $C_2H_5$ | $CH_3$ | 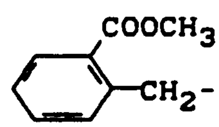 |
| $C_3H_7$ | $CH_3$ | 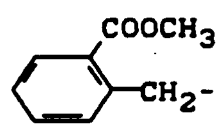 |

Tabelle 1 - Fortsetzung

| $R^1$ | $R^2$ | $R^3$ |
|---|---|---|
| $CH(CH_3)_2$ | $CH_3$ | 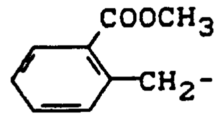 |
| $C_4H_9$ | $CH_3$ | 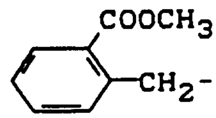 |
| $C_2H_5$ | $C_2H_5$ | 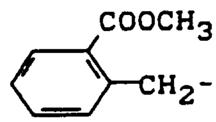 |
| $CHF_2$ | $CH_3$ | 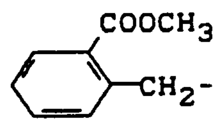 |
| $CHF_2$ | $C_2H_5$ | 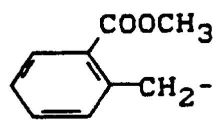 |
| $CH_3$ | $CF_3$ | 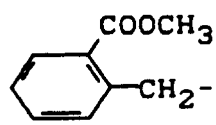 |
| $C_2H_5$ | $CF_3$ | 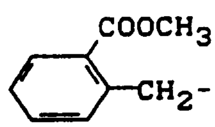 |
| $-(CH_2)_3-$ | | 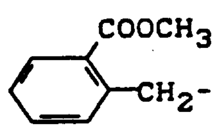 |

**Tabelle 1** - Fortsetzung

| $R^1$ | $R^2$ | $R^3$ |
|---|---|---|
| $-(CH_2)_4-$ | | 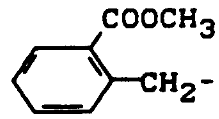 |
| $-(CH_2)_5-$ | | 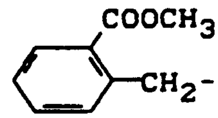 |
| $CH_3$ | $CH_3$ | 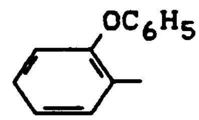 |
| H | $CH_3$ | 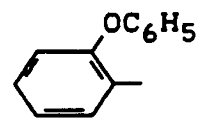 |
| $CH_3$ | H | 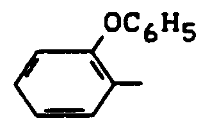 |
| $CH_3$ | H | 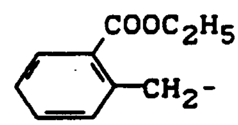 |
| $C_2H_5$ | H | 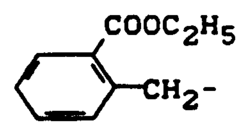 |
| $C_3H_7$ | H | 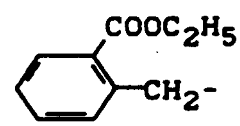 |

Tabelle 1 - Fortsetzung

| $R^1$ | $R^2$ | $R^3$ |
| --- | --- | --- |
| $CH(CH_3)_2$ | H | 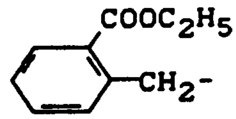 |
| $C_4H_9$ | H | 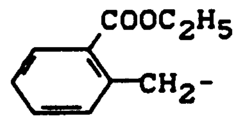 |
| $CH_2CH(CH_3)_2$ | H | 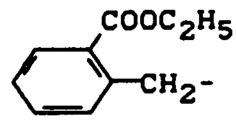 |
| $C(CH_3)_3$ | H | 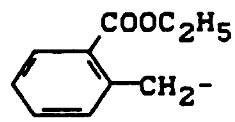 |
| H | $CH_3$ | 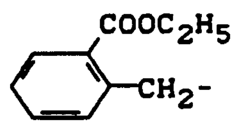 |
| H | $C_2H_5$ | 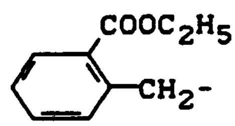 |
| H | $C_3H_7$ | 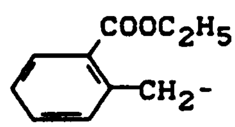 |
| H | $CH(CH_3)_2$ | 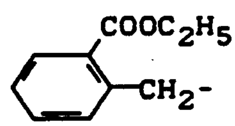 |

<u>Tabelle 1</u> - Fortsetzung

| $R^1$ | $R^2$ | $R^3$ |
|---|---|---|
| H | $C_4H_9$ | 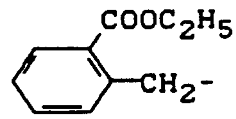 |
| H | $CH_2CH(CH_3)_2$ | 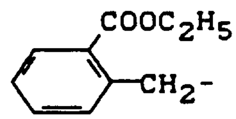 |
| H | $C(CH_3)_3$ | 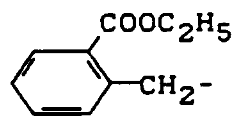 |
| $CHF_2$ | H | 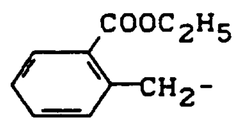 |
| $CH_2CH_2CN$ | H | 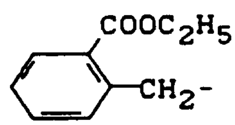 |
| $CH_2CH_2OCH_3$ | H | 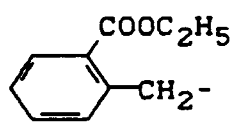 |
| H | $CF_3$ | 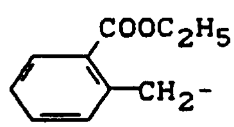 |
| $CH_2OCH_3$ | H | 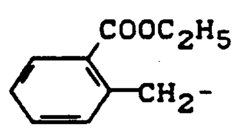 |

<u>Tabelle 1</u> - Fortsetzung

| $R^1$ | $R^2$ | $R^3$ |
|---|---|---|
| H | $CH_2OCH_3$ | 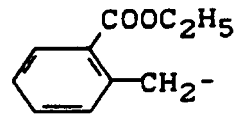 |
| H | $CH_2CH_2OCH_3$ | 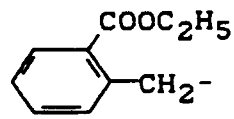 |
| $CH_3$ | $CH_3$ | 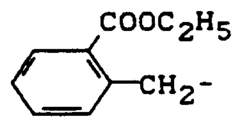 |
| $CH_3$ | $C_2H_5$ | 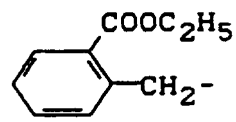 |
| $CH_3$ | $C_3H_7$ | 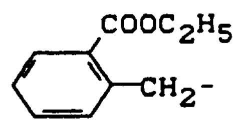 |
| $CH_3$ | $CH(CH_3)_2$ | 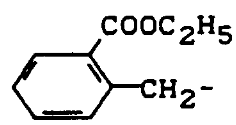 |
| $CH_3$ | $C_4H_9$ | 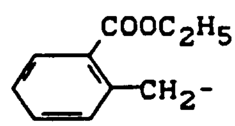 |
| $CH_3$ | $CH_2CH(CH_3)_2$ | 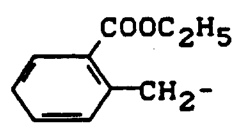 |

Tabelle 1 - Fortsetzung

| $R^1$ | $R^2$ | $R^3$ |
|---|---|---|
| $CH_3$ | $C(CH_3)_3$ | 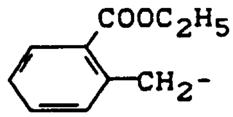 |
| $C_2H_5$ | $CH_3$ | 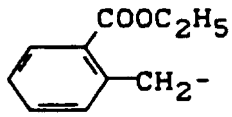 |
| $C_3H_7$ | $CH_3$ | 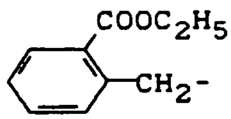 |
| $CH(CH_3)_2$ | $CH_3$ | 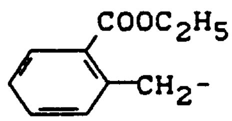 |
| $C_4H_9$ | $CH_3$ | 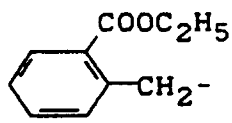 |
| $C_2H_5$ | $C_2H_5$ | 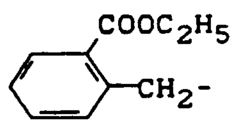 |
| $CHF_2$ | $CH_3$ | 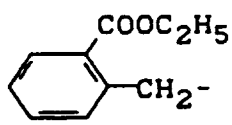 |
| $CHF_2$ | $C_2H_5$ | 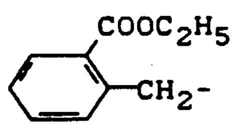 |

Tabelle 1 - Fortsetzung

| $R^1$ | $R^2$ | $R^3$ |
|---|---|---|
| $CH_3$ | $CF_3$ | 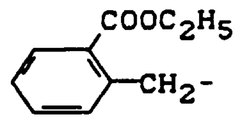 |
| $C_2H_5$ | $CF_3$ | 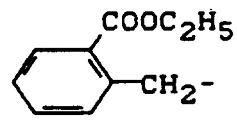 |
| $-(CH_2)_3-$ | | 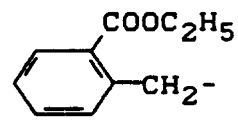 |
| $-(CH_2)_4-$ | | 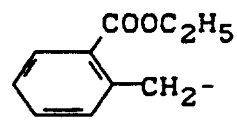 |
| $-(CH_2)_5-$ | | 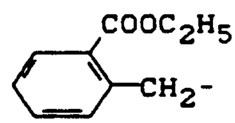 |
| $CH_3$ | $CH_3$ | 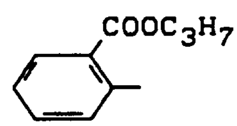 |
| H | $CH_3$ | 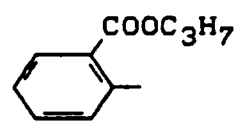 |
| $CH_3$ | H | 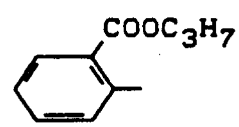 |

Tabelle 1 - Fortsetzung

| $R^1$ | $R^2$ | $R^3$ |
|---|---|---|
| $CH_3$ | H | 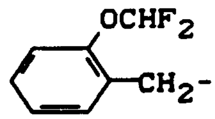 |
| $C_2H_5$ | H | 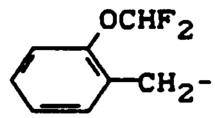 |
| $C_3H_7$ | H | 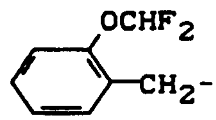 |
| $CH(CH_3)_2$ | H | 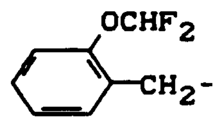 |
| $C_4H_9$ | H | 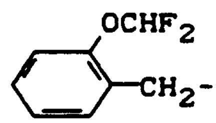 |
| $CH_2CH(CH_3)_2$ | H | 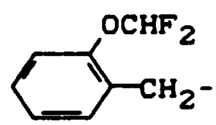 |
| $C(CH_3)_3$ | H | 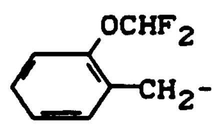 |
| H | $CH_3$ | 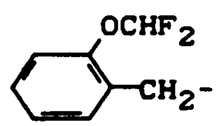 |

Tabelle 1 - Fortsetzung

| $R^1$ | $R^2$ | $R^3$ |
|---|---|---|
| H | $C_2H_5$ | 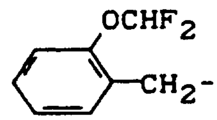 |
| H | $C_3H_7$ | 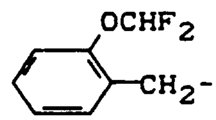 |
| H | $CH(CH_3)_2$ | 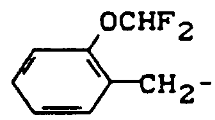 |
| H | $C_4H_9$ | 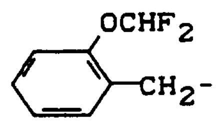 |
| H | $CH_2CH(CH_3)_2$ | 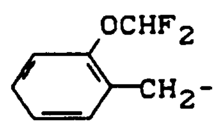 |
| H | $C(CH_3)_3$ | 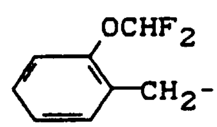 |
| $CHF_2$ | H | 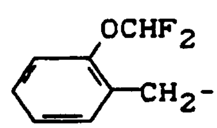 |
| $CH_2CH_2CN$ | H | 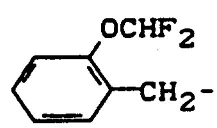 |

Tabelle 1 - Fortsetzung

| $R^1$ | $R^2$ | $R^3$ |
|---|---|---|
| $CH_2CH_2OCH_3$ | H | 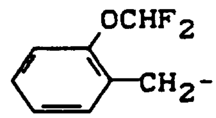 |
| H | $CF_3$ | 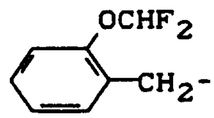 |
| $CH_2OCH_3$ | H | 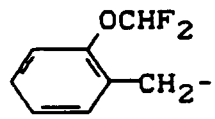 |
| H | $CH_2OCH_3$ | 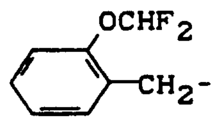 |
| H | $CH_2CH_2OCH_3$ | 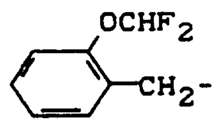 |
| $CH_3$ | $CH_3$ | 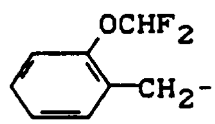 |
| $CH_3$ | $C_2H_5$ | 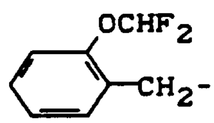 |
| $CH_3$ | $C_3H_7$ | 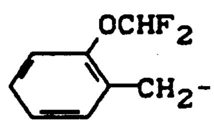 |

Tabelle 1 - Fortsetzung

| $R^1$ | $R^2$ | $R^3$ |
|---|---|---|
| $CH_3$ | $CH(CH_3)_2$ | 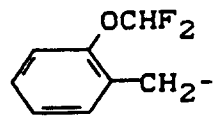 |
| $CH_3$ | $C_4H_9$ | 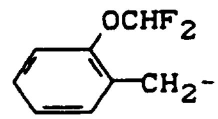 |
| $CH_3$ | $CH_2CH(CH_3)_2$ | 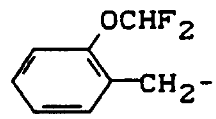 |
| $CH_3$ | $C(CH_3)_3$ | 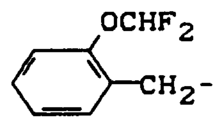 |
| $C_2H_5$ | $CH_3$ | 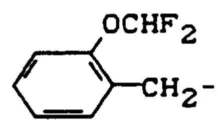 |
| $C_3H_7$ | $CH_3$ | 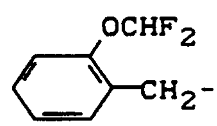 |
| $CH(CH_3)_2$ | $CH_3$ | 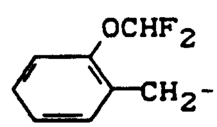 |
| $C_4H_9$ | $CH_3$ | 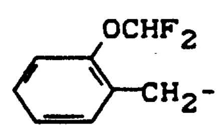 |

Tabelle 1 - Fortsetzung

| $R^1$ | $R^2$ | $R^3$ |
|---|---|---|
| $C_2H_5$ | $C_2H_5$ | 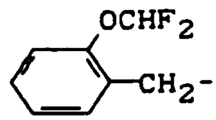 |
| $CHF_2$ | $CH_3$ | 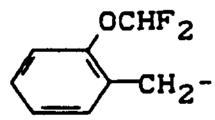 |
| $CHF_2$ | $C_2H_5$ | 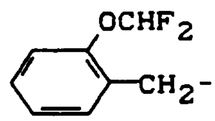 |
| $CH_3$ | $CF_3$ | 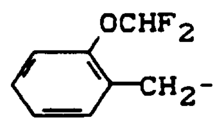 |
| $C_2H_5$ | $CF_3$ | 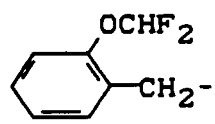 |
| $-(CH_2)_3-$ | | 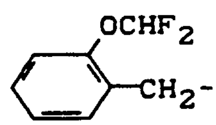 |
| $-(CH_2)_4-$ | | 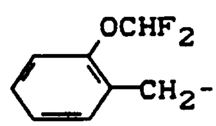 |
| $-(CH_2)_5-$ | | 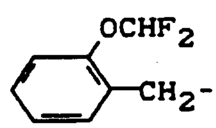 |

Tabelle 1 - Fortsetzung

| $R^1$ | $R^2$ | $R^3$ |
|---|---|---|
| $CH_3$ | $CH_3$ | 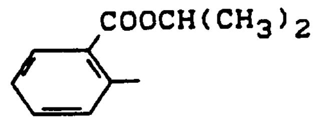 |
| H | $CH_3$ | 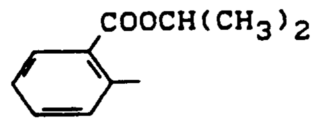 |
| $CH_3$ | H | 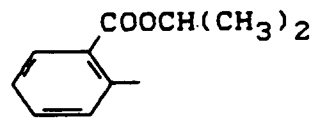 |
| $CH_3$ | H | 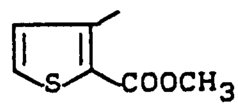 |
| $C_2H_5$ | H | 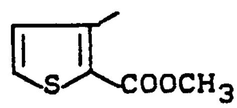 |
| $C_3H_7$ | H | 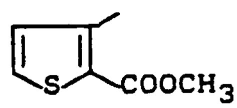 |
| $CH(CH_3)_2$ | H | 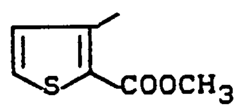 |
| $C_4H_9$ | H | 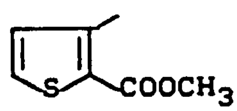 |
| $CH_2CH(CH_3)_2$ | H | 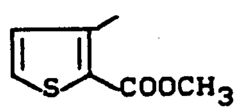 |
| $C(CH_3)_3$ | H | 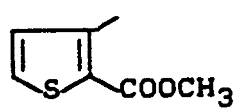 |

Tabelle 1 - Fortsetzung

| $R^1$ | $R^2$ | $R^3$ |
|---|---|---|
| H | $CH_3$ | 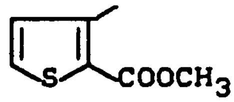 |
| H | $C_2H_5$ | 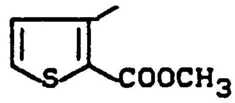 |
| H | $C_3H_7$ | 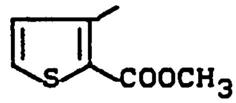 |
| H | $CH(CH_3)_2$ | 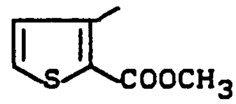 |
| H | $C_4H_9$ | 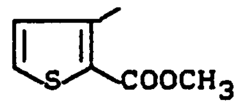 |
| H | $CH_2CH(CH_3)_2$ | 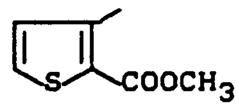 |
| H | $C(CH_3)_3$ | 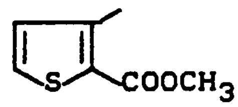 |
| $CHF_2$ | H | 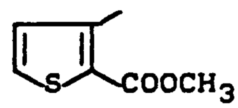 |
| $CH_2CH_2CN$ | H | 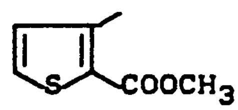 |
| $CH_2CH_2OCH_3$ | H | 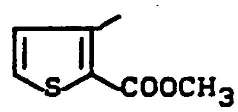 |

Tabelle 1 - Fortsetzung

| $R^1$ | $R^2$ | $R^3$ |
|---|---|---|
| H | $CF_3$ | 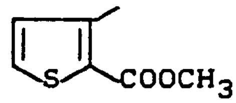 |
| $CH_2OCH_3$ | H | 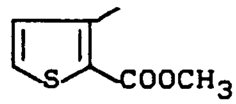 |
| H | $CH_2OCH_3$ | 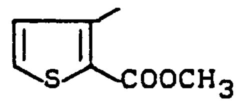 |
| H | $CH_2CH_2OCH_3$ | 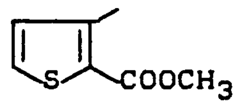 |
| $CH_3$ | $CH_3$ | 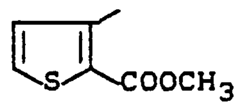 |
| $CH_3$ | $C_2H_5$ | 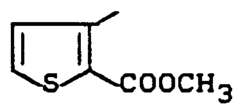 |
| $CH_3$ | $C_3H_7$ | 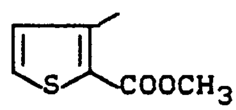 |
| $CH_3$ | $CH(CH_3)_2$ | 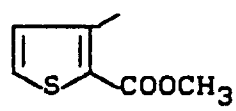 |
| $CH_3$ | $C_4H_9$ | 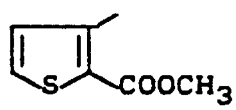 |
| $CH_3$ | $CH_2CH(CH_3)_2$ | 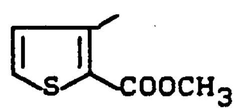 |

Tabelle 1 - Fortsetzung

| $R^1$ | $R^2$ | $R^3$ |
|---|---|---|
| $CH_3$ | $C(CH_3)_3$ | 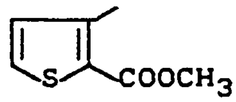 |
| $C_2H_5$ | $CH_3$ | 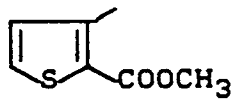 |
| $C_3H_7$ | $CH_3$ | 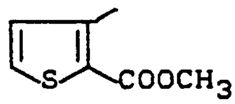 |
| $CH(CH_3)_2$ | $CH_3$ | 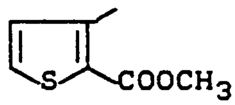 |
| $C_4H_9$ | $CH_3$ | 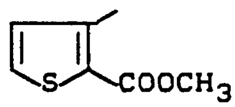 |
| $C_2H_5$ | $C_2H_5$ | 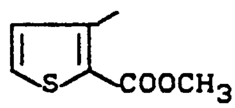 |
| $CHF_2$ | $CH_3$ | 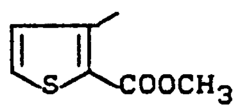 |
| $CHF_2$ | $C_2H_5$ | 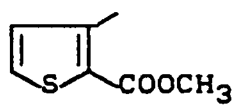 |
| $CH_3$ | $CF_3$ | 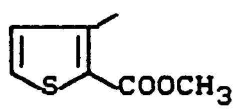 |
| $C_2H_5$ | $CF_3$ | 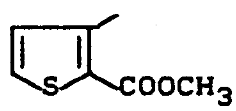 |

Tabelle 1 - Fortsetzung

| $R^1$ | $R^2$ | $R^3$ |
|---|---|---|
| $-(CH_2)_3-$ | | 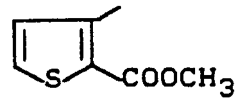 |
| $-(CH_2)_4-$ | | 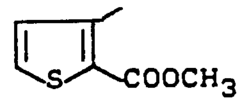 |
| $-(CH_2)_5-$ | | 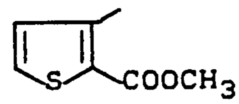 |
| $CH_3$ | $CH_3$ | 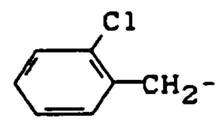 |
| H | $CH_3$ | 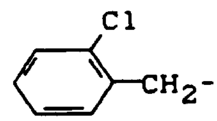 |
| $CH_3$ | H | 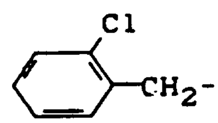 |
| $CH_3$ | H | 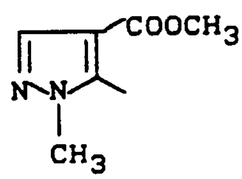 |
| $C_2H_5$ | H | 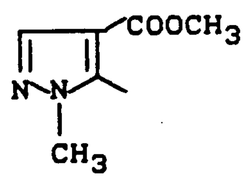 |
| $C_3H_7$ | H | 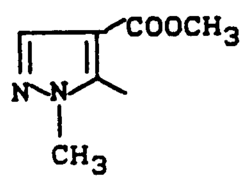 |

Tabelle 1 - Fortsetzung

| $R^1$ | $R^2$ | $R^3$ |
|---|---|---|
| $CH(CH_3)_2$ | H | 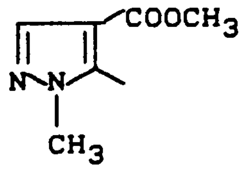 |
| $C_4H_9$ | H | 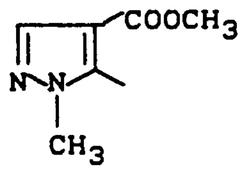 |
| $CH_2CH(CH_3)_2$ | H | 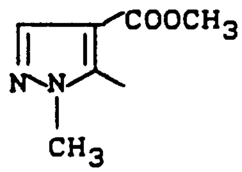 |
| $C(CH_3)_3$ | H | 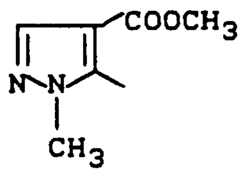 |
| H | $CH_3$ | 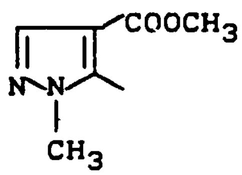 |
| H | $C_2H_5$ | 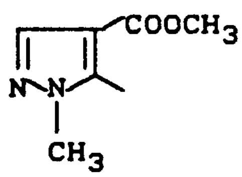 |
| H | $C_3H_7$ | 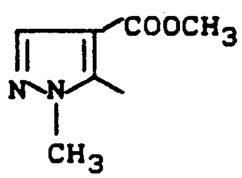 |

<u>Tabelle 1</u> - Fortsetzung

| $R^1$ | $R^2$ | $R^3$ |
|---|---|---|
| H | $CH(CH_3)_2$ | 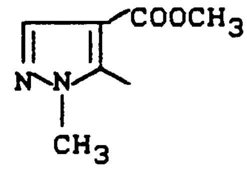 |
| H | $C_4H_9$ | 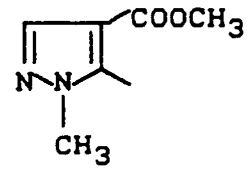 |
| H | $CH_2CH(CH_3)_2$ | 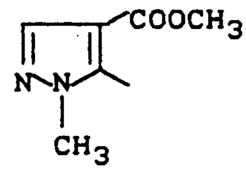 |
| H | $C(CH_3)_3$ | 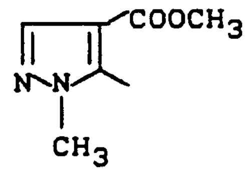 |
| $CHF_2$ | H | 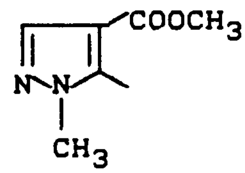 |
| $CH_2CH_2CN$ | H | 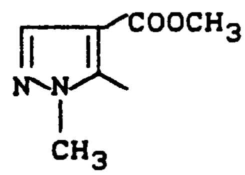 |
| $CH_2CH_2OCH_3$ | H | 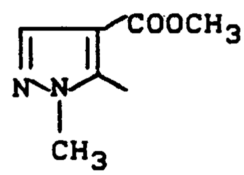 |

<u>Tabelle 1</u> - Fortsetzung

| $R^1$ | $R^2$ | $R^3$ |
|---|---|---|
| H | $CF_3$ | 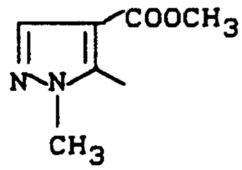 |
| $CH_2OCH_3$ | H | 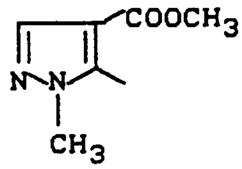 |
| H | $CH_2OCH_3$ | 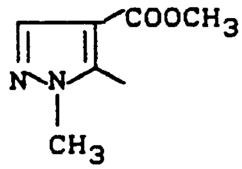 |
| H | $CH_2CH_2OCH_3$ | 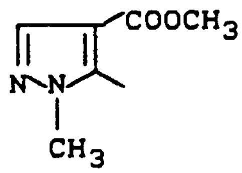 |
| $CH_3$ | $CH_3$ | 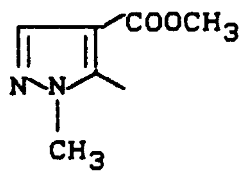 |
| $CH_3$ | $C_2H_5$ | 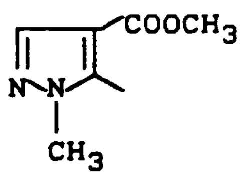 |
| $CH_3$ | $C_3H_7$ | 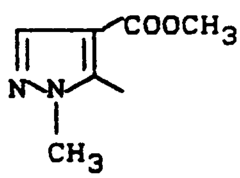 |

Tabelle 1 - Fortsetzung

| $R^1$ | $R^2$ | $R^3$ |
|---|---|---|
| $CH_3$ | $CH(CH_3)_2$ | 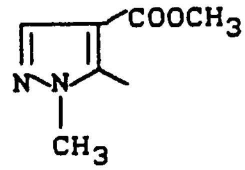 |
| $CH_3$ | $C_4H_9$ | 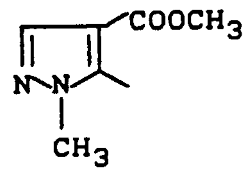 |
| $CH_3$ | $CH_2CH(CH_3)_2$ | 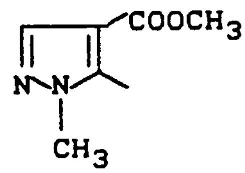 |
| $CH_3$ | $C(CH_3)_3$ | 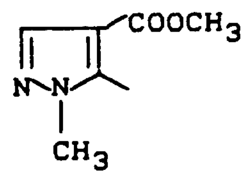 |
| $C_2H_5$ | $CH_3$ | 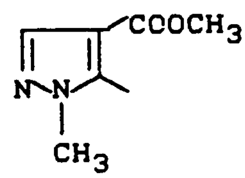 |
| $C_3H_7$ | $CH_3$ | 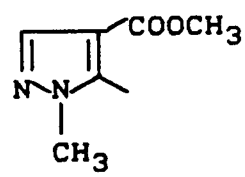 |
| $CH(CH_3)_2$ | $CH_3$ | 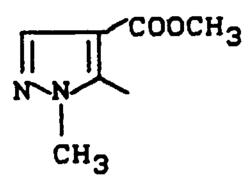 |

<u>Tabelle 1</u> - Fortsetzung

| $R^1$ | $R^2$ | $R^3$ |
|---|---|---|
| $C_4H_9$ | $CH_3$ | 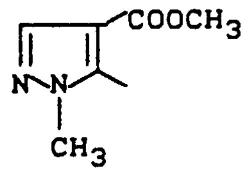 |
| $C_2H_5$ | $C_2H_5$ | 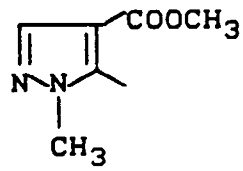 |
| $CHF_2$ | $CH_3$ | 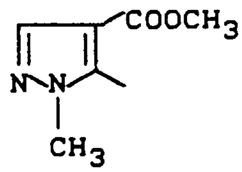 |
| $CHF_2$ | $C_2H_5$ | 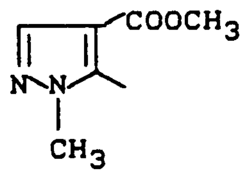 |
| $CH_3$ | $CF_3$ | 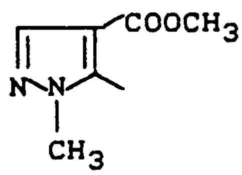 |
| $C_2H_5$ | $CF_3$ | 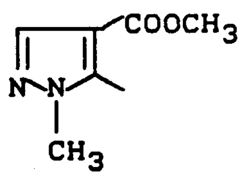 |
| $-(CH_2)_3-$ | | 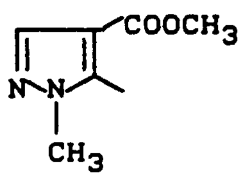 |

<u>Tabelle 1</u> - Fortsetzung

| $R^1$ | $R^2$ | $R^3$ |
|---|---|---|
| $-(CH_2)_4-$ | | 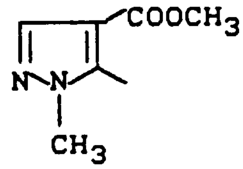 |
| $-(CH_2)_5-$ | | 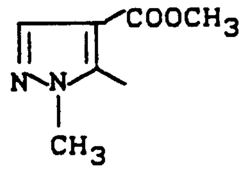 |
| $CH_3$ | $CH_3$ | 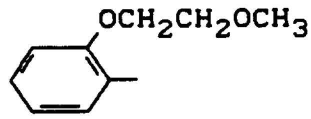 |
| H | $CH_3$ | 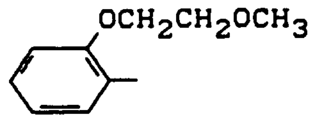 |
| $CH_3$ | H | 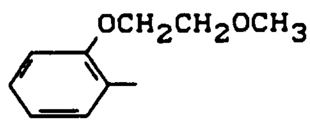 |
| $CH_3$ | H | 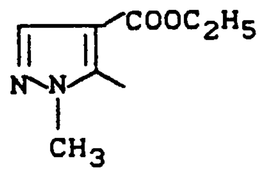 |
| $C_2H_5$ | H | 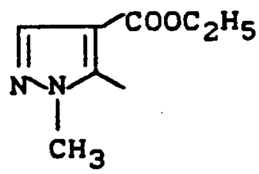 |
| $C_3H_7$ | H | 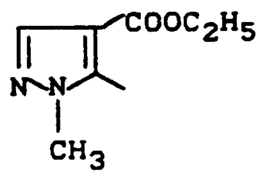 |

Tabelle 1 - Fortsetzung

| $R^1$ | $R^2$ | $R^3$ |
|---|---|---|
| $CH(CH_3)_2$ | H | 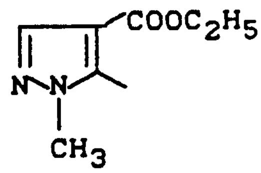 |
| $C_4H_9$ | H | 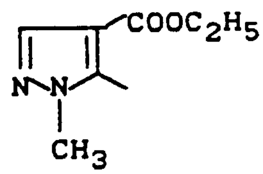 |
| $CH_2CH(CH_3)_2$ | H | 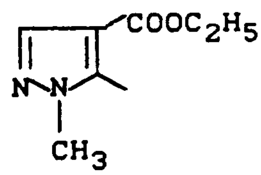 |
| $C(CH_3)_3$ | H | 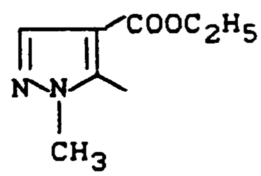 |
| H | $CH_3$ | 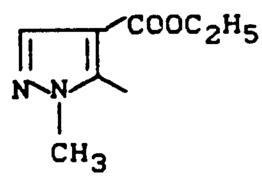 |
| H | $C_2H_5$ | 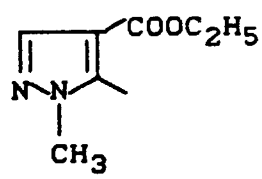 |
| H | $C_3H_7$ | 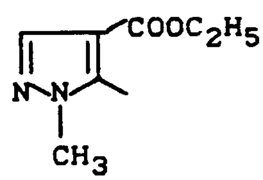 |

Tabelle 1 - Fortsetzung

| $R^1$ | $R^2$ | $R^3$ |
|---|---|---|
| H | $CH(CH_3)_2$ | 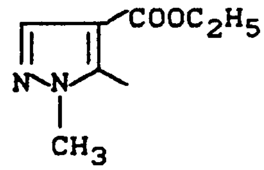 |
| H | $C_4H_9$ | 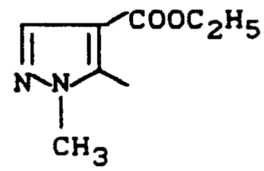 |
| H | $CH_2CH(CH_3)_2$ | 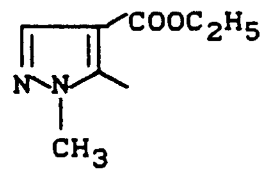 |
| H | $C(CH_3)_3$ | 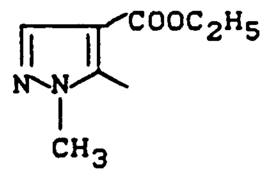 |
| $CHF_2$ | H | 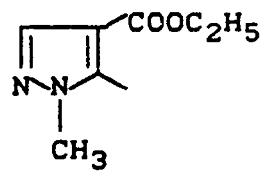 |
| $CH_2CH_2CN$ | H | 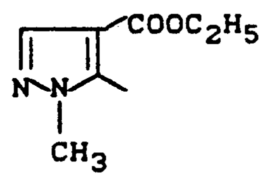 |
| $CH_2CH_2OCH_3$ | H | 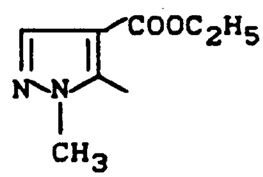 |

Tabelle 1 - Fortsetzung

| $R^1$ | $R^2$ | $R^3$ |
|---|---|---|
| H | $CF_3$ | 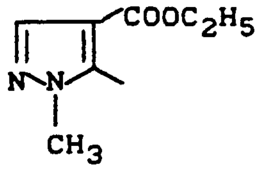 |
| $CH_2OCH_3$ | H | 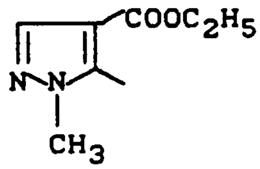 |
| H | $CH_2OCH_3$ | 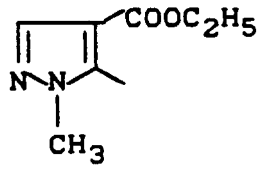 |
| H | $CH_2CH_2OCH_3$ | 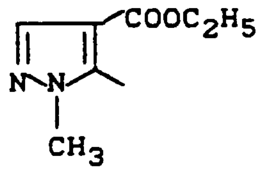 |
| $CH_3$ | $CH_3$ | 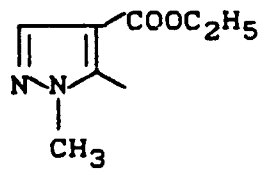 |
| $CH_3$ | $C_2H_5$ | 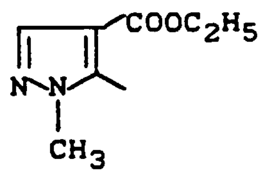 |
| $CH_3$ | $C_3H_7$ | 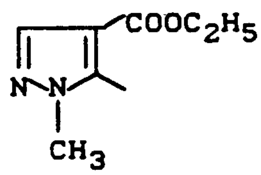 |

Tabelle 1 - Fortsetzung

| $R^1$ | $R^2$ | $R^3$ |
| --- | --- | --- |
| $CH_3$ | $CH(CH_3)_2$ | 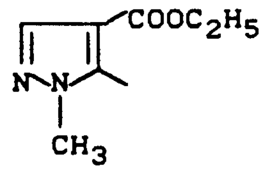 |
| $CH_3$ | $C_4H_9$ | 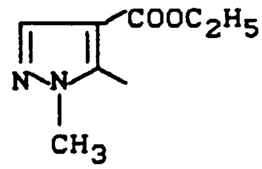 |
| $CH_3$ | $CH_2CH(CH_3)_2$ | 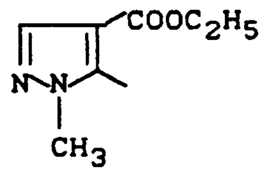 |
| $CH_3$ | $C(CH_3)_3$ | 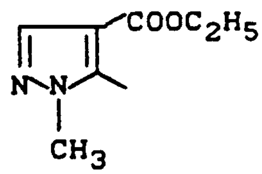 |
| $C_2H_5$ | $CH_3$ | 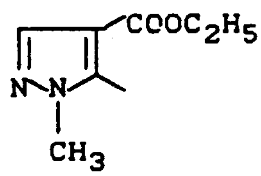 |
| $C_3H_7$ | $CH_3$ | 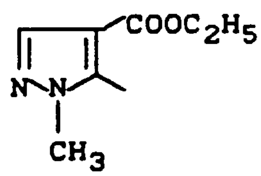 |
| $CH(CH_3)_2$ | $CH_3$ | 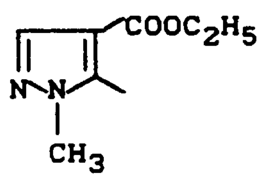 |

Tabelle 1 - Fortsetzung

| $R^1$ | $R^2$ | $R^3$ |
|---|---|---|
| $C_4H_9$ | $CH_3$ | 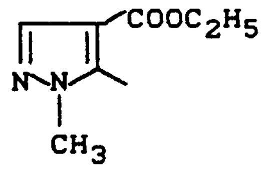 |
| $C_2H_5$ | $C_2H_5$ | 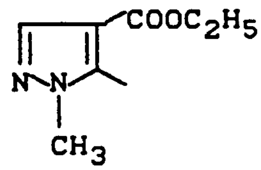 |
| $CHF_2$ | $CH_3$ | 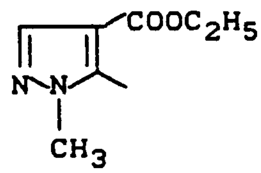 |
| $CHF_2$ | $C_2H_5$ | 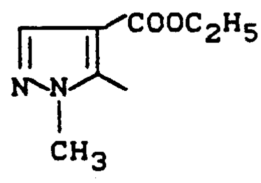 |
| $CH_3$ | $CF_3$ | 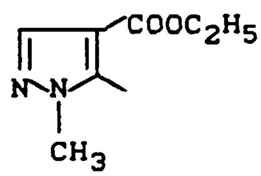 |
| $C_2H_5$ | $CF_3$ | 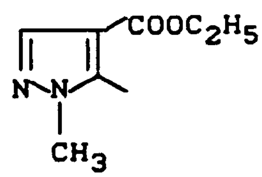 |
| $-(CH_2)_3-$ | | 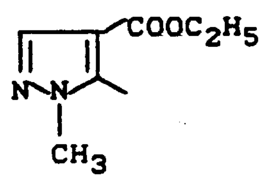 |

Tabelle 1 - Fortsetzung

| $R^1$ | $R^2$ | $R^3$ |
|---|---|---|
| $-(CH_2)_4-$ | | 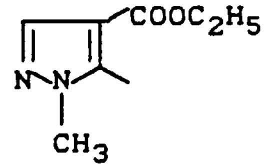 |
| $-(CH_2)_5-$ | | 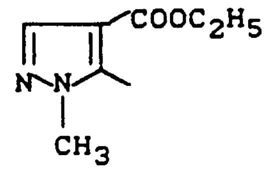 |
| $CH_3$ | $CH_3$ | 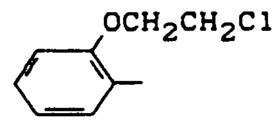 |
| H | $CH_3$ | 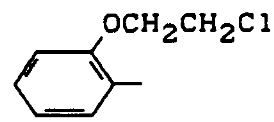 |
| $CH_3$ | H | 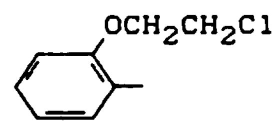 |
| $CH_3$ | H | 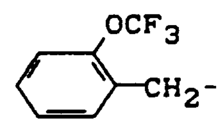 |
| $C_2H_5$ | H | 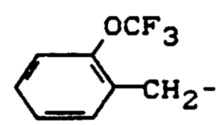 |
| $C_3H_7$ | H | 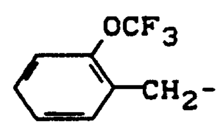 |

Tabelle 1 - Fortsetzung

| $R^1$ | $R^2$ | $R^3$ |
|---|---|---|
| $CH(CH_3)_2$ | H | 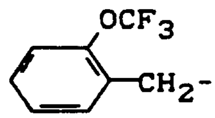 |
| $C_4H_9$ | H | 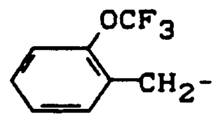 |
| $CH_2CH(CH_3)_2$ | H | 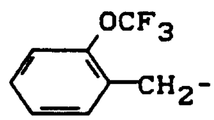 |
| $C(CH_3)_3$ | H | 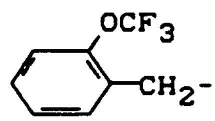 |
| H | $CH_3$ | 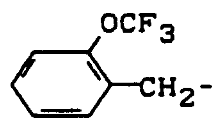 |
| H | $C_2H_5$ | 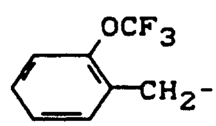 |
| H | $C_3H_7$ | 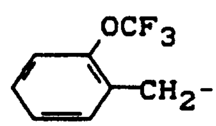 |
| H | $CH(CH_3)_2$ | 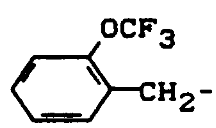 |

Tabelle 1 - Fortsetzung

| $R^1$ | $R^2$ | $R^3$ |
|---|---|---|
| H | $C_4H_9$ | 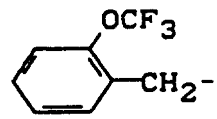 |
| H | $CH_2CH(CH_3)_2$ | 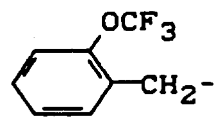 |
| H | $C(CH_3)_3$ | 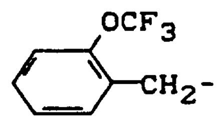 |
| $CHF_2$ | H | 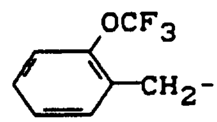 |
| $CH_2CH_2CN$ | H | 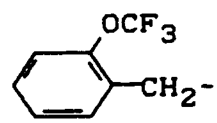 |
| $CH_2CH_2OCH_3$ | H | 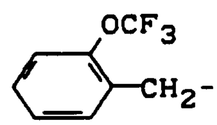 |
| H | $CF_3$ | 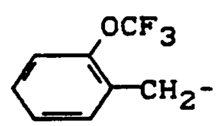 |
| $CH_2OCH_3$ | H | 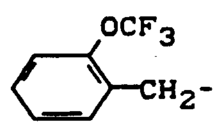 |

Tabelle 1 - Fortsetzung

| $R^1$ | $R^2$ | $R^3$ |
| --- | --- | --- |
| H | $CH_2OCH_3$ | 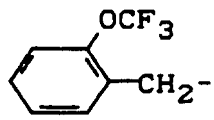 |
| H | $CH_2CH_2OCH_3$ | 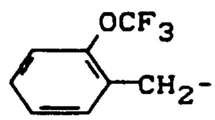 |
| $CH_3$ | $CH_3$ | 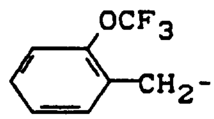 |
| $CH_3$ | $C_2H_5$ | 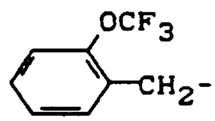 |
| $CH_3$ | $C_3H_7$ | 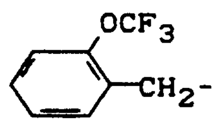 |
| $CH_3$ | $CH(CH_3)_2$ | 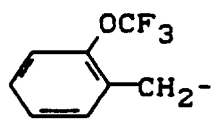 |
| $CH_3$ | $C_4H_9$ | 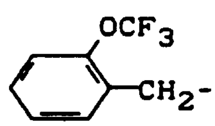 |
| $CH_3$ | $CH_2CH(CH_3)_2$ | 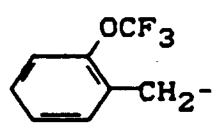 |

Tabelle 1 - Fortsetzung

| $R^1$ | $R^2$ | $R^3$ |
|---|---|---|
| $CH_3$ | $C(CH_3)_3$ | 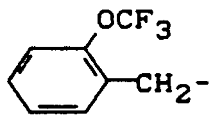 |
| $C_2H_5$ | $CH_3$ | 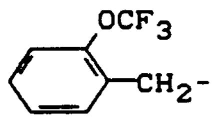 |
| $C_3H_7$ | $CH_3$ | 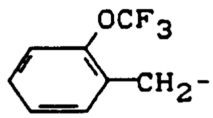 |
| $CH(CH_3)_2$ | $CH_3$ | 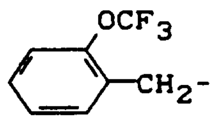 |
| $C_4H_9$ | $CH_3$ | 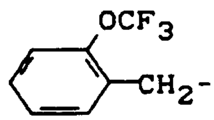 |
| $C_2H_5$ | $C_2H_5$ | 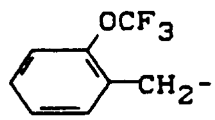 |
| $CHF_2$ | $CH_3$ | 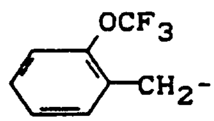 |
| $CHF_2$ | $C_2H_5$ | 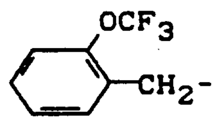 |

Tabelle 1 - Fortsetzung

| $R^1$ | $R^2$ | $R^3$ |
|---|---|---|
| $CH_3$ | $CF_3$ | 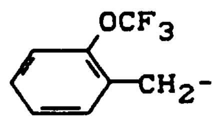 |
| $C_2H_5$ | $CF_3$ | 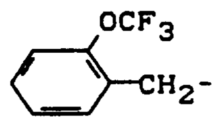 |
| $-(CH_2)_3-$ | | 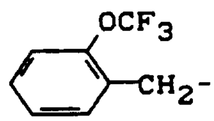 |
| $-(CH_2)_4-$ | | 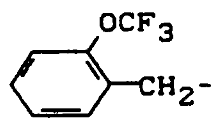 |
| $-(CH_2)_5-$ | | 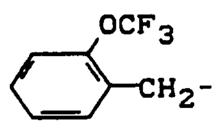 |
| $CH_3$ | H | 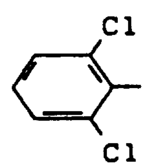 |
| $C_2H_5$ | H | 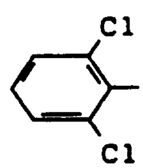 |
| $C_3H_7$ | H | 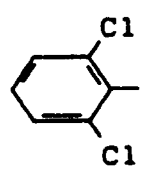 |

Tabelle 1 - Fortsetzung

| $R^1$ | $R^2$ | $R^3$ |
|---|---|---|
| $CH(CH_3)_2$ | H | 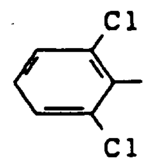 |
| $C_4H_9$ | H | 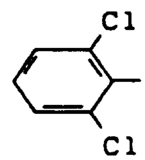 |
| $CH_2CH(CH_3)_2$ | H | 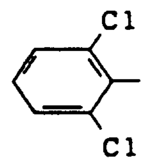 |
| $C(CH_3)_3$ | H | 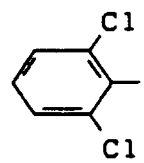 |
| H | $CH_3$ | 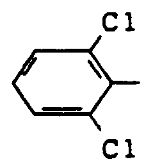 |
| H | $C_2H_5$ | 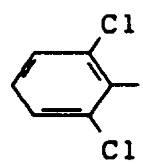 |
| H | $C_3H_7$ | 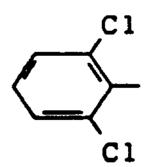 |

Tabelle 1 - Fortsetzung

| $R^1$ | $R^2$ | $R^3$ |
|---|---|---|
| H | $CH(CH_3)_2$ | 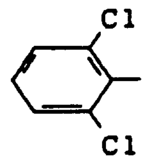 |
| H | $C_4H_9$ | 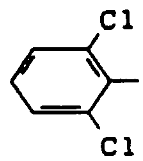 |
| H | $CH_2CH(CH_3)_2$ | 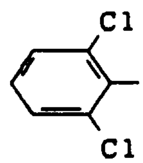 |
| H | $C(CH_3)_3$ | 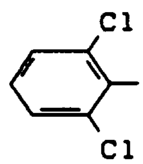 |
| $CHF_2$ | H | 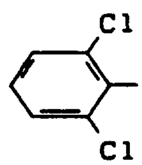 |
| $CH_2CH_2CN$ | H | 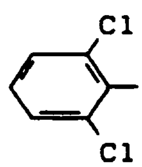 |
| $CH_2CH_2OCH_3$ | H | 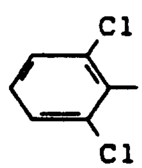 |

Tabelle 1 - Fortsetzung

| $R^1$ | $R^2$ | $R^3$ |
|---|---|---|
| H | $CF_3$ | 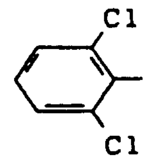 |
| $CH_2OCH_3$ | H | 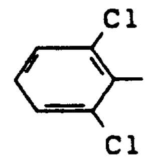 |
| H | $CH_2OCH_3$ | 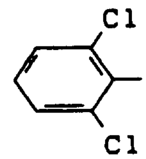 |
| H | $CH_2CH_2OCH_3$ | 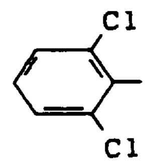 |
| $CH_3$ | $CH_3$ | 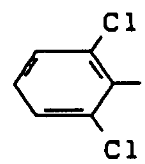 |
| $CH_3$ | $C_2H_5$ | 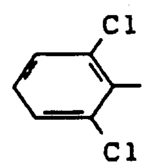 |
| $CH_3$ | $C_3H_7$ | 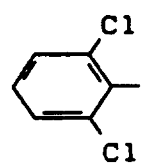 |

Tabelle 1 - Fortsetzung

| $R^1$ | $R^2$ | $R^3$ |
|---|---|---|
| $CH_3$ | $CH(CH_3)_2$ | 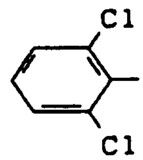 |
| $CH_3$ | $C_4H_9$ | 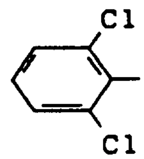 |
| $CH_3$ | $CH_2CH(CH_3)_2$ | 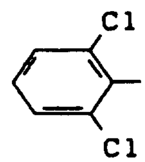 |
| $CH_3$ | $C(CH_3)_3$ | 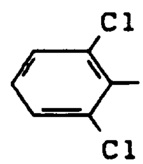 |
| $C_2H_5$ | $CH_3$ | 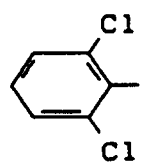 |
| $C_3H_7$ | $CH_3$ | 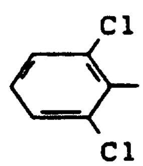 |
| $CH(CH_3)_2$ | $CH_3$ | 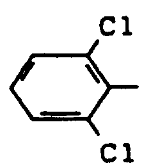 |

Tabelle 1 - Fortsetzung

| $R^1$ | $R^2$ | $R^3$ |
|---|---|---|
| $C_4H_9$ | $CH_3$ | 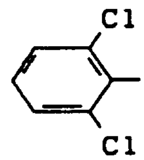 |
| $C_2H_5$ | $C_2H_5$ | 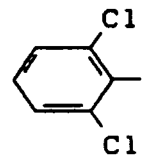 |
| $CHF_2$ | $CH_3$ | 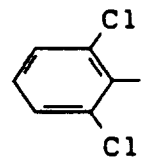 |
| $CHF_2$ | $C_2H_5$ | 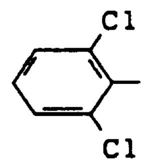 |
| $CH_3$ | $CF_3$ | 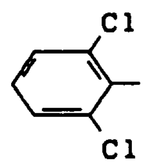 |
| $C_2H_5$ | $CF_3$ | 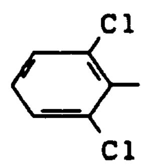 |
| $-(CH_2)_3-$ | | 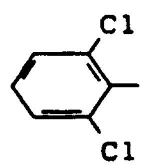 |

Tabelle 1 - Fortsetzung

| $R^1$ | $R^2$ | $R^3$ |
|---|---|---|
| $-(CH_2)_4-$ | | 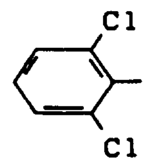 |
| $-(CH_2)_5-$ | | 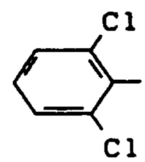 |
| $CH_3$ | $CH_3$ | 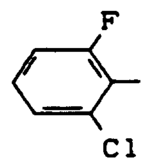 |
| H | $CH_3$ | 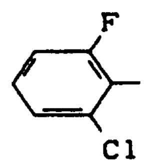 |
| $CH_3$ | H | 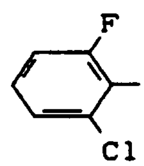 |
| $CH_3$ | H | 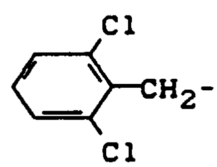 |
| $C_2H_5$ | H | 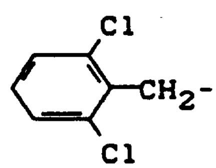 |

<u>Tabelle 1</u> - Fortsetzung

| $R^1$ | $R^2$ | $R^3$ |
|---|---|---|
| $C_3H_7$ | H | 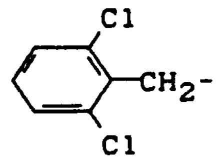 |
| $CH(CH_3)_2$ | H | 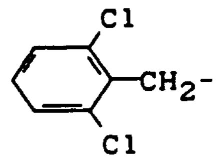 |
| $C_4H_9$ | H | 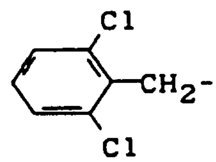 |
| $CH_2CH(CH_3)_2$ | H | 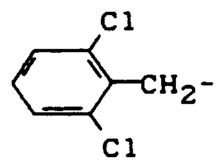 |
| $C(CH_3)_3$ | H | 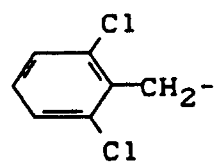 |
| H | $CH_3$ | 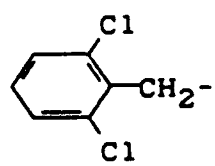 |
| H | $C_2H_5$ | 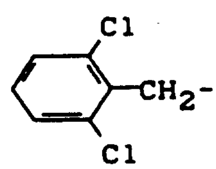 |

Tabelle 1 - Fortsetzung

| $R^1$ | $R^2$ | $R^3$ |
|---|---|---|
| H | $C_3H_7$ | 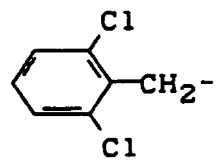 |
| H | $CH(CH_3)_2$ | 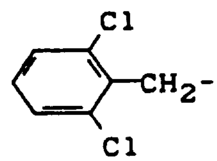 |
| H | $C_4H_9$ | 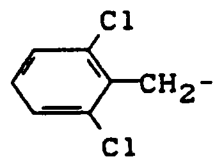 |
| H | $CH_2CH(CH_3)_2$ | 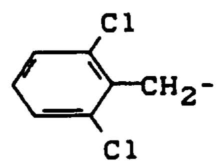 |
| H | $C(CH_3)_3$ | 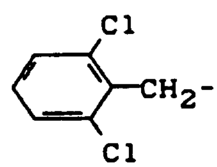 |
| $CHF_2$ | H | 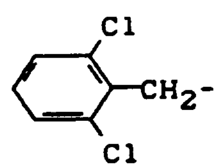 |
| $CH_2CH_2CN$ | H | 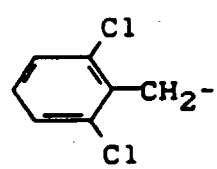 |

Tabelle 1 - Fortsetzung

| $R^1$ | $R^2$ | $R^3$ |
|---|---|---|
| $CH_2CH_2OCH_3$ | H | 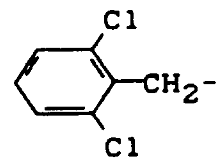 |
| H | $CF_3$ | 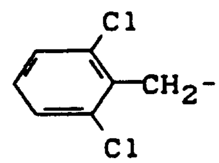 |
| $CH_2OCH_3$ | H | 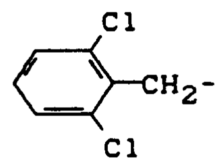 |
| H | $CH_2OCH_3$ | 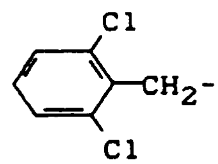 |
| H | $CH_2CH_2OCH_3$ | 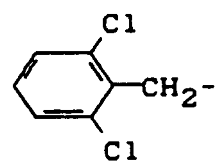 |
| $CH_3$ | $CH_3$ | 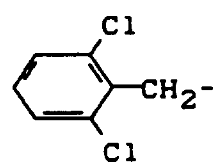 |
| $CH_3$ | $C_2H_5$ | 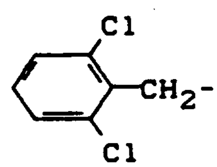 |

Tabelle 1 - Fortsetzung

| $R^1$ | $R^2$ | $R^3$ |
|---|---|---|
| $CH_3$ | $C_3H_7$ | 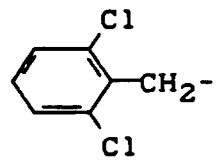 |
| $CH_3$ | $CH(CH_3)_2$ | 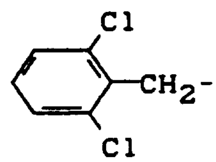 |
| $CH_3$ | $C_4H_9$ | 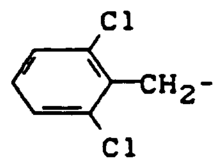 |
| $CH_3$ | $CH_2CH(CH_3)_2$ | 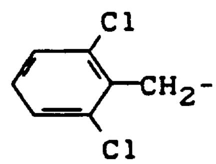 |
| $CH_3$ | $C(CH_3)_3$ | 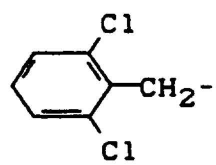 |
| $C_2H_5$ | $CH_3$ | 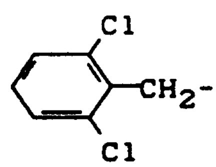 |
| $C_3H_7$ | $CH_3$ | 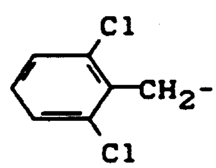 |

Tabelle 1 - Fortsetzung

| $R^1$ | $R^2$ | $R^3$ |
|---|---|---|
| $CH(CH_3)_2$ | $CH_3$ | 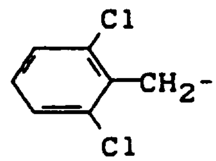 |
| $C_4H_9$ | $CH_3$ | 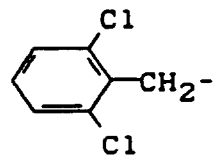 |
| $C_2H_5$ | $C_2H_5$ | 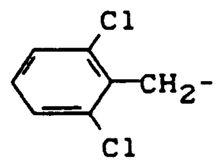 |
| $CHF_2$ | $CH_3$ | 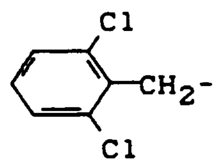 |
| $CHF_2$ | $C_2H_5$ | 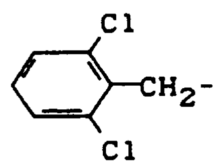 |
| $CH_3$ | $CF_3$ | 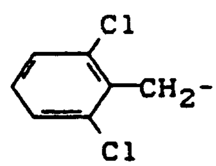 |
| $C_2H_5$ | $CF_3$ | 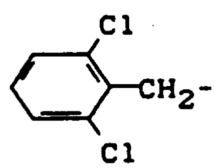 |

<u>Tabelle 1</u> - Fortsetzung

| $R^1$ | $R^2$ | $R^3$ |
|---|---|---|
| $-(CH_2)_3-$ | | 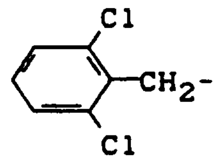 |
| $-(CH_2)_4-$ | | 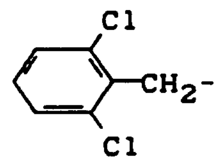 |
| $-(CH_2)_5-$ | | 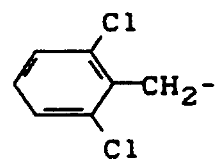 |
| $CH_3$ | $CH_3$ | 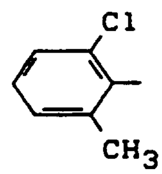 |
| $CH_3$ | $CH_3$ | 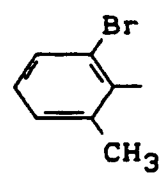 |

Verwendet man beispielsweise 2-Fluor-phenylsulfonyl-isocyanat und 4,5-Dimethyl-2,4-dihydro-3H-1,2,4-triazol-3-on als Ausgangsstoffe, so kann der Reaktionsablauf beim erfindungsgemäßen Verfahren durch folgendes Formelschema skizziert werden:

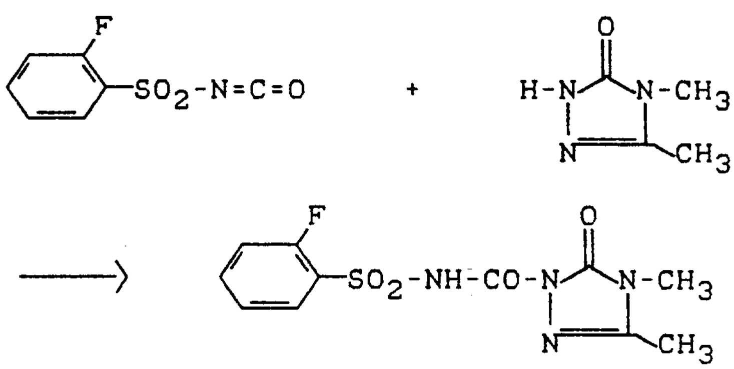

Die beim erfindungsgemäßen Verfahren zur Herstellung von Verbindungen der Formel (I) als Ausgangsstoffe zu verwendenden Triazolinone sind durch die Formel (II) allgemein definiert.

In Formel (II) haben $R^1$ und $R^2$ vorzugsweise diejenigen Bedeutungen, die bereits oben im Zusammenhang mit der Beschreibung der erfindungsgemäßen Verbindungen der Formel (I) als insbesondere bevor-

zugt für $R^1$ und $R^2$ angegeben wurden.

Beispiele für die Ausgangsstoffe der Formel (II) sind in der nachstehenden Tabelle 2 aufgeführt.

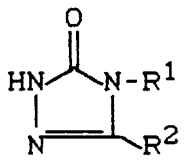

(II)

Tabelle 2: Beispiele für die Ausgangsstoffe der Formel (II)

| $R^1$ | $R^2$ |
|---|---|
| H | H |
| $CH_3$ | H |
| $C_2H_5$ | H |
| $C_3H_7$ | H |
| $CH(CH_3)_2$ | H |
| $C_4H_9$ | H |
| $CH_2CH(CH_3)_2$ | H |
| $C(CH_3)_3$ | H |
| H | $CH_3$ |
| H | $C_2H_5$ |
| H | $C_3H_7$ |
| H | $CH(CH_3)_2$ |
| H | $C_4H_9$ |
| H | $CH_2CH(CH_3)_2$ |
| H | $C(CH_3)_3$ |
| $CHF_2$ | H |
| $CH_2CH_2CN$ | H |
| $CH_2CH_2OCH_3$ | H |
| $C_2H_5$ | $SCH_3$ |
| H | $CF_3$ |
| H | $CH_2OCH_3$ |

Tabelle 2 - Fortsetzung

| $R^1$ | $R^2$ |
|---|---|
| H | $CH_2OC_2H_5$ |
| H | $CH_2CH_2OCH_3$ |
| $CH_3$ | $CH_3$ |
| $CH_3$ | $C_2H_5$ |
| $CH_3$ | $C_3H_7$ |
| $CH_3$ | $CH(CH_3)_2$ |
| $CH_3$ | $C_4H_9$ |
| $CH_3$ | $CH_2CH(CH_3)_2$ |
| $CH_3$ | $C(CH_3)_3$ |
| $C_2H_5$ | $CH_3$ |
| $C_3H_7$ | $CH_3$ |
| $CH(CH_3)_2$ | $CH_3$ |
| $C_4H_9$ | $CH_3$ |
| $CH_2CH(CH_3)_2$ | $CH_3$ |
| $C_2H_5$ | $C_2H_5$ |
| $C_3H_7$ | $C_3H_7$ |
| $CH_3$ | $SC_2H_5$ |
| $CHF_2$ | $CH_3$ |
| $CHF_2$ | $C_2H_5$ |
| $CH_3$ | $CF_3$ |
| $C_2H_5$ | $CF_3$ |

Tabelle 2 - Fortsetzung

| $R^1$ | $R^2$ |
|---|---|
| $CF_2CHF_2$ | $CH_3$ |
| $C_2H_5$ | $C_3H_7$ |
| $C_2H_5$ | $C_4H_9$ |
| $C_6H_5$ | $CH_3$ |
| 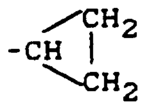 | $CH_3$ |
| $CH_3$ | 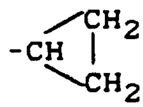 |
| 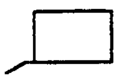 | $CH_3$ |
| 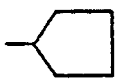 | $CH_3$ |
| $CH_3$ | $N(CH_3)_2$ |
| $-(CH_2)_3-$ | |
| $-(CH_2)_4-$ | |
| $-(CH_2)_5-$ | |
| $-(CH_2)_6-$ | |
| $-(CH_2)_7-$ | |
| $-(CH_2)_{11}-$ | |
| 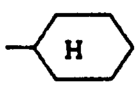 | $CH_3$ |

Tabelle 2 - Fortsetzung

| $R^1$ | $R^2$ |
|---|---|
| $CH_3$ | 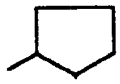 |
| $CH_3$ | 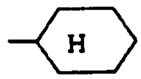 |
| $NH_2$ | $CH_3$ |
| $NH_2$ | $SCH_3$ |
| $CH_3$ | $SCH_3$ |
| $CH_3$ | $NHCH_3$ |

Die Ausgangsstoffe der Formel (II) sind bekannt und/oder können nach an sich bekannten Verfahren hergestellt werden (vgl. Chem. Ber. 90 (1957), 909 - 921; ibid. 98 (1965), 3025 - 3099; J. Heterocycl. Chem. 15 (1978), 237 - 240; Tetrahedron 32 (1976), 2347 - 2352; Helv. Chim. Acta 63 (1980), 841 - 859; J. Chem. Soc. C 1967, 746 - 751).

Neu sind die Verbindungen der Formel (IIa),

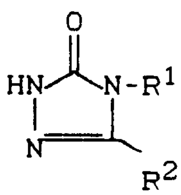

(IIa)

in welcher

$R^1$ und $R^2$ zusammen für Alkandiyl mit 6 und mit 8 bis 11 Kohlenstoffatomen stehen.

Man erhält diese neuen 4,5-Alkandiyl-2,4-dihydro-3H-1,2,4-triazol-3-one der Formel (IIa), wenn man Lactim-ether der Formel (IV)

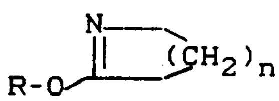

(IV)

in welcher

n für die Zahlen 6 und für 8 bis 11 steht und

R für $C_1$-$C_4$-Alkyl steht,

mit Carbazinsäureestern der Formel (V)

$H_2N$ - NH - CO - OR (V)

in welcher

R für $C_1$-$C_4$-Alkyl steht,

gegebenenfalls in Gegenwart eines Verdünnungsmittels, wie z. B. Ethanol, bei Temperaturen Zwischen 20 °C und 100 °C umsetzt.

Die beim erfindungsgemäßen Verfahren zur Herstellung von Verbindungen der Formel (I) als Ausgangsstoffe zu verwendenden Sulfonylisocyanate sind durch die Formel (III) allgemein definiert.

In Formel (III) hat $R^3$ vorzugsweise diejenige Bedeutung, die bereits oben im Zusammenhang mit der Beschreibung der erfindungsgemäßen Verbindungen der Formel (I) als insbesondere bevorzugt für $R^3$ angegeben wurde.

Als Beispiele für die Ausgangsstoffe der Formel (III) seien genannt:

2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren wird im allgemeinen bei Normaldruck durchgeführt.

Zur Durchführung des erfindungsgemäßen Verfahrens setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt, eingeengt und das im Rückstand verbleibende Rohprodukt mit einem geeigneten Lösungsmittel, wie z. B. Diethylether, zur Kristallisation gebracht. Das kristallin angefallene Produkt der Formel (I) wird durch Absaugen isoliert.

Zur Überführung in Salze werden die Verbindungen der Formel (I) mit geeigneten Salzbildnern, wie z. B. Natrium- oder Kalium-hydroxid, -methylat oder -ethylat, Ammoniak, Isopropylamin, Dibutylamin oder Triethylamin, in geeigneten Verdünnungsmitteln, wie z. B. Wasser, Methanol oder Ethanol, verrührt. Die Salze können dann - gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden, Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich zur selektiven Bekämpfung monokotyler und dikotyler Unkräuter in monokotylen Kulturen im Vorauflauf- und im Nachauflauf-Verfahren. Sie sind bei praktisch gleich guter Verträglichkeit für Gerste gegen Unkräuter deutlich wirksamer als z. B. Isocarbamid.

Einige der erfindungsgemäßen Verbindungen zeigen auch fungizide Wirkung, z. B. gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Tragerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-2,2-dimethyl-4,6-dioxo-5-[1-(2-propenyloxyamino)-butyliden]-cyclo hexancarboxylat (ALLOXYDIM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorophenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); [(4-Amino-3,5-dichlor-6-fluor-2-pyridinyl)-oxy]-essigsäure bzw. deren 1-Methylheptylester (FLUROXYPYR); 5-(2-Chlor-4-trifluoromethyl-phenoxy)-N-methylsulfonyl-2-nitrobenzamid (FOMESAFEN); N-Phosphonomethyl-glycin (GLYPHOSATE); 2-{4-[(3-Chlor-5-(trifluormethyl)-2-pyridinyl)-oxy]-phenoxy}-propansäure bzw. deren Ethylester (HALOXYFOP); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Methoxycarbonylaminophenyl)-N-(3'-methylphenyl)-carbamat (PHENMEDIPHAM); α-Chlor-2',6'-diethyl-N-(2-propoxyethyl)-acetanilid (PRETILACHLOR); 2-[1-(Ethoxamino)-butyliden]-5-(2-ethylthiopropyl)-1,3-cyclohexadion (SETHOXYDIM); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2,6-Dinitro-4-trifluormethyl-N,N-dipropylanilin (TRIFLURALIN) und 2-[4-(6-Chlor-2-chonoxalinyloxy)-phenoxy]-propionsäure-ethylester. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

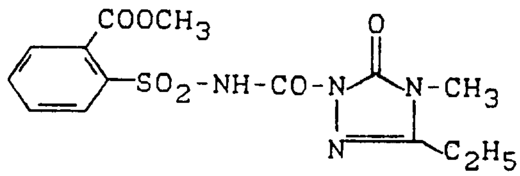

Eine Mischung aus 3,8 g (0,03 Mol) 5-Ethyl-4-methyl-2,4-dihydro-3H-1,2,4-triazol-3-on, 12 g (0,05 Mol) 2-Methoxycarbonyl-phenylsulfonylisocyanat und 50 ml Methylenchlorid wird 20 Stunden bei 20 °C gerührt und anschließend im Wasserstrahlvakuum eingeengt. Der Rückstand wird mit Diethylether verrieben und das dabei kristallin angefallene Produkt durch Absaugen isoliert.

Man erhält 6 g (54 % der Theorie) 5-Ethyl-4-methyl-2-(2-methoxycarbonyl-phenylsulfonyl-aminocarbonyl)-2,4-dihydro-3H-1,2,4-triazol-3-on vom Schmelzpunkt 131 °C.

Analog Beispiel 1 und entsprechend der allgemeinen Beschreibung des erfindungsgemäßen Herstellungsverfahrens können beispielsweise die in der nachstehenden Tabelle 3 aufgeführten Verbindungen der Formel (I) hergestellt werden.

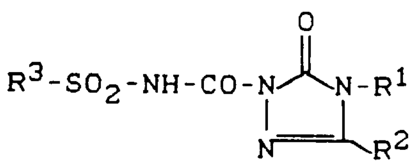

(I)

**Tabelle 3:** Herstellungsbeispiele für die Verbindungen der Formel (I)

| Beisp.-Nr. | $R^1$ | $R^2$ | $R^3$ | Schmelzpunkt (°C) |
|---|---|---|---|---|
| 2 | $-(CH_2)_5-$ | | 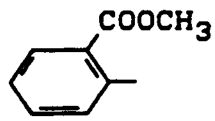 | 122 |
| 3 | $C_2H_5$ | H | 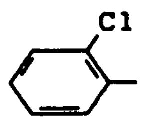 | 118 |
| 4 | $CH_3$ | $CH_3$ | 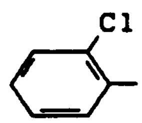 | 172 |
| 5 | $-(CH_2)_5-$ | | 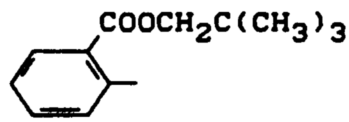 | 42 |
| 6 | $-(CH_2)_3-$ | | 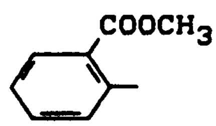 | amorph |
| 7 | $-(CH_2)_5-$ | | 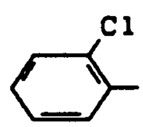 | 122 |
| 8 | $-(CH_2)_5-$ | | 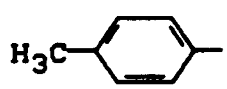 | 130 |
| 9 | $CH_3$ | $C_2H_5$ | 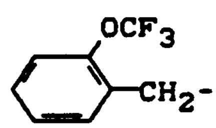 | 122 |

<u>Tabelle 3</u> - Fortsezung

| Beisp.-Nr. | $R^1$ | $R^2$ | $R^3$ | Schmelzpunkt (°C) |
|---|---|---|---|---|
| 10 | $CH_3$ | $CH_2CH_2OCH_3$ | 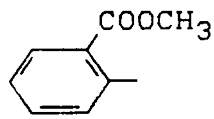 | amorph |
| 11 | $-(CH_2)_{11}-$ | | 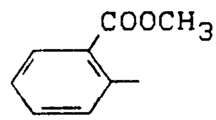 | 131 |
| 12 | $-(CH_2)_5-$ | | 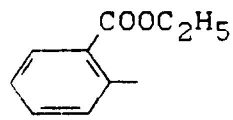 | rf-Wert* 0,17 |
| 13 | $-(CH_2)_6-$ | | 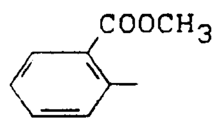 | rf-Wert* 0,26 |
| 14 | $-(CH_2)_7-$ | | 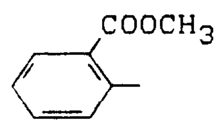 | rf-Wert* 0,28 |
| 15 | $-(CH_2)_4-$ | | 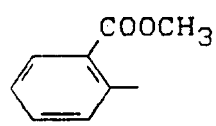 | 142 |
| 16 | $CH_3$ | $C_2H_5$ | 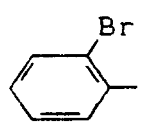 | 164 |
| 17 | $C_2H_5$ | $C_2H_5$ | 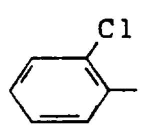 | 172 |

Tabelle 3 - Fortsezung

| Beisp.-Nr. | $R^1$ | $R^2$ | $R^3$ | Schmelzpunkt (°C) |
|---|---|---|---|---|
| 18 | $CH_3$ | $C_3H_7$-n | 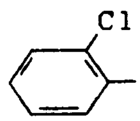 | 132 |
| 19 | $C_2H_5$ | $C_3H_7$-n | 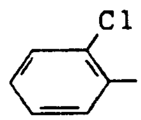 | 153 |
| 20 | $CH(CH_3)_2$ | $C_3H_7$-n | 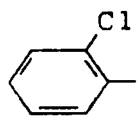 | 157 |
| 21 | $CH_3$ | $C_2H_5$ | 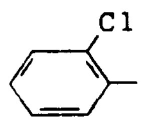 | 200 |
| 22 | $C_2H_5$ | $CH_3$ | 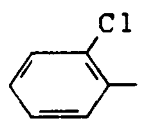 | 173 |
| 23 | $C_2H_5$ | $C_2H_5$ | 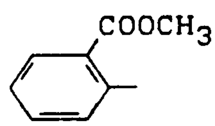 | 207 |
| 24 | $CH(CH_3)_2$ | $CH_3$ | 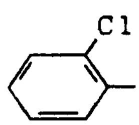 | 127 |
| 25 | $CH(CH_3)_2$ | $CH(CH_3)_2$ | 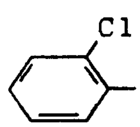 | 142 |

<u>Tabelle 3</u> - Fortsezung

| Beisp.-Nr. | $R^1$ | $R^2$ | $R^3$ | Schmelzpunkt (°C) |
|---|---|---|---|---|
| 26 | $CH(CH_3)_2$ | $C_2H_5$ | 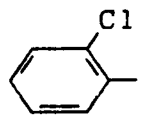 | 112 |
| 27 | $CH_3$ | $CH(CH_3)_2$ | 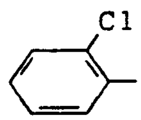 | 125 |
| 28 | $C_2H_5$ | $CH(CH_3)_2$ | 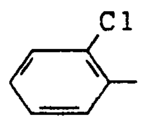 | 250 |
| 29 | $C_3H_7$-n | $CH(CH_3)_2$ | 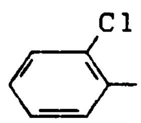 | 255 |
| 30 | $CH(CH_3)_2$ | $CH_3$ | 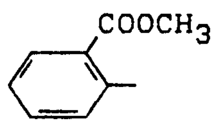 | 165 |
| 31 | $C_3H_7$-n | $CH_3$ | 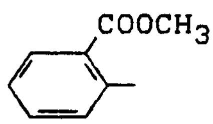 | 180 |
| 32 | $C_3H_7$-n | $C_2H_5$ | 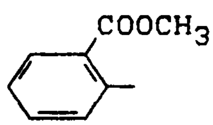 | 187 |
| 33 | $CH(CH_3)_2$ | $C_2H_5$ | 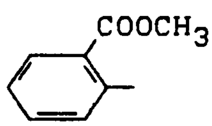 | 133 |

Tabelle 3 - Fortsezung

| Beisp.-Nr. | $R^1$ | $R^2$ | $R^3$ | Schmelzpunkt (°C) |
|---|---|---|---|---|
| 34 | $CH_3$ | $C_3H_7$-n | 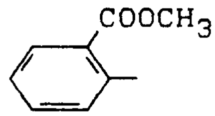 | 225 |
| 35 | $C_2H_5$ | $C_3H_7$-n | 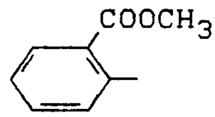 | 173 |
| 36 | $C_3H_7$-n | $C_3H_7$-n | 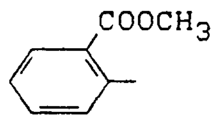 | 143 |
| 37 | $CH(CH_3)_2$ | $C_3H_7$-n | 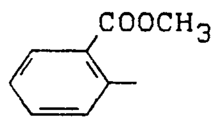 | 120 |
| 38 | $CH_3$ | $CH(CH_3)_2$ | 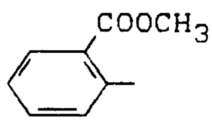 | 147 |
| 39 | $C_2H_5$ | $CH(CH_3)_2$ | 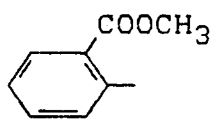 | 187 |
| 40 | $C_3H_7$-n | $CH(CH_3)_2$ | 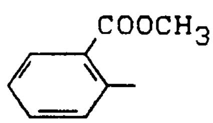 | 78 |
| 41 | $CH(CH_3)_2$ | $CH(CH_3)_2$ | 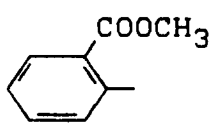 | 167 |

Tabelle 3 - Fortsezung

| Beisp.-Nr. | $R^1$ | $R^2$ | $R^3$ | Schmelzpunkt (°C) |
|---|---|---|---|---|
| 42 | $CH_3$ | $C_2H_5$ | 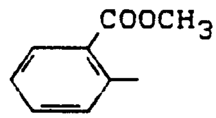 | 144 |
| 43 | $CH_3$ | $CH_3$ | 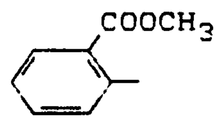 | 133 |
| 44 | $C_2H_5$ | $CH_3$ | 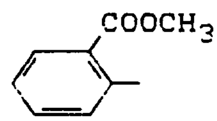 | 141 |
| 45 | 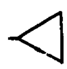 | $CH_3$ | 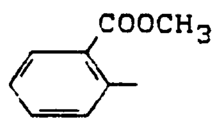 | 144 |
| 46 | 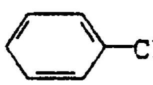 $CH_2$- | $CH_3$ | 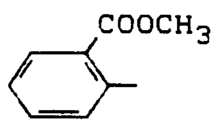 | 173 |
| 47 | $-N(CH_3)_2$ | $CH_3$ | 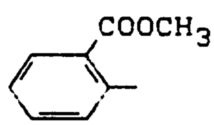 | 165 |

* rf-Werte gemessen durch Dünnschichtchromatographie - stationäre Phase: Kieselgel 60; Laufmittel: Essigsäure/Ethylacetat/Toluol (Volumenverhältnis 1:4:2).

Ausgangsstoffe der Formel (II)

Beispiel (II-1)

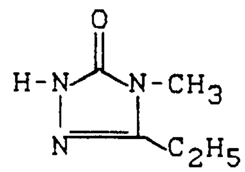

57 g (1 Mol) Methylisocyanat werden bei 20 °C bis 30 °C unter Rühren zu einer Mischung aus 50 g (1 Mol) Hydrazinhydrat und 200 ml Wasser tropfenweise gegeben; das Reaktionsgemisch wird 2 Stunden bei 20 °C bis 30 °C gerührt und anschließend wird das Lösungsmittel im Wasserstrahlvakuum sorgfältig abdestilliert.

Das so erhaltene Methylaminocarbonylhydrazin ($H_2N$-NH-CO-$NHCH_3$) - 82,5 g (0,93 Mol) - wird in 800 ml Methylenchlorid aufgenommen und bei 20 °C bis 30 °C werden unter Rühren 114 g (0,88 Mol) Propionsäureanhydrid tropfenweise dazu gegeben. Das Reaktionsgemisch wird 30 Minuten unter Rückfluß zum Sieden erhitzt und noch 15 Stunden bei 20 °C gerührt. Das kristallin angefallene Produkt wird durch Absaugen isoliert.

Das so erhaltene N-Methylaminocarbonyl-N'-propionylhydrazin ($H_5C_2$-CO-NH-NH-CO-$NHCH_3$) - 114 g (0,79 Mol) - wird zu einer auf 90 °C erhitzten Lösung von 31,4 g (0,79 Mol) Natrium-hydroxid in 2,4 l Wasser gegeben und das Reaktionsgemisch wird 60 Minuten bei 90 °C gerührt. Dann wird eingeengt, der Rückstand mit 300 ml Ethanol/Essigsäureethylester verrührt und filtriert. Das Filtrat wird eingeengt, mit Diethylether verrührt und das hierbei kristallin angefallene Produkt durch Absaugen isoliert.

Man erhält 67,4 g (67 % der Theorie) 5-Ethyl-4-methyl-2,4-dihydro-3H-1,2,4-triazol-3-on vom Schmelzpunkt 86 °C.

Beispiel (II-2)

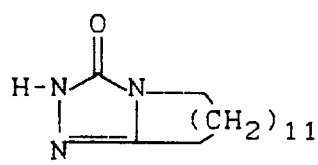

Eine Mischung aus 16 g (0,076 Mol) Dodecansäurelactim-O-methylether, 8 g (0,087 Mol) Carbazinsäureethylester und 100 ml Ethanol wird 23 Stunden unter Rückfluß zum Sieden erhitzt. Das beim Abkühlen kristallin angefallene Produkt wird durch Absaugen isoliert.

Man erhält 11,1 g (62 % der Theorie) 4,5-Undecan-1,11-diyl-2,4-dihydro-3H-1,2,4-triazol-3-on vom Schmelzpunkt 163 °C.

Beispiel (II-3)

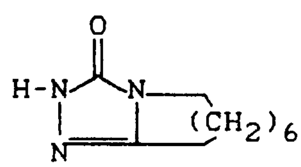

Eine Mischung aus 72 g (0,51 Mol) Oenanthsäurelactim-O-methylether, 26 g (0,55 Mol) Carbazinsäureethylester und 400 ml Ethanol sowie 150 ml Butanol wird 26 Stunden zum Sieden erhitzt. Die Lösung wird dann auf ein geringes Volumen eingeengt; die dabei angefallenen Kristalle werden durch Filtration

abgetrennt und mit Ethanol gewaschen.

Man erhält 17,1 g (20 % der Theorie) 4,5-Hexan-1,6-diyl-2,4-dihydro-3H-1,2,4-triazol-3-on vom Schmelzpunkt 114 °C.

Ausgangsstoffe der Formel (III)

Beispiel (III-1)

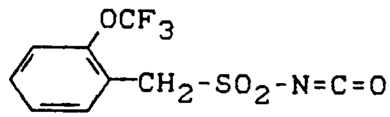

900 g (5,1 Mol) 2-Trifluormethoxy-toluol (2-Methyl-trifluoranisol) werden auf 100 °C erhitzt und bei dieser Temperatur werden unter UV-Bestrahlung 180 g (2,54 Mol) Chlor eingeleitet. Dann wird Stickstoff durchgeblasen und das Reaktionsgemisch wird unter vermindertem Druck fraktioniert destilliert.

Als Hauptfraktion erhält man 425 g (40 % der Theorie) 2-Trifluormethoxy-benzylchlorid (2-Chlormethyltrifluoranisol) vom Siedepunkt 110 °C/100 mbar und vom Brechungsinsdex $n_D^{20}$ = 1,5450.

21,0 g (0,1 Mol) 2-Trifluormethoxy-benzylchlorid werden mit einer gesättigten Lösung aus 13,9 g (0,11 Mol) Natriumsulfit in Wasser unter gutem Rühren 5 Stunden unter Rückfluß erhitzt. Nach dem Abkühlen wird der ausgefallene weiße Niederschlag abgesaugt und mit wenig eiskaltem Wasser nachgewaschen.

Nach Trocknen über Phosphorpentoxid werden 26,4 g (95 % der Theorie) 2-Trifluormethoxy-benzylsulfonsäure-Natriumsalz vom Schmelzpunkt 115 °C erhalten.

23,7 g (0,085 Mol) 2-Trifluormethoxy-benzylsulfonsäure-Natriumsalz werden mit 35,5 g (0,17 Mol) Phosphorpentachlorid vermischt und ca. 2 Stunden bei 80 °C - 90 °C Badtemperatur am Rotationsverdampfer umgeschwenkt. Es wird abgekühlt und das gebildete Phosphoroxychlorid im Vakuum entfernt. Der Rückstand wird in Methylenchlorid suspendiert und auf Eiswasser gegossen. Die organische Phase wird abgetrennt, neutral gewaschen, getrocknet und eingeengt.

Man erhält 19,0 g (81,4 % der Theorie) 2-Trifluormethoxy-benzylsulfonsäurechlorid als Rohware, das für die folgende Umsetzung zum Sulfonamid eine hinreichende Reinheit aufweist. Zur Reinigung kann die Rohware in Methylenchlorid aufgenommen und über Kieselgel gereinigt werden:

$$n_D^{22,5} = 1,4854.$$

205,9 g (0,75 Mol) 2-Trifluormethoxy-benzylsulfochlorid werden bei 30 °C - 40 °C in 1,5 l gesättigte wäßrige Ammoniaklösung eingetragen und 3 Stunden bei 50 °C - 60 °C nachgerührt. Nach dem Abkühlen wird der ausgefallene Niederschlag abgesaugt, mit Wasser neutral gewaschen und getrocknet.

Man erhält 136,5 g (71 % der Theorie) 2-Trifluormethoxybenzylsulfonsäureamid vom Schmelzpunkt 127 °C.

Eine Mischung aus 8,9 g (0,035 Mol) 2-Trifluormethoxybenzylsulfonsäureamid, 3,5 g (0,035 Mol) n-Butylisocyanat, 0,2 g Diaza-bicyclo-[2,2,2]-octan (DABCO) und 150 ml wasserfreiem Xylol wird auf Rückflußtemperatur erhitzt und für zwei Stunden wird ein schwacher Phosgen-Strom durchgeleitet. Es wird noch 30 Minuten bei Rückfluß nachgerührt, dann abgekühlt, filtriert und eingeengt. Der Rückstand wird in Methylenchlorid aufgenommen und erneut filtriert. Das Filtrat enthalt 2-Trifluormethoxy-benzylsulfonylisocyanat als Rohware im Gemisch mit DABCO und wird als solches für die Folgeumsetzung weiterverwendet, da bei der Destillation im Hochvakuum teilweise Zersetzung eintritt.

Anwendungsbeispiele

Bei den folgenden Anwendungsbeispielen wird das bekannte Herbizid Isocarbamid nachstehender Formel als Vergleichssubstanz herangezogen:

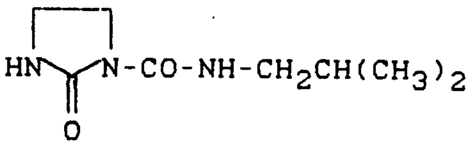 (A)

Isocarbamid

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)
100 % = totale Vernichtung

Eine deutliche Überlegenheit in der Wirksamkeit gegenüber dem Stand der Technik zeigen in diesem Test z. B. die Verbindungen gemäß Herstellungsbeispielen (2), (16), (18), (23).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)
100 % = totale Vernichtung

Eine deutliche Überlegenheit in der Wirksamkeit gegenüber dem Stand der Technik zeigen in diesem Test z. B. die Verbindungen gemäß Herstellungsbeispielen (2), (23).

Beispiel C

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen

mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Wirkstoffe eine gute fungizide Wirksamkeit.

**Patentansprüche**

**1.** Sulfonylaminocarbonyltriazolinone der allgemeinen Formel (I)

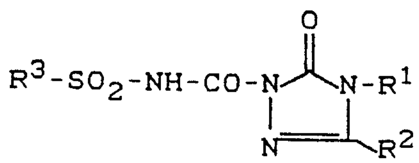

(I)

in welcher

$R^1$ für Wasserstoff, Hydroxy, Amino, für gegebenenfalls durch Fluor, Chlor, Brom, Cyano, $C_1$-$C_4$-Alkoxy, $C_1$-$C_4$-Alkylcarbonyl oder $C_1$-$C_4$-Alkoxy-carbonyl substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio, $C_1$-$C_4$-Alkylsulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für gegebenenfalls durch Fluor, Cyano, $C_1$-$C_4$-Alkoxy oder $C_1$-$C_4$-Alkoxy-carbonyl substituiertes $C_1$-$C_4$-Alkylamino oder für Di-($C_1$-$C_4$-alkyl)-amino steht,

$R^2$ für Wasserstoff, Hydroxy, Mercapto, Amino, für gegebenenfalls durch Fluor, Chlor, Brom, Cyano, $C_1$-$C_4$-Alkoxy oder $C_1$-$C_4$-Alkoxy-carbonyl substituiertes $C_1$-$C_6$-Alkyl, für gegebenenfalls durch Fluor, Chlor, Brom und/oder $C_1$-$C_4$-Alkyl substituiertes $C_3$-$C_6$-Cycloalkyl, für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl-$C_1$-$C_3$-alkyl, für gegebenenfalls durch Fluor, Chlor, Brom, Cyano, Nitro, $C_1$-$C_4$-Alkyl, Trifluormethyl, $C_1$-$C_4$-Alkoxy, Fluor- und/oder Chlor-substituiertes $C_1$-$C_3$-Alkoxy, $C_1$-$C_4$-Alkylthio, Fluor- und/oder Chlor-substituiertes $C_1$-$C_3$-Alkylthio,$C_1$-$C_4$-Alkyl-sulfinyl, $C_1$-$C_4$-Alkylsulfonyl und/oder $C_1$-$C_4$-Alkoxy-carbonyl substituiertes Phenyl, für gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, für gegebenenfalls durch Fluor, Chlor, Cyano, $C_1$-$C_4$-Alkoxy oder $C_1$-$C_4$-Alkoxy-carbonyl substituiertes Alkylthio, für $C_1$-$C_4$-Alkylamino oder Di-($C_1$-$C_4$-alkyl)-amino steht, oder

$R^1$ und $R^2$ zusammen für gegebenenfalls verzweigtes Alkandiyl mit 3 bis 11 Kohlenstoffatomen stehen und

$R^3$ für die Gruppierung

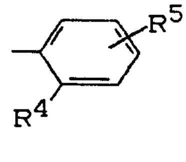

steht, worin

$R^4$ und $R^5$ gleich oder verschieden sind und für Wasserstoff, Fluor, Chlor, Brom, Iod, Nitro, $C_1$-$C_6$-Alkyl [welches gegebenenfalls durch Fluor, Chlor, Brom, Cyano, Carboxy,

$C_1$-$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxycarbonyl substituiert ist], $C_3$-$C_6$-Alkinyloxy, $C_3$-$C_6$-Alkinylthio oder für den Rest $-S(O)_p-R^6$ stehen, wobei

p für die Zahlen 1 oder 2 steht und

$R^6$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^7$ steht, wobei

$R^7$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^4$ und/oder $R^5$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^8$ stehen, wobei

$R^8$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^4$ und/oder $R^5$ weiterhin für $C_1$-$C_4$-Alkylsulfonyloxy, Di-($C_1$-$C_4$-alkyl)-aminosulfonylamino oder für den Rest $-CH=N-R^9$ stehen, wobei

$R^9$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$ -$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^3$ für den Rest

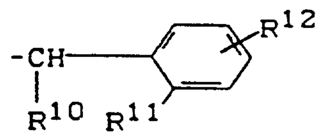

steht, worin

| | |
|---|---|
| $R^{10}$ | für Wasserstoff oder $C_1$-$C_4$-Alkyl steht, |
| $R^{11}$ und $R^{12}$ | gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/ oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter |
| $R^3$ | für den Rest |

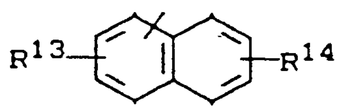

steht, worin

| | |
|---|---|
| $R^{13}$ und $R^{14}$ | gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter |
| $R^3$ | für den Rest |

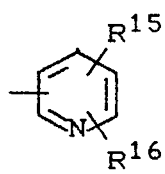

steht, worin

| | |
|---|---|
| $R^{15}$ und $R^{16}$ | gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; worin weiter |
| $R^3$ | für den Rest |

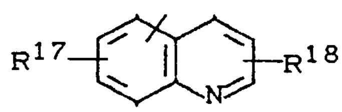

steht, worin

| | |
|---|---|
| $R^{17}$ und $R^{18}$ | gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter |
| $R^3$ | für den Rest |

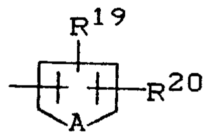

steht, worin

$R^{19}$ und $R^{20}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und

A für Sauerstoff, Schwefel oder die Gruppierung N-$Z^1$ steht, wobei

$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter

$R^3$ für den Rest

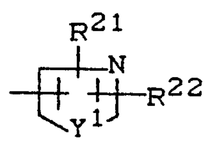

steht, worin

$R^{21}$ und $R^{22}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{23}$ steht, wobei

$R^{23}$ für Wasserstoff oder $C_1$-$C_4$-Alkyl steht; worin weiter

$R^3$ für den Rest

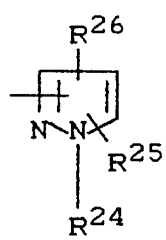

steht, worin

$R^{24}$ für Wasserstoff, $C_1$-$C_4$-Alkyl, Benzyl oder Phenyl steht,

$R^{25}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist] oder $C_1$-$C_4$-Alkoxy-carbonyl steht und

$R^{26}$ für Wasserstoff, Halogen oder $C_1$-$C_4$-Alkyl steht,

sowie Salze von Verbindungen der Formel (I).

**2.** Sulfonylaminocarbonyltriazolinone der allgemeinen Formel (I) gemäß Anspruch 1, dadurch gekennzeichnet, daß darin

$R^1$ für Wasserstoff, für gegebenenfalls durch Fluor, Cyano, Methoxy oder Ethoxy substituiertes $C_1$-$C_4$-Alkyl, für Cyclopropyl, Benzyl oder Dimethylamino steht,

$R^2$ für Wasserstoff oder für gegebenenfalls durch Fluor und/oder Chlor, Methoxy oder Ethoxy substituiertes $C_1$-$C_4$-Alkyl steht, oder

$R^1$ und $R^2$ zusammen für Alkandiyl mit 3 bis 11 Kohlenstoffatomen stehen und

$R^3$ für die Gruppierung

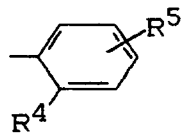

steht, worin

$R^4$ für Fluor, Chlor, Brom, Methyl, Trifluormethyl, Methoxy, Difluormethoxy, Trifluormethoxy, 2-Chlor-ethoxy, 2-Methoxy-ethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und

$R^5$ für Wasserstoff, Fluor, Chlor oder Brom steht; worin weiter

$R^3$ für den Rest

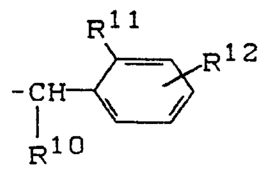

steht, worin

$R^{10}$ für Wasserstoff steht,

$R^{11}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{12}$ für Wasserstoff steht; worin weiter

$R^3$ für den Rest

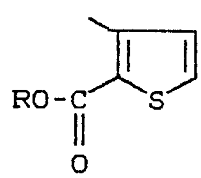

steht,
worin R für $C_1$-$C_4$-Alkyl steht, oder
für den Rest

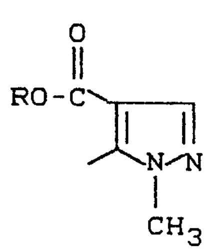

steht,
worin R für $C_1$-$C_4$-Alkyl steht,
und deren Salze.

**3.** Die Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, $C_5$- oder $C_6$-Cycloalkyl-ammonium- und Di-($C_1$-$C_2$-alkyl)-

benzyl-ammonium-Salze von Verbindungen der Formel (I) gemäß Anspruch 1.

4. Verfahren zur Herstellung von Sulfonylaminocarbonyltriazolinonen der allgemeinen Formel (I) gemäß Anspruch 1 und deren Salzen, dadurch gekennzeichnet, daß man Triazolinone der allgemeinen Formel (II)

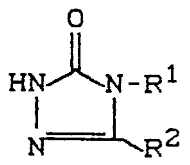

(II)

in welcher
$R^1$ und $R^2$ die in Anspruch 1 angegebenen Bedeutungen haben,
mit Sulfonylisocyanaten der allgemeinen Formel (III)

$R^3 - SO_2 - N = C = O$ (III)

in welcher
$R^3$ die in Anspruch 1 angegebene Bedeutung hat,
gegebenenfalls in Gegenwart eines Verdünnungsmittels umsetzt und gegebenenfalls im Anschluß daran Salze nach üblichen Methoden erzeugt.

5. Herbizide Mittel, gekennzeichnet durch einen Gehalt an mindestens einem Sulfonylaminocarbonyltriazolinon der Formel (I) gemäß Anspruch 1.

6. Verwendung von Sulfonylaminocarbonyltriazolinonen der allgemeinen Formel (I) gemäß Anspruch 1 zur Bekämpfung von unerwünschtem Pflanzenwachstum.

7. Fungizide Mittel, gekennzeichnet durch einen Gehalt an mindestens einem Sulfonylaminocarbonyltriazolinon der allgemeinen Formel (I) gemäß Anspruch 1.

8. Verwendung von Sulfonylaminocarbonyltriazolinonen der allgemeinen Formel (I) gemäß Anspruch 1 zur Bekämpfung von unerwünschten Mikroorganismen an Pflanzen.

9. Verfahren zur Herstellung von herbiziden und fungiziden Mitteln, dadurch gekennzeichnet, daß man Sulfonylaminocarbonyltriazolinone der allgemeinen Formel (I) gemäß Anspruch 1 mit Streckmitteln und/oder oberflächenaktiven Mitteln vermischt.

10. 4,5-Alkandiyl-2,4-dihydro-3H-1,2,4-triazol-3-one der Formel (IIa)

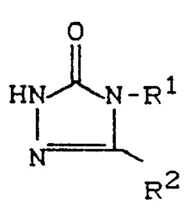

(IIa)

in welcher
$R^1$ und $R^2$ zusammen für Alkandiyl mit 6 und mit 8 bis 11 Kohlenstoffatomen stehen.

**11.** Triazolinone der Formel (II),

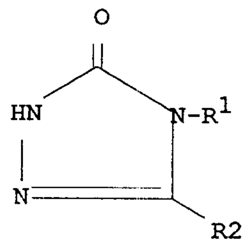

(II)

in welcher

(a) $R^1$ für $CHF_2$ und $R^2$ für H,
(b) $R^1$ für H und $R^2$ für $CF_3$,
(c) $R^1$ für $CH_3$ und $R^2$ für $CH(CH_3)_2$,
(d) $R^1$ für $CH_3$ und $R^2$ für $C_4H_9$,
(e) $R^1$ für $CHF_2$ und $R^2$ für $CH_3$,
(f) $R^1$ für $CHF_2$ und $R^2$ für $C_2H_5$,
(g) $R^1$ für

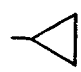

und $R^2$ für $CH_3$,
(h) $R^1$ für $CH_3$ und $R^2$ für

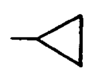

oder
(i) $R^1$ für

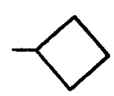

und $R^2$ für $CH_3$ stehen.

**Claims**

**1.** Sulphonylaminocarbonyltriazolinones of the general formula (I)

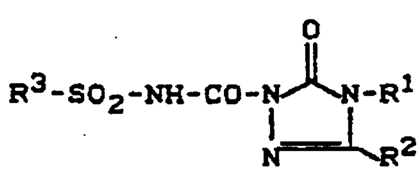

(I)

in which

$R^1$ represents hydrogen, hydroxyl, amino, $C_1$-$C_6$-alkyl which is optionally substituted by fluorine, chlorine, bromine, cyano, $C_1$-$C_4$-alkoxy, $C_1$-$C_4$-alkylcarbonyl or $C_1$-$C_4$-alkoxy-carbonyl, or represents $C_3$-$C_6$-cycloalkyl which is optionally substituted by fluorine, chlorine, bromine and/or $C_1$-$C_4$-alkyl, or represents phenyl-$C_1$-$C_3$-alkyl which is optionally substituted by fluorine, chlorine, bromine, cyano, nitro, $C_1$-$C_4$-

alkyl, trifluoromethyl, $C_1$-$C_4$-alkoxy and/or $C_1$-$C_4$-alkoxycarbonyl, or represents phenyl which is optionally substituted by fluorine, chlorine, bromine, cyano, nitro, $C_1$-$C_4$-alkyl, trifluoromethyl, $C_1$-$C_4$-alkoxy, fluorine- and/or chlorine-substituted $C_1$-$C_3$-alkoxy, $C_1$-$C_4$-alkylthio, fluorine- and/or chlorine-substituted $C_1$-$C_3$-alkylthio, $C_1$-$C_4$-alkylsulphinyl, $C_1$-$C_4$-alkylsulphonyl and/or $C_1$-$C_4$-alkoxy-carbonyl, or represents $C_1$-$C_6$-alkoxy which is optionally substituted by fluorine, chlorine, cyano, $C_1$-$C_4$-alkoxy or $C_1$-$C_4$-alkoxy-carbonyl, or represents $C_1$-$C_4$-alkylamino which is optionally substituted by fluorine, cyano, $C_1$-$C_4$-alkoxy or $C_1$-$C_4$-alkoxycarbonyl, or represents di-($C_1$-$C_4$-alkyl)-amino,

$R^2$ represents hydrogen, hydroxyl, mercapto, amino, $C_1$-$C_6$-alkyl which is optionally substituted by fluorine, chlorine, bromine, cyano, $C_1$-$C_4$-alkoxy or $C_1$-$C_4$-alkoxy-carbonyl, or represents $C_3$-$C_6$-cycloalkyl which is optionally substituted by fluorine, chlorine, bromine and/or $C_1$-$C_4$-alkyl, or represents phenyl-$C_1$-$C_3$-alkyl which is optionally substituted by fluorine, chlorine, bromine, cyano, nitro, $C_1$-$C_4$-alkyl,trifluoromethyl, $C_1$-$C_4$-alkoxy and/or $C_1$-$C_4$-alkoxy-carbonyl, or represents phenyl which is optionally substituted by fluorine, chlorine, bromine, cyano, nitro, $C_1$-$C_4$-alkyl, trifluoromethyl, $C_1$-$C_4$-alkoxy, fluorine- and/or chlorine-substituted $C_1$-$C_3$-alkoxy, $C_1$-$C_4$-alkylthio, fluorine- and/or chlorine-substituted $C_1$-$C_3$-alkylthio, $C_1$-$C_4$-alkylsulphinyl, $C_1$-$C_4$-alkylsulphonyl and/or $C_1$-$C_4$-alkoxy-carbonyl, or represents $C_1$-$C_6$-alkoxy which is optionally substituted by fluorine, chlorine, cyano, $C_1$-$C_4$-alkoxy or $C_1$-$C_4$-alkoxy-carbonyl, or represents alkylthio which is optionally substituted by fluorine, chlorine, cyano, $C_1$-$C_4$-alkoxy or $C_1$-$C_4$-alkoxy-carbonyl, or represents $C_1$-$C_4$-alkylamino or di-($C_1$-$C_4$-alkyl)-amino, or

$R^1$ and $R^2$ together represent optionally branched alkanediyl having 3 to 11 carbon atoms and

$R^3$ represents the group

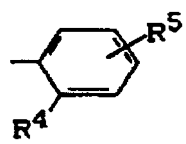

in which

$R^4$ and $R^5$ are identical or different and represent hydrogen, fluorine, chlorine, bromine, iodine, nitro, $C_1$-$C_6$-alkyl [which is optionally substituted by fluorine, chlorine, bromine, cyano, carboxyl, $C_1$-$C_4$-alkoxycarbonyl, $C_1$-$C_4$-alkylamino-carbonyl, di-($C_1$-$C_4$-alkyl)amino-carbonyl, hydroxyl, $C_1$-$C_4$-alkoxy, formyloxy, $C_1$-$C_4$-alkyl-carbonyloxy, $C_1$-$C_4$-alkoxy-carbonyloxy, $C_1$-$C_4$-alkylaminocarbonyloxy, $C_1$-$C_4$-alkylthio, $C_1$-$C_4$-alkylsulphinyl, $C_1$-$C_4$-alkylsulphonyl, di-($C_1$-$C_4$-alkyl)-aminosulphonyl, $C_3$-$C_6$-cycloalkyl or phenyl], or represent $C_2$-$C_6$-alkenyl [which is optionally substituted by fluorine, chlorine, bromine, cyano, $C_1$-$C_4$-alkoxy-carbonyl, carboxyl or phenyl], or represent $C_2$-$C_6$-alkinyl [which is optionally substituted by fluorine, chlorine, bromine, cyano, $C_1$-$C_4$-alkoxycarbonyl, carboxyl or phenyl], or represent $C_1$-$C_4$-alkoxy [which is optionally substituted by fluorine, chlorine, bromine, cyano, carboxyl, $C_1$-$C_4$-alkoxy-carbonyl, $C_1$-$C_4$-alkoxy, $C_1$-$C_4$-alkylthio, $C_1$-$C_4$-alkylsulphinyl or $C_1$-$C_4$-alkylsulphonyl], or represent $C_1$-$C_4$-alkylthio [which is optionally substituted by fluorine, chlorine, bromine, cyano, carboxyl, $C_1$-$C_4$-alkoxy-carbonyl, $C_1$-$C_4$-alkylthio, $C_1$-$C_4$-alkylsulphinyl or $C_1$-$C_4$-alkylsulphonyl], or represent $C_3$-$C_6$-alkenyloxy [which is optionally substituted by fluorine, chlorine, bromine, cyano or $C_1$-$C_4$-alkoxy-carbonyl], or represent $C_2$-$C_6$-alkenylthio [which is optionally substituted by fluorine, chlorine, bromine, cyano, nitro, $C_1$-$C_3$-alkylthio or $C_1$-$C_4$-alkoxycarbonyl], $C_3$-$C_6$-alkinyloxy, $C_3$-$C_6$-alkinylthio or the radical $-S(O)_p-R^6$ in which

p represents the numbers 1 or 2 and

$R^6$ represents $C_1$-$C_4$-alkyl [which is optionally substituted by fluorine, chlorine, bromine, cyano or $C_1$-$C_4$-alkoxy-carbonyl],$C_3$-$C_6$-alkenyl, $C_3$-$C_6$-alkinyl, $C_1$-$C_4$-alkoxy, $C_1$-$C_4$-alkoxy-$C_1$-$C_4$-alkylamino, $C_1$-$C_4$-alkylamino, di-($C_1$-$C_4$-alkyl)-amino or the radical $-NHOR^7$ in which

$R^7$ represents $C_1$-$C_{12}$-alkyl [which is optionally substituted by fluorine, chlorine, cyano,

$C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkyl-carbonyl, $C_1$-$C_4$-alkoxy-carbonyl, $C_1$-$C_4$-alkylamino-carbonyl or di-($C_1$-$C_4$-alkyl)-amino-carbonyl], or represents $C_3$-$C_6$-alkenyl [which is optionally substituted by fluorine, chlorine or bromine], $C_3$-$C_6$-alkinyl, $C_3$-$C_6$-cycloalkyl, $C_3$-$C_6$-cycloalkyl-$C_1$-$C_2$-alkyl, phenyl-$C_1$-$C_2$-alkyl [which is optionally substituted by fluorine, chlorine, nitro, cyano, $C_1$-$C_4$-alkyl, $C_1$-$C_4$-alkoxy or $C_1$-$C_4$-alkoxy-carbonyl], or represents benzhydryl, or represents phenyl [which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl],

$R^4$ and/or $R^5$ furthermore represent phenyl or phenoxy, or represent $C_1$-$C_4$-alkylcarbonylamino, $C_1$-$C_4$-alkoxy-carbonylamino, $C_1$-$C_4$-alkylamino-carbonyl-amino, di-($C_1$-$C_4$-alkyl)-amino-carbonylamino, or represent the radical -CO-$R^8$ in which

$R^8$ represents $C_1$-$C_6$-alkyl, $C_1$-$C_6$-alkoxy, $C_3$-$C_6$-cycloalkoxy, $C_3$-$C_6$-alkenyloxy, $C_1$-$C_4$-alkylthio, $C_1$-$C_4$-alkylamino, $C_1$-$C_4$-alkoxyamino, $C_1$-$C_4$-alkoxy-$C_1$-$C_4$-alkyl-amino or di-($C_1$-$C_4$-alkyl)-amino [which are optionally substituted by fluorine and/or chlorine],

$R^4$ and/or $R^5$ furthermore represent $C_1$-$C_4$-alkylsulphonyloxy or di-($C_1$-$C_4$-alkyl)-aminosulphonylamino or the radical -CH=N-$R^9$ in which

$R^9$ represents $C_1$-$C_6$-alkyl which is optionally substituted by fluorine, chlorine, cyano, carboxyl, $C_1$-$C_4$-alkoxy, $C_1$-$C_4$-alkylthio, $C_1$-$C_4$-alkylsulphinyl or $C_1$-$C_4$-alkylsulphonyl, or represents benzyl which is optionally substituted by fluorine or chlorine, or represents $C_3$-$C_6$-alkenyl or $C_3$-$C_6$-alkinyl, each of which is optionally substituted by fluorine or chlorine, or represents phenyl which is optionally substituted by fluorine, chlorine, bromine, $C_1$-$C_4$-alkyl, $C_1$-$C_4$-alkoxy, trifluoromethyl, trifluoromethoxy or trifluoromethylthio, or represents $C_1$-$C_6$-alkoxy, $C_3$-$C_6$-alkenoxy, $C_3$-$C_6$-alkinoxy or benzyloxy, each of which is optionally substituted by fluorine and/or chlorine, or represents amino, $C_1$-$C_4$-alkylamino, di-($C_1$-$C_4$-alkyl)-amino, phenylamino- $C_1$-$C_4$-alkyl-carbonyl-amino, $C_1$-$C_4$-alkoxy-carbonylamino, $C_1$-$C_4$-alkyl-sulphonylamino, or represents phenylsulphonylamino which is optionally substituted by fluorine, chlorine, bromine or methyl,

in which furthermore

$R^3$ represents the radical

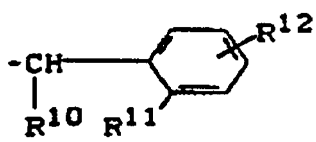

in which

$R^{10}$ represents hydrogen or $C_1$-$C_4$-alkyl,

$R^{11}$ and $R^{12}$ are identical or different and represent hydrogen, fluorine, chlorine, bromine, nitro, cyano, $C_1$-$C_4$-alkyl [which is optionally substituted by fluorine and/or chlorine], $C_1$-$C_4$-alkoxy [which is optionally substituted by fluorine and/or chlorine], carboxyl, $C_1$-$C_4$-alkoxy-carbonyl, $C_1$-$C_4$-alkylsulphonyl or di-($C_1$-$C_4$-alkyl)-aminosulphonyl; in which furthermore

$R^3$ represents the radical

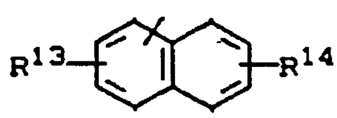

in which

$R^{13}$ and $R^{14}$ are identical or different and represent hydrogen, fluorine, chlorine, bromine, nitro, cyano, $C_1$-$C_4$-alkyl [which is optionally substituted by fluorine and/or chlorine] or $C_1$-$C_4$-alkoxy [which is optionally substituted by fluorine and/or chlorine]; in which furthermore

$R^3$ represents the radical

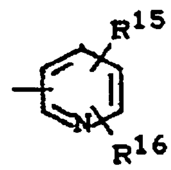

in which

$R^{15}$ and $R^{16}$ are identical or different and represent hydrogen, fluorine, chlorine, bromine, nitro, cyano, $C_1$-$C_4$-alkyl [which is optionally substituted by fluorine and/or chlorine], $C_1$-$C_4$-alkoxy [which is optionally substituted by fluorine and/or chlorine], or represent $C_1$-$C_4$-alkylthio, $C_1$-$C_4$-alkylsulphinyl or $C_1$-$C_4$-alkylsulphonyl [which are optionally substituted by fluorine and/or chlorine], or represent di-($C_1$-$C_4$-alkyl)-aminosulphonyl or $C_1$-$C_4$-alkoxy-carbonyl; in which furthermore

$R^3$ represents the radical

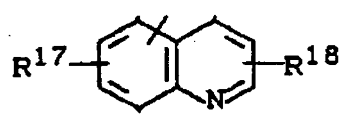

in which

$R^{17}$ and $R^{18}$ are identical or different and represent hydrogen, fluorine, chlorine, bromine, $C_1$-$C_4$-alkyl [which is optionally substituted by fluorine and/or bromine], $C_1$-$C_4$-alkoxy [which is optionally substituted by fluorine and/or chlorine], or represent $C_1$-$C_4$-alkylthio, $C_1$-$C_4$-alkylsulphinyl or $C_1$-$C_4$-alkylsulphonyl [which are optionally substituted by fluorine and/or chlorine], or represent di-($C_1$-$C_4$-alkyl)-aminosulphonyl; in which furthermore

$R^3$ represents the radical

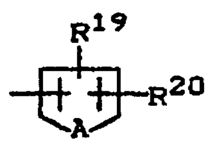

in which

$R^{19}$ and $R^{20}$ are identical or different and represent hydrogen, fluorine, chlorine, bromine, cyano, nitro, $C_1$-$C_4$-alkyl [which is optionally substituted by fluorine and/or chlorine], $C_1$-$C_4$-alkoxy [which is optionally substituted by fluorine and/or chlorine], $C_1$-$C_4$-alkylthio, $C_1$-$C_4$-alkylsulphinyl or $C_1$-$C_4$-alkylsulphonyl [which is optionally substituted by fluorine and/or chlorine], di-($C_1$-$C_4$-alkyl)-amino-sulphonyl or $C_1$-$C_4$-alkoxy-carbonyl, and

A represents oxygen, sulphur or the group N-$Z^1$ in which

$Z^1$ represents hydrogen, $C_1$-$C_4$-alkyl [which is optionally substituted by fluorine, chlorine, bromine or cyano], $C_3$-$C_6$-cycloalkyl, benzyl, phenyl [which is optionally substituted by fluorine, chlorine, bromine or nitro], $C_1$-$C_4$-alkylcarbonyl, $C_1$-$C_4$-alkoxy-carbonyl or di-($C_1$-$C_4$-alkyl)-aminocarbonyl; in which furthermore

$R^3$ represents the radical

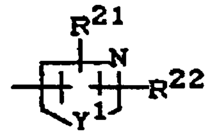

in which

| | |
|---|---|
| $R^{21}$ and $R^{22}$ | are identical or different and represent hydrogen, $C_1$-$C_4$-alkyl, halogen, $C_1$-$C_4$-alkoxycarbonyl, $C_1$-$C_4$-alkoxy or $C_1$-$C_4$-halogenoalkoxy, |
| $Y^1$ | represents sulphur or the group N-$R^{23}$ in which |
| $R^{23}$ | represents hydrogen or $C_1$-$C_4$-alkyl; in which furthermore |
| $R^3$ | represents the radical |

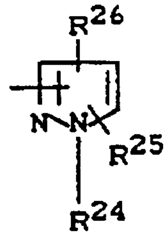

in which

| | |
|---|---|
| $R^{24}$ | represents hydrogen, $C_1$-$C_4$-alkyl, benzyl or phenyl, |
| $R^{25}$ | represents hydrogen, halogen, cyano, nitro, $C_1$-$C_4$-alkyl [which is optionally substituted by fluorine and/or chlorine], $C_1$-$C_4$-alkoxy [which is optionally substituted by fluorine and/or chlorine] or $C_1$-$C_4$-alkoxycarbonyl and |
| $R^{26}$ | represents hydrogen, halogen or $C_1$-$C_4$-alkyl, |
| | and salts of compounds of the formula (I). |

**2.** Sulphonylaminocarbonyltriazolinones of the general formula (I) according to Claim 1, characterized in that, in this formula,

| | |
|---|---|
| $R^1$ | represents hydrogen, $C_1$-$C_4$-alkyl which is optionally substituted by fluorine, cyano, methoxy or ethoxy, or represents cyclopropyl, benzyl or dimethylamino, |
| $R^2$ | represents hydrogen or $C_1$-$C_4$-alkyl which is optionally substituted by fluorine and/or chlorine, methoxy or ethoxy, or |
| $R^1$ and $R^2$ | together represent alkanediyl having 3 to 11 carbon atoms and |
| $R^3$ | represents the group |

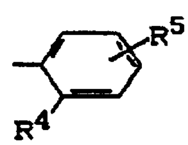

in which

| | |
|---|---|
| $R^4$ | represents fluorine, chlorine, bromine, methyl, trifluoromethyl, methoxy, difluoromethoxy, trifluoromethoxy, 2-chloro-ethoxy, 2-methoxyethoxy, $C_1$-$C_3$-alkylthio, $C_1$-$C_3$-alkylsulphinyl, $C_1$-$C_3$-alkylsulphonyl, dimethylaminosulphonyl, diethylaminosulphonyl, N-methoxy-N-methylaminosulphonyl, phenyl, phenoxy or $C_1$-$C_3$-alkoxy-carbonyl and |
| $R^5$ | represents hydrogen, fluorine, chlorine or bromine; in which furthermore |
| $R^3$ | represents the radical |

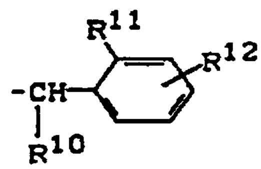

in which

$R^{10}$ represents hydrogen,

$R^{11}$ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and

$R^{12}$ represents hydrogen; in which furthermore

$R^3$ represents the radical

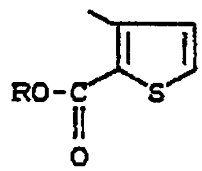

in which R represents $C_1$-$C_4$-alkyl,
or represents the radical

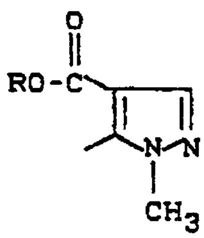

in which R represents $C_1$-$C_4$-alkyl,
and their salts.

**3.** The sodium, potassium, magnesium, calcium, ammonium, $C_1$-$C_4$-alkyl-ammonium, di-($C_1$-$C_4$-alkyl)-ammonium, tri-($C_1$-$C_4$-alkyl)-ammonium, $C_5$- or $C_6$-cycloalkyl-ammonium and di-($C_1$-$C_2$-alkyl)-benzyl-ammonium salts of compounds of the formula (I) according to Claim 1.

**4.** Process for the preparation of sulphonylaminocarbonyltriazolinones of the general formula (I) according to Claim 1 and their salts, characterized in that triazolinones of the general formula (II)

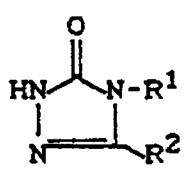

(II)

in which

$R^1$ and $R^2$ have the meanings given in Claim 1

are reacted with sulphonyl isocyanates of the general formula (III)

$R^3 - SO_2 - N = C = O$ (III)

in which

$R^3$ has the meaning given in Claim 1,

if appropriate in the presence of a diluent, and, if appropriate, salts are subsequently prepared by customary methods.

**5.** Herbicidal compositions, characterized in that they comprise at least one sulphonylaminocarbonyl-triazolinone of the formula (I) according to Claim 1.

**6.** Use of sulphonylaminocarbonyltriazolinones of the general formula (I) according to Claim 1 for combating undesired plant growth.

**7.** Fungicidal compositions, characterized in that they comprise at least one sulphonylaminocarbonyl-triazolinone of the general formula (I) according to Claim 1.

**8.** Use of sulphonylaminocarbonyltriazolinones of the general formula (I) according to Claim 1 for combating undesirable microorganisms on plants.

**9.** Process for the preparation of herbicidal and fungicidal compositions, characterized in that sulphonylaminocarbonyltriazolinones of the general formula (I) according to Claim 1 are mixed with extenders and/or surface-active agents.

**10.** 4,5-Alkanediyl-2,4-dihydro-3H-1,2,4-triazol-3-ones of the formula (IIa)

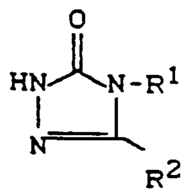

(IIa)

in which

$R^1$ and $R^2$ together represent alkanediyl having 6 and having 8 to 11 carbon atoms.

**11.** Triazolinones of the formula (11),

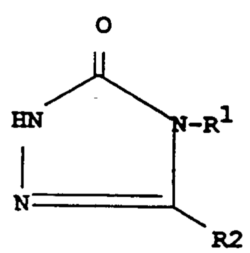

(II)

in which

(a) $R^1$ represents $CHF_2$ and $R^2$ represents H,

(b) $R^1$ represents H and $R^2$ represents $CF_3$,

(c) $R^1$ represents $CH_3$ and $R^2$ represents $CH(CH_3)_2$,

(d) $R^1$ represents $CH_3$ and $R^2$ represents $C_4H_9$,

(e) $R^1$ represents $CHF_2$ and $R^2$ represents $CH_3$,

(f) $R^1$ represents $CHF_2$ and $R^2$ represents $C_2H_5$,

(g) $R^1$ represents

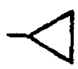

and $R^2$ represents $CH_3$,
(h) $R^1$ represents $CH_3$ and $R^2$ represents

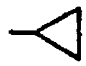

or
(i) $R^1$ represents

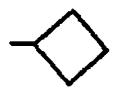

and $R^2$ represents $CH_3$ .

## Revendications

**1.** Sulfonylaminocarbonyltriazolinones répondant à la formule générale (I)

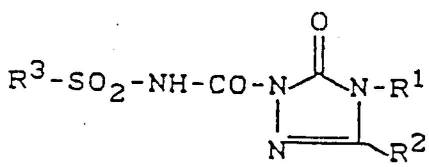 (I)

dans laquelle

$R^1$ représente un atome d'hydrogène, un groupe hydroxyle, un groupe amino; un groupe alkyle en $C_1$-$C_6$ éventuellement substitué une ou plusieurs fois de manière identique ou différente par un atome de fluor, par un atome de chlore, par un atome de brome, par un groupe cyano, par un groupe alcoxy en $C_1$-$C_4$, par un groupe alkyl(en $C_1$-$C_4$)carbonyle ou par un groupe alcoxy(en $C_1$-$C_4$)carbonyle; un groupe cycloalkyle en $C_3$-$C_6$ éventuellement substitué une ou plusieurs fois de manière identique ou différente par un atome de fluor, par un atome de chlore, par un atome de brome et/ou par un groupe alkyle en $C_1$-$C_4$; un groupe phénylalkyle en $C_1$-$C_3$ éventuellement substitué une ou plusieurs fois de manière identique ou différente par un atome de fluor, par un atome de chlore, par un atome de brome, par un groupe cyano, par un groupe nitro, par un groupe alkyle en $C_1$-$C_4$, par un groupe trifluorométhyle, par un groupe alcoxy en $C_1$-$C_4$ et/ou par un groupe alcoxy(en $C_1$-$C_4$)carbonyle; un groupe phényle éventuellement substitué une ou plusieurs fois de manière identique ou différente par un atome de fluor, par un atome de chlore, par un atome de brome, par un groupe cyano, par un groupe nitro, par un groupe alkyle en $C_1$-$C_4$, par un groupe trifluorométhyle, par un groupe alcoxy en $C_1$-$C_4$, par un groupe alcoxy en $C_1$-$C_3$ portant un ou plusieurs substituants identiques ou différents fluoro et/ou chloro, par un groupe alkyl(en $C_1$-$C_4$)thio, par un troupe alkyl(en $C_1$-$C_3$)-thio portant un ou plusieurs substituants identiques ou différents fluoro et/ou chloro, par un groupe alkyl(en $C_1$-$C_4$-sulfinyle), par un groupe alkyl(en $C_1$-$C_4$)sulfonyle et/ou par un groupe alcoxy(en $C_1$-$C_4$)carbonyle; un groupe alcoxy en $C_1$-$C_6$ éventuellement substitué une ou plusieurs fois de manière identique ou différente par un atome de fluor, par un atome de chlore, par un groupe cyano, par un groupe alcoxy en $C_1$-$C_4$ ou par un groupe alcoxy(en $C_1$-$C_4$)carbonyle; un groupe alkyl(en $C_1$-$C_4$)-amino éventuellement substitué une ou plusieurs fois de manière identique ou différente par un atome de fluor, par un groupe cyano, par un groupe alcoxy en $C_1$-$C_4$ ou encore par un groupe alcoxy(en $C_1$-$C_4$)carbonyle; ou représente un groupe di-(alkyl en $C_1$-$C_4$)amino,

$R^2$ représente un atome d'hydrogène, un groupe hydroxyle, un groupe mercapto, un

groupe amino; un groupe alkyle en $C_1$-$C_6$ éventuellement substitué une ou plusieurs fois de manière identique ou différente par un atome de fluor, par un atome de chlore, par un atome de brome, par un groupe cyano, par un groupe alcoxy en $C_1$-$C_4$ ou par un groupe alcoxy(en $C_1$-$C_4$)carbonyle; un groupe cycloalkyle en $C_3$-$C_6$ éventuellement substitué une ou plusieurs fois de manière identique ou différente par un atome de fluor, par un atome de chlore, par un atome de brome et/ou par un groupe alkyle en $C_1$-$C_4$; un groupe phénylalkyle en $C_1$-$C_3$ éventuellement substitué une ou plusieurs fois de manière identique ou différente par un atome de fluor, par un atome de chlore, par un atome de brome, par un groupe cyano, par un groupe nitro, par un groupe alkyle en $C_1$-$C_4$, par un groupe trifluorométhyle, par un groupe alcoxy en $C_1$-$C_4$ et/ou par un groupe alcoxy(en $C_1$-$C_4$)carbonyle; un groupe phényle éventuellement substitué une ou plusieurs fois de manière identique ou différente par un atome de fluor, par un atome de chlore, par un atome de brome, par un groupe cyano, par un groupe nitro, par un groupe alkyle en $C_1$-$C_4$, par un groupe trifluorométhyle, par un groupe alcoxy en $C_1$-$C_4$, par un groupe alcoxy en $C_1$-$C_3$ portant un ou plusieurs substituants identiques ou différents fluoro et/ou chloro, par un groupe alkyl(en $C_1$-$C_4$)thio, par un groupe alkyl(en $C_1$-$C_3$)thio portant un ou plusieurs substituants identiques ou différents fluoro et/ou chloro, par un groupe alkyl(en $C_1$-$C_4$-sulfinyle), par un groupe alkyl(en $C_1$-$C_4$)sulfonyle et/ou par un groupe alcoxy(en $C_1$-$C_4$)carbonyle; un groupe alcoxy en $C_1$-$C_6$ éventuellement substitué une ou plusieurs fois de manière identique ou différente par un atome de fluor, par un atome de chlore, par un groupe cyano, par un groupe alcoxy en $C_1$-$C_4$ ou par un groupe alcoxy(en $C_1$-$C_4$)carbonyle; un groupe alkylthio éventuellement substitué une ou plusieurs fois de manière identique ou différente par un atome de fluor, par un atome de chlore, par un groupe cyano, par un groupe alcoxy en $C_1$-$C_4$ ou par un groupe alcoxy(en $C_1$-$C_4$)carbonyle; un groupe alkyl(en $C_1$-$C_4$)amino ou représente un groupe di-(alkyl en $C_1$-$C_4$)amino, ou

$R^1$ et $R^2$ représentent ensemble un groupe alcanediyle éventuellement ramifié contenant de 3 à 11 atomes de carbone, et

$R^3$ représente le groupement

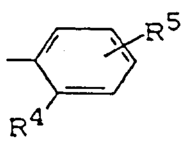

dans lequel

$R_4$ et $R_5$ sont identiques ou différents et représentent un atome d'hydrogène, un atome de fluor, un atome de chlore, un atome de brome, un atome d'iode, un groupe nitro, un groupe alkyle en $C_1$-$C_6$ [qui est éventuellement substitué une ou plusieurs fois de manière identique ou différente par un atome de fluor, par un atome de chlore, par un atome de brome, par un groupe cyano, par un groupe carboxyle, par un groupe alcoxy(en $C_1$-$C_4$)carbonyle, par un groupe alkyl(en $C_1$-$C_4$)aminocarbonyle, par un groupe di-(alkyl en $C_1$-$C_4$)aminocarbonyle, par un groupe hydroxyle, par un groupe alcoxy en $C_1$-$C_4$, par un groupe formyloxy, par un groupe alkyl(en $C_1$-$C_4$)-carbonyloxy, par un groupe alcoxy(en $C_1$-$C_4$)carbonyloxy, par un groupe alkyl(en $C_1$-$C_4$)aminocarbonyloxy, par un groupe alkyl(en $C_1$-$C_4$)thio, par un groupe alkyl(en $C_1$-$C_4$)sulfinyle, par un groupe alkyl(en $C_1$-$C_4$)sulfonyle, par un groupe di-(alkyl en $C_1$-$C_4$)aminosulfonyle, par un groupe cycloalkyle en $C_3$-$C_6$ ou par un groupe phényle], un groupe alcényle en $C_2$-$C_6$ [qui est éventuellement substitué une ou plusieurs fois de manière identique ou différente par un atome de fluor, par un atome de chlore, par un atome de brome, par un groupe cyano, par un groupe alcoxy(en $C_1$-$C_4$)carbonyle, par un groupe carboxyle ou par un groupe phényle], un groupe alcynyle en $C_2$-$C_6$ [qui est éventuellement substitué une ou plusieurs fois de manière identique ou différente par un atome de fluor, par un atome de chlore, par un atome de brome, par un groupe cyano, par un groupe alcoxy(en $C_1$-$C_4$)-carbonyle, par un groupe carboxyle ou par un groupe phényle], un groupe alcoxy en

$C_1$-$C_4$ [qui est éventuellement substitué une ou plusieurs fois de manière identique ou différente par un atome de fluor, par un atome de chlore, par un atome de brome, par un groupe cyano, par un groupe carboxyle, par un groupe alcoxy(en $C_1$-$C_4$)carbonyle, par un groupe alcoxy en $C_1$-$C_4$, par un groupe alkyl(en $C_1$-$C_4$)thio, par un groupe alkyl(en $C_1$-$C_4$)sulfinyle ou par un groupe alkyl(en $C_1$-$C_4$)sulfonyle], un groupe alkyl(en $C_1$-$C_4$)thio [qui est éventuellement substitué une ou plusieurs fois de manière identique ou différente par un atome de fluor, par un atome de chlore, par un atome de brome, par un groupe cyano, par un groupe carboxyle, par un groupe alcoxy(en $C_1$-$C_4$)carbonyle, par un groupe alkyl(en $C_1$-$C_4$)thio, par un groupe alkyl-(en $C_1$-$C_4$)sulfinyle ou par un groupe alkyl(en $C_1$-$C_4$)sulfonyle], un groupe alcényl(en $C_3$-$C_6$)oxy [qui est éventuellement substitué une ou plusieurs fois de manière identique ou différente par un atome de fluor, par un atome de chlore, par un atome de brome, par un groupe cyano, par un groupe alcoxy(en $C_1$-$C_4$)carbonyle], un groupe alcényl(en $C_2$-$C_6$)thio [qui est éventuellement substitué une ou plusieurs fois de manière identique ou différente par un atome de fluor, par un atome de chlore, par un atome de brome, par un groupe cyano, par un groupe nitro, par un groupe alkyl(en $C_1$-$C_3$)thio ou par un groupe alcoxy(en $C_1$-$C_4$)carbonyle], un groupe alcynyl-(en $C_3$-$C_6$)oxy, un groupe alcynyl(en $C_3$-$C_6$)thio, ou encore représente le radical -S-$(O)_p$-$R^6$ où

p représente les chiffres 1 ou 2, et

$R^6$ représente un groupe alkyle en $C_1$-$C_4$ [qui est éventuellement substitué une ou plusieurs fois de manière identique ou différente par un atome de fluor, par un atome de chlore, par un atome de brome, par un groupe cyano ou par un groupe alcoxy(en $C_1$-$C_4$)carbonyle], un groupe alcényle en $C_3$-$C_6$, un groupe alcynyle en $C_3$-$C_6$, un groupe alcoxy en $C_1$-$C_4$, un groupe alcoxy(en $C_1$-$C_4$)alkyl(en $C_1$-$C_4$)-amino, un groupe alkyl(en $C_1$-$C_4$)amino, un groupe di-(alkyl en $C_1$-$C_4$)amino, ou représente le radical -NHOR$^7$ où

$R^7$ représente un groupe alkyle en $C_1$-$C_{12}$ [qui est éventuellement substitué une ou plusieurs fois de manière identique ou différente par un atome de fluor, par un atome de chlore, par un groupe cyano, par un groupe alcoxy en $C_1$-$C_4$, par un groupe alkyl(en $C_1$-$C_4$)thio, par un groupe alkyl(en $C_1$-$C_4$)sulfinyle, par un groupe alkyl(en $C_1$-$C_4$)sulfonyle, par un groupe alkyl(en $C_1$-$C_4$)carbonyle, par un groupe alcoxy(en $C_1$-$C_4$)carbonyle, par un groupe alkyl(en $C_1$-$C_4$)aminocarbonyle ou encore par un groupe di-(alkyl en $C_1$-$C_4$) aminocarbonyle], un groupe alcényle en $C_3$-$C_6$ - [qui est éventuellement substitué une ou plusieurs fois de manière identique ou différente par un atome de fluor, par un atome de chlore ou par un atome de brome], un groupe alcynyle en $C_3$-$C_6$, un groupe cycloalkyle en $C_3$-$C_6$, un groupe cycloalkyl(en $C_3$-$C_6$)alkyle en $C_1$-$C_2$; un groupe phénylalkyle en $C_1$-$C_2$ [qui est éventuellement substitué une ou plusieurs fois de manière identique ou différente par un atome de fluor, par un atome de chlore, par un groupe nitro, par un groupe cyano, par un groupe alkyle en $C_1$-$C_4$, par un groupe alcoxy en $C_1$-$C_4$ ou par un groupe alcoxy(en $C_1$-$C_4$)carbonyle], un groupe benzhydryle ou encore un groupe phényle [qui est éventuellement substitué une ou plusieurs fois de manière identique ou différente par un atome de fluor, par un atome de chlore, par un groupe nitro, par un groupe cyano, par un groupe alkyle en $C_1$-$C_4$, par un groupe trifluorométhyle, par un groupe alcoxy en $C_1$-$C_4$, par un groupe fluoralcoxy en $C_1$-$C_2$, par un groupe alkyl(en $C_1$-$C_4$)thio, par un groupe trifluorométhylthio ou par un groupe alcoxy(en $C_1$-$C_4$)carbonyle],

$R^4$ et/ou $R^5$ représentent, en outre, un groupe phényle ou un groupe phénoxy; un groupe alkyl-(en $C_1$-$C_4$)carbonylamino, un groupe alcoxy(en $C_1$-$C_4$)carbonylamino, un groupe alkyl(en $C_1$-$C_4$)aminocarbonylamino, un groupe di-(alkyl en $C_1$-$C_4$)-aminocarbonylamino ou encore représentent le radical -CO-$R^8$ où

$R^8$ représente un groupe alkyle en $C_1$-$C_6$, un groupe alcoxy en $C_1$-$C_6$, un groupe cycloalcoxy en $C_3$-$C_6$, un groupe alcényl(en $C_3$-$C_6$)oxy, un groupe alkyl(en $C_1$-$C_4$)-thio, un groupe alkyl(en $C_1$-$C_4$)amino, un groupe alcoxy(en $C_1$-$C_4$)amino, un groupe alcoxy(en $C_1$-$C_4$)alkyl(en $C_1$-$C_4$)amino ou encore un groupe di-(alkyl en $C_1$-$C_4$)amino [qui sont éventuellement substitués une ou plusieurs fois de manière identique ou différente par un atome de fluor et/ou par un atome de chlore],

$R^4$ et/ou $R^5$ représentent, en outre, un groupe alkyl(en $C_1$-$C_4$)sulfonyloxy, un groupe di-(alkyl en $C_1$-$C_4$)aminosulfonylamino ou représentent le radical -CH = N-$R^9$ où

$R^9$ représente un groupe alkyle en $C_1$-$C_6$ éventuellement substitué une ou plusieurs fois de manière identique ou différente par un atome de fluor, par un atome de chlore, par un groupe cyano, par un groupe carboxyle, par un groupe alcoxy en $C_1$-$C_4$, par un groupe alkyl(en $C_1$-$C_4$)thio, par un groupe alkyl(en $C_1$-$C_4$)sulfinyle ou par un groupe alkyl(en $C_1$-$C_4$)sulfonyle; un groupe benzyle éventuellement substitué une ou plusieurs fois de manière identique ou différente par un atome de fluor ou par un atome de chlore; un groupe alcényle en $C_3$-$C_6$ ou un groupe alcynyle en $C_3$-$C_6$ éventuellement substitués une ou plusieurs fois de manière identique ou différente par un atome de fluor ou par un atome de chlore; un groupe phényle éventuellement substitué une ou plusieurs fois de manière identique ou différente par un atome de fluor, par un atome de chlore, par un atome de brome, par un groupe alkyle en $C_1$-$C_4$, par un groupe alcoxy en $C_1$-$C_4$ ou par un groupe trifluorométhyle, par un groupe trifluorométhoxy ou par un groupe trifluorométhylthio; un groupe alcoxy en $C_1$-$C_6$, un groupe alcénoxy en $C_3$-$C_6$, un groupe alcynoxy en $C_3$-$C_6$ ou un groupe benzyloxy, chacun de ces groupes étant éventuellement substitué une ou plusieurs fois de manière identique ou différente par un atome de fluor et/ou par un atome de chlore; un groupe amino, un groupe alkyl(en $C_1$-$C_4$)amino, un groupe di-(alkyl en $C_1$-$C_4$)-amino, un groupe phénylamino, un groupe alkyl(en $C_1$-$C_4$)carbonylamino,un groupe alcoxy(en $C_1$-$C_4$)carbonylamino, un groupe alkyl(en $C_1$-$C_4$)sulfonylamino, ou représente un groupe phénylsulfonylamino éventuellement substitué une ou plusieurs fois de manière identique ou différente par un atome de fluor, par un atome de chlore, par un atome de brome ou par un groupe méthyle;

dans laquelle, en outre,

$R^3$ représente le radical

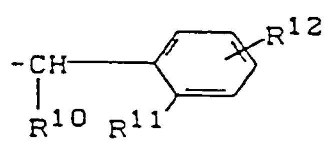

dans lequel

$R^{10}$ représente un atome d'hydrogène ou un groupe alkyle en $C_1$-$C_4$,

$R^{11}$ et $R^{12}$ sont identiques ou différents et représentent un atome d'hydrogène, un atome de fluor, un atome de chlore, un atome de brome, un groupe nitro, un groupe cyano, un groupe alkyle en $C_1$-$C_4$ [qui est éventuellement substitué une ou plusieurs fois de manière identique ou différente par un atome de fluor et/ou par un atome de chlore], un groupe alcoxy en $C_1$-$C_4$ [qui est éventuellement substitué une ou plusieurs fois de manière identique ou différente par un atome de fluor et/ou par un atome de chlore], un groupe carboxyle, un groupe alcoxy(en $C_1$-$C_4$)carbonyle, un groupe alkyl(en $C_1$-$C_4$)sulfonyle ou encore un groupe di-(alkyl en $C_1$-$C_4$)aminosulfonyle;

dans laquelle, en outre,

$R^3$ représente le radical

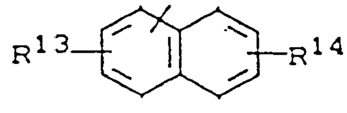

dans lequel

$R^{13}$ et $R^{14}$ sont identiques ou différents et représentent un atone d'hydrogène, un atome de fluor, un atome de chlore, un atome de brome, un groupe nitro, un groupe cyano, un groupe alkyle en $C_1$-$C_4$ [qui est éventuellement substitué une ou plusieurs fois de manière identique ou différente par un atome de fluor et/ou par un atome de chlore] ou encore un groupe alcoxy en $C_1$-$C_4$ [qui est éventuellement substitué une ou plusieurs fois de manière identique ou différente par un atome de fluor et/ou par un

atome de chlore];

dans laquelle, en outre,

$R^3$ représente le radical

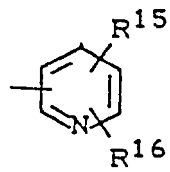

dans lequel

$R^{15}$ et $R^{16}$ sont identiques ou différents et représente un atome d'hydrogène, un atome de fluor, un atome de chlore, un atome de brome, un groupe nitro, un groupe cyano, un groupe alkyle en $C_1$-$C_4$ [qui est éventuellement substitué une ou plusieurs fois de manière identique ou différente par un atome de fluor et/ou par un atome de chlore], un groupe alcoxy en $C_1$-$C_4$ [qui est éventuellement substitué une ou plusieurs fois de manière identique ou différente par un atome de fluor et/ou par un atome de chlore], un groupe alkyl(en $C_1$-$C_4$)thio, un groupe alkyl(en $C_1$-$C_4$)sulfinyle ou un groupe alkyl-(en $C_1$-$C_4$)sulfonyle [qui sont éventuellement substitués une ou plusieurs fois de manière identique ou différente par un atome de fluor et/ou par un atome de chlore], ainsi qu'un groupe di-(alkyl en $C_1$-$C_4$)aminosulfonyle ou encore un groupe alcoxy(en $C_1$-$C_4$)carbonyle;

dans laquelle, en outre,

$R^3$ représente le radical

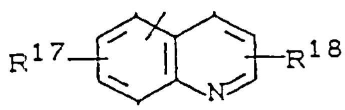

dans lequel

$R^{17}$ et $R^{18}$ sont identiques ou différents et représentent un atome d'hydrogène, un atome de fluor, un atome de chlore, un atome de brome, un groupe alkyle en $C_1$-$C_4$ [qui est éventuellement substitué une ou plusieurs fois de manière identique ou différente par un atome de fluor et/ou par un atome de brome], un groupe alcoxy en $C_1$-$C_4$ [qui est éventuellement substitué une ou plusieurs fois de manière identique ou différente par un atome de fluor et/ou par un atome de chlore], un groupe alkyl(en $C_1$-$C_4$)thio, un groupe alkyl(en $C_1$-$C_4$)sulfinyle ou un groupe alkyl(en $C_1$-$C_4$)sulfonyle [qui sont éventuellement substitués une ou plusieurs fois de manière identique ou différente par un atome de fluor et/ou par un atome de chlore], ou encore représentent un groupe di-(alkyl en $C_1$-$C_4$)aminosulfonyle;

dans laquelle, en outre,

$R^3$ représente le radical

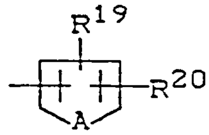

dans lequel

$R^{19}$ et $R^{20}$ sont identiques ou différents et représentent un atome d'hydrogène, un atome de fluor, un atome de chlore, un atome de brome, un groupe cyano, un groupe nitro, un groupe alkyle en $C_1$-$C_4$ [qui est éventuellement substitué une ou plusieurs fois de manière identique ou différente par un atome de fluor et/ou par un atome de chlore], un groupe alcoxy en $C_1$-$C_4$ [qui est éventuellement substitué une ou plusieurs fois de manière identique ou différente par un atome de fluor et/ou par un atome de chlore],

un groupe alkyl(en $C_1$-$C_4$)thio, un groupe alkyl(en $C_1$-$C_4$)sulfinyle ou un groupe alkyl-(en $C_1$-$C_4$)sulfonyle [qui sont éventuellement substitués une ou plusieurs fois de manière identique ou différente par un atome de fluor et/ou par un atome de chlore], un groupe di-(alkyl en $C_1$-$C_4$)aminosulfonyle ou encore un groupe alcoxy(en $C_1$-$C_4$)-carbonyle, et

A représente un atome d'oxygène, un atome de soufre ou encore le groupement N-$Z^1$ où

$Z^1$ représente un atome d'hydrogène, un groupe alkyle en $C_1$-$C_4$ [qui est éventuellement substitué une ou plusieurs fois de manière identique ou différente par un atome de fluor, par un atome de chlore, par un atome de brome ou par un groupe cyano], un groupe cycloalkyle en $C_3$-$C_6$, un groupe benzyle, un groupe phényle [qui est éventuellement substitué une ou plusieurs fois de manière identique ou différente par un atome de fluor, par un atome de chlore, par un atome de brome et/ou par un groupe nitro], un groupe alkyl(en $C_1$-$C_4$)carbonyle, un groupe alcoxy(en $C_1$-$C_4$)carbonyle ou un groupe di(alkyl en $C_1$-$C_4$)aminocarbonyle;

dans laquelle, en outre,

$R^3$ représente le radical

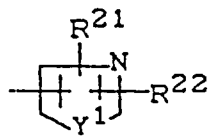

dans lequel

$R^{21}$ et $R^{22}$ sont identiques ou différents et représentent un atome d'hydrogène, un groupe alkyle en $C_1$-$C_4$, un atome d'halogène, un groupe alcoxy(en $C_1$-$C_4$)carbonyle, un groupe alcoxy en $C_1$-$C_4$ ou encore un groupe halogénalcoxy en $C_1$-$C_4$,

$Y^1$ représente un atome de soufre ou le groupement N-$R^{23}$ où

$R^{23}$ représente un atome d'hydrogène ou un groupe alkyle en $C_1$-$C_4$;

dans laquelle, en outre,

$R^3$ représente le radical

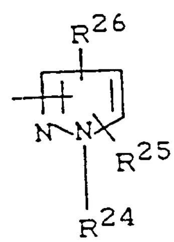

dans lequel

$R^{24}$ représente un atome d'hydrogène, un groupe alkyle en $C_1$-$C_4$, un groupe benzyle ou un groupe phényle,

$R^{25}$ représente un atome d'hydrogène, un atome d'halogène, un groupe cyano, un groupe nitro, un groupe alkyle en $C_1$-$C_4$ [qui est éventuellement substitué une ou plusieurs fois de manière identique ou différente par un atome de fluor et/ou par un atome de chlore], un groupe alcoxy en $C_1$-$C_4$ [qui est éventuellement substitué une ou plusieurs fois de manière identique ou différente par un atome de fluor et/ou par un atome de chlore] ou encore un groupe alcoxy(en $C_1$-$C_4$)carbonyle, et

$R^{26}$ représente un atome d'hydrogène, un atome d'halogène ou un groupe alkyle en $C_1$-$C_4$,

ainsi que des sels des composés de formule (I).

2. Sulfonylaminocarbonyltriazolinones répondant à la formule (I) selon la revendication 1, caractérisées en ce que, dans cette formule,

$R^1$ représente un atome d'hydrogène; un groupe alkyle en $C_1$-$C_4$ éventuellement substitué

une ou plusieurs fois de manière identique ou différente par un atome de fluor, par un groupe cyano, par un groupe méthoxy ou par un groupe éthoxy; un groupe cyclopropyle, un groupe benzyle ou un groupe diméthylamino,

$R^2$ représente un atome d'hydrogène ou encore un groupe alkyle en $C_1$-$C_4$ éventuellement substitué une ou plusieurs fois de manière identique ou différente par un atome de fluor et/ou par un atome de chlore, par un groupe méthoxy ou par un groupe éthoxy, ou

$R^1$ et $R^2$ représentent ensemble un groupe alcanediyle contenant de 3 à 11 atomes de carbone, et

$R^3$ représente le groupement

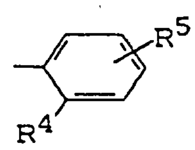

dans lequel

$R^4$ représente un atome de fluor, un atome de chlore, un atome de brome, un groupe méthyle, un groupe trifluorométhyle, un groupe méthoxy, un groupe difluorométhoxy, un groupe trifluorométhoxy, un groupe 2-chloroéthoxy, un groupe 2-méthoxyéthoxy, un groupe alkyl(en $C_1$-$C_3$)thio, un groupe alkyl(en $C_1$-$C_3$)sulfinyle, un groupe alkyl(en $C_1$-$C_3$)sulfonyle, un groupe diméthylaminosulfonyle, un groupe diéthylaminosulfonyle, un groupe N-méthoxy-N-méthylaminosulfonyle, un groupe phényle, un groupe phénoxy ou encore un groupe alcoxy(en $C_1$-$C_3$)carbonyle, et

$R^5$ représente un atome d'hydrogène, un atome de fluor, un atome de chlore ou un atome de brome;

dans laquelle, en outre,

$R^3$ représente le radical

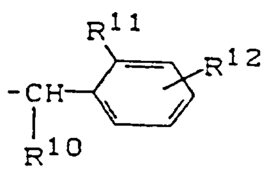

dans lequel

$R^{10}$ représente un atome d'hydrogène,

$R^{11}$ représente un atome de fluor, un atome de chlore, un atome de brome, un groupe méthyle, un groupe méthoxy, un groupe difluorométhoxy, un groupe trifluorométhoxy, un groupe éthoxy, un groupe méthoxycarbonyle, un groupe éthoxycarbonyle, un groupe méthylsulfonyle ou un groupe diméthylaminosulfonyle, et

$R^{12}$ représente un atome d'hydrogène;

dans laquelle, en outre,

$R^3$ représente le radical

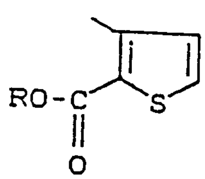

dans lequel R représente un groupe alkyle en $C_1$-$C_4$ ou représente le radical

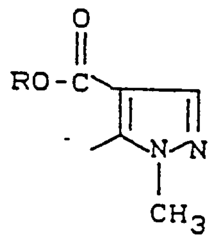

dans lequel R représente un groupe alkyle en $C_1$-$C_4$,
ainsi que leurs sels

3. Les sels de sodium, de potassium, de magnésium, de calcium, d'ammonium, d'alkyl(en $C_1$-$C_4$)-ammonium, de di-(alkyl en $C_1$-$C_4$)ammonium, de tri-(alkyl en $C_1$-$C_4$)ammonium, de cycloalkyl(en $C_5$ ou $C_6$)ammonium et de di-(alkyl en $C_1$-$C_2$)benzylammonium des composés de formule (I) selon la revendication 1.

4. Procédé pour la préparation de sulfonylaminocarbonyltriazolinones répondant à la formule générale (I) selon la revendication 1 et de leurs sels, caractérisé en ce qu'on fait réagir des triazolinones répondant à la formule générale (II)

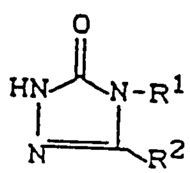

(II)

dans laquelle
$R^1$ et $R^2$ ont les significations indiquées à la revendication 1,
avec des sulfonylisocyanates répondant à la formule générale (III)

$R^3$ - $SO_2$ - N = C = O (III)

dans laquelle
$R^3$ a la signification indiquée à la revendication 1,
éventuellement en présence d'un diluant et, éventuellement à la suite de cette mise en réaction, on prépare des sels conformément à des procédés habituels.

5. Agents herbicides caractérisés par une teneur en au moins une sulfonylaminocarbonyltriazolinone de formule (I) selon la revendication 1.

6. Utilisation de sulfonylaminocarbonyltriazolinones répondant à la formule générale (I) selon la revendication 1, pour lutter contre la croissance indésirable de plantes.

7. Agents fongicides caractérisés par une teneur en au moins une sulfonylaminocarbonyltriazolinone répondant à la formule générale (I) selon la revendication 1.

8. Utilisation de sulfonylaminocarbonyltriazolinones répondant à la formule générale (I) selon la revendication 1 pour lutter contre des microorganismes non désirés sur des plantes.

9. Procédé pour la préparation d'agents herbicides et fongicides, caractérisé en ce qu'on mélange des sulfonylaminocarbonyltriazolinones répondant à la formule générale (I) selon la revendication 1, avec des diluants et/ou des agents tensioactifs.

**10.** 4,5-alcanediyl-2,4-dihydro-3H-1,2,4-triazol-3-ones répondant à la formule (IIa)

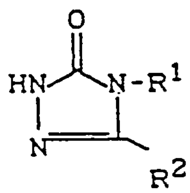

(IIa)

dans laquelle

$R^1$ et $R^2$ représentent ensemble un groupe alcanediyle contenant 6 et contenant de 8 à 11 atomes de carbone.

**11.** Triazolinones répondant à la formule (II)

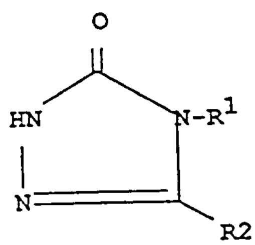

(II)

dans laquelle

(a) $R^1$ représente $CHF_2$ et $R^2$ représente H,
(b) $R^1$ représente H et $R^2$ représente $CF_3$,
(c) $R^1$ représente $CH_3$ et $R^2$ représente $CH(CH_3)_2$,
(d) $R^1$ représente $CH_3$ et $R^2$ représente $C_4H_9$,
(e) $R^1$ représente $CHF_2$ et $R^2$ représente $CH_3$,
(f) $R^1$ représente $CHF_2$ et $R^2$ représente $C_2H_5$,
(g) $R^1$ représente

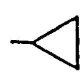

et $R^2$ représente $CH_3$,
(h) $R^1$ représente $CH_3$ et $R^2$ représente

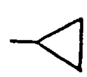

ou
(i) $R^1$ représente

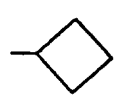

et $R^2$ représente $CH_3$.